# EUROPEAN PATENT APPLICATION

(11) **EP 4 834 252 A2**
(43) Date of publication of application: **04.11.2026**
(21) Application number: 26205221.0
(22) Date of filing: 14.03.2024
(51) Int. Cl.: A61B 17/3207

(54) **MEDICAL DEVICE FOR ENTERING A LESION WITHIN A VESSEL**

(30) Priority: 14.03.2023 WO PCT/JP2023/009924
(62) Document number(s) of the earlier application(s) in accordance with Art. 76 EPC: 24770966.0 / 4 512 455
(71) Applicant: ASAHI INTECC CO., LTD., Seto-shi, Aichi 4890071 (JP); Mustapha, Jihad A., Grandville, Michigan 49468 (US)
(72) Inventor: MATSUOKA, Erina, Seto-shi, Aichi, 4890071 (JP); HIRATA, Yamato, Seto-shi, Aichi, 4890071 (JP); NISHIGISHI, Makoto, Seto-shi, Aichi, 4890071 (JP); KAWAI, Shintaro, Seto-shi, Aichi, 4890071 (JP); TAKEMOTO, Hirokatsu, Seto-shi, Aichi, 4890071 (JP); TODA, Takuya, Seto-shi, Aichi, 4890071 (JP); LEE, Aeri, Seto-shi, Aichi, 4890071 (JP)
(74) Representative: Winter, Brandl - Partnerschaft mbB

(57) **Abstract**

A medical device (100) comprises an elongated main body part (10) and a leading portion (20) connected to a distal end (16) of the main body part (10). The leading portion (20) has a first end portion (21) located outside an outer peripheral surface (17) of the main body part (10).

## Description

### TECHNICAL FIELD

A technology as disclosed herein relates to a medical device.

### BACKGROUND ART

A guide wire is used for treating a constricted portion and an obstructed portion in a blood vessel (hereinafter referred to as a "lesion"). A guide wire is required to have high passability through a lesion.

A known guide wire includes a core wire and a coil. In the known guide wire, the outer diameter of a strand of the coil increases from the distal end of the coil toward the proximal end side, and reaches its maximum at the middle portion of the coil, and then decreases from the middle portion toward the proximal end side, in order to improve passability of the guide wire through a lesion (for example, see Patent Literature 1).

### CITATION LIST

### Patent Literature

Patent Literature 1: Japanese Patent Application Laid-Open No. 2020-39377

### SUMMARY OF INVENTION

### Technical Problem

Known guide wires have room for improvement in passability through a lesion.

Disclosed herein is a technology that can solve the problem as described above.

### Solution to Problem

The technology as disclosed herein can be embodied, for example, according to the following aspects.
(1) A medical device (100) as disclosed herein includes an elongated main body part (10) and a leading portion (20). The leading portion (20) is connected to a distal end (16) of the main body part (10), and enters into a lesion (220). The leading portion (20) has a first end portion (21) located outside an outer peripheral surface (17) of the main body part (10). According to the present medical device (100), the leading portion (20) having the first end portion (21) located outside the outer peripheral surface (17) of the main body part (10) can rotate to efficiently drill the lesion (220). This can improve the passability of the medical device (100) through the lesion (220).
(2) In the above medical device (100), the main body part (10) may extend along an axis (Ax), and the first end portion may be located outside the outer peripheral surface (17) of the main body part (10) in a first direction orthogonal to the axis (Ax) of the main body part (10). According to this configuration, the leading portion (20) having the first end portion (21) located outside the outer peripheral surface (17) of the main body part (10) in the first direction orthogonal to the axis (Ax) of the main body part (10) can rotate to efficiently drill the lesion (220). This can improve the passability of the medical device (100) through the lesion (220).
(3) In the above medical device (100), the leading portion (20) may have a second end portion (22) located outside the outer peripheral surface (17) of the main body part (10) in a second direction orthogonal to the axis (Ax). According to this configuration, the leading portion (20) having the second end portion (22) located outside the outer peripheral surface (17) of the main body part (10) in addition to the first end portion (21) can rotate to more efficiently drill the lesion (220). This can further improve the passability of the medical device (100) through the lesion (220).
(4) In the above medical device (100), a protrusion amount of the leading portion (20) from the outer peripheral surface (17) of the main body part (10) at the first end portion (21) may be different from a protrusion amount of the leading portion (20) from the outer peripheral surface (17) of the main body part (10) at the second end portion (22). According to this configuration, the leading portion (20) can well drill the lesion (220) by means of an end portion having a large protrusion amount when the leading portion (20) rotates to drill the lesion (220). This can improve the passability of the medical device (100) through the lesion (220).
(5) In the above medical device (100), the first end portion (21) and the second end portion (22) of the leading portion (20) may be opposite to each other across a virtual line (VL) extended from the axis (Ax) of the main body part (10). According to this configuration, rotation of the leading portion (20) having the first end portion (21) and the second end portion (22) opposite to each other across the virtual line (VL) can more efficiently drill the lesion (220). This can improve the passability of the medical device (100) through the lesion (220).
(6) In the above medical device (100), a third end portion (25) opposite to the first end portion (21) across a hypothetical line (VL) extended from the axis (Ax) of the main body part (10) in the leading portion (20) may be located inside the outer peripheral surface (17) of the main body part (10) in the first direction. According to this configuration, the third end portion (25) opposite to the first end portion (21) in the leading portion (20) is located inside the outer peripheral surface (17) of the main body part (10), thereby preventing an outer diameter of the leading portion (20) from being excessively large. This in turn can prevent the leading portion (20) from unintentionally contacting a living body lumen (200) to damage the living body lumen (200).
(7) In the above medical device (100), the outer diameter of the leading portion (20) in a direction orthogonal to the axis (Ax) of the main body part (10) may be largest at a position of the first end portion (21) in a direction where the axis (Ax) extends. According to this configuration, rotation of the leading portion (20) having the first end portion (21) protruding in a direction having the largest outer diameter can more efficiently drill the lesion (220). This can further improve the passability of the medical device (100) through the lesion (220).
(8) In the above medical device (100), a largest outer diameter (Dx) of the leading portion (20) may be larger than a largest outer diameter (D1) of the distal end (16) of the main body part(10), and a thickness (T2) of the leading position (20) as an outer diameter of the leading portion (20) in a direction orthogonal to the direction of the largest outer diameter (Dx), may be smaller than the largest outer diameter (D1) of the distal end (16) of the main body part (10). According to this configuration, the leading portion (20) has a flattened shape, and the leading portion (20) can efficiently drill the lesion (220). This can further improve the passability of the medical device (100) through the lesion (220).
(9) In the above medical device (100), a difference between the largest outer diameter (Dx) of the leading portion (20) and the largest outer diameter (D1) of the distal end (16) of the main body part (10) may be 0.06 mm or more and 0.15 mm or less. According to this configuration, the passability of the medical device (100) through the lesion (220) can be improved.
(10) In the above medical device (100), the difference may be 0.07 mm or more and 0.15 mm or less. According to this configuration, the passability of the medical device (100) through the lesion (220) can be further improved.
(11) In the above medical device (100), the difference may be 0.08 mm or more and 0.15 mm or less. According to this configuration, the passability of the medical device (100) through the lesion (220) can be further improved.
(12) In the above medical device (100), a ratio of the largest outer diameter (Dx) of the leading portion (20) to the largest outer diameter (D1) of the distal end (16) of the main body part (10) may be 1.15 or more and 1.50 or less. According to this configuration, the passability of the medical device (100) through the lesion (220) can be improved.
(13) In the above medical device (100), the ratio may be 1.19 or more and 1.50 or less. According to this configuration, the passability of the medical device (100) through the lesion (220) can be further improved.
(14) In the medical device (100), the ratio may be 1.23 or more and 1.50 or less. According to this configuration, the passability of the medical device (100) through the lesion (220) can be further improved.
(15) A medical device (100) as disclosed herein includes a main body part (10) and a leading portion (20). The main body part (10) is elongated, and has a lesion discharge function. The leading portion (20) is connected to a distal end (16) of the main body part (10) and enters into a lesion (220). According to this medical device (100), a small piece of the lesion (220) generated by contact between the leading portion (20) and the lesion (220) is smoothly discharged from a distal end side toward a proximal end side of the main body part (10) so that the small piece does not interfere with drilling by the leading portion (20). This can improve the passability of the medical device (100) through the lesion (220).
(16) In the above medical device (100), the lesion discharge function may discharge a small piece of the lesion (220) generated by contact between the leading portion (20) and the lesion (220) from the distal end side toward the proximal end side of the main body part (10). According to this configuration, the passability of the medical device (100) through the lesion (220) can be improved.
(17) In the above medical device (100), the lesion discharge function may be achieved by virtue of a spiral groove (18) formed on the outer peripheral surface (17) of the main body part (10). According to this configuration, a small piece of the lesion (220) is smoothly discharged along the groove (18) from the distal end side toward the proximal end side of the main body part (10). This can improve the passability of the medical device (100) through the lesion (220).
(18) In the above medical device (100), the main body part (10) may include a coil (50), and the spiral groove (18) may be formed on an outer peripheral surface (52) of the coil (50). According to this configuration, a small piece of the lesion (220) can be discharged more smoothly along the groove (18) from the distal end side toward the proximal end side of the main body part (10). This can further improve the passability of the medical device (100) through the lesion (220).
(19) In the above medical device (100), a small piece of the lesion (220) may move along the groove (18) from the distal end side toward the proximal end side of the main body part (10). According to this configuration, the passability of the medical device (100) through the lesion (220) can be improved.
(20) In the above medical device (100), the lesion discharge function may be achieved by a structure in which a largest outer diameter (D1) of the distal end (16) of the main body part (10) is smaller than a largest outer diameter (Dx) of the leading portion (20). According to this configuration, a small piece of the lesion (220) is smoothly discharged along the outer peripheral surface of the main body part (10) from the distal end side toward the proximal end side of the main body part (10). This can improve the passability of the medical device (100) through the lesion (220).
(21) In the above medical device (100), the lesion discharge function may be achieved by a structure in which the main body part (10) has a tapered portion (14) that reduces in diameter from the proximal end side toward the distal end side. According to this configuration, a small piece of the lesion (220) is more smoothly discharged along the outer peripheral surface of the main body part (10) from the distal end side toward the proximal end side of the main body part (10). This can further improve the passability of the medical device (100) through the lesion (220).
(22) In the above medical device (100), the leading portion (20) may have an edge shaped so as to scrape the lesion (220) when rotated around its axis while pressed against the lesion (220). According to this configuration, a small piece of the lesion (220) scraped with the edge of the leading portion (20) can be smoothly discharged from the distal end side toward the proximal end side of the main body part (10). This can improve the passability of the medical device (100) through the lesion (220).
(23) In the above medical device (100), the leading portion may enter into a space created by scraping the lesion (220) with the edge of the leading portion (20) to form a through-hole in the lesion (220). According to this configuration, a small piece of the lesion (220) efficiently scraped with the edge of the leading portion (20) is smoothly discharged from the distal end side toward the proximal end side of the main body part (10), allowing the leading portion (20) to smoothly enter into the distal end side. This can further improve the passability of the medical device (100) through the lesion (220).
(24) A medical device (100) as disclosed herein includes a main body part (10) and a leading portion (20). The main body part (10) is elongated and extends along an axis (Ax). The leading portion (20) is connected to a distal end (16) of the main body part (10), and enters into a lesion (220). A lesion passability when the medical device (100) is rotated in a first rotational direction around the axis (Ax) differs from a lesion passability when the medical device (100) is rotated in a second rotational direction opposite to the first rotational direction. According to this medical device (100), the rotational directions of the medical device (100) can be selected according to required lesion passability, allowing a procedure to proceed carefully.
(25) In the above medical device (100), the lesion passability when the medical device (100) is rotated in the first rotational direction may be higher than the lesion passability when the medical device (100) is rotated in the second rotational direction, and a torque generated when the medical device (100) is rotated in the first rotational direction may be lower than a torque generated when the medical device (100) is rotated in the second rotational direction. According to this configuration, the medical device (100) may be rotated in the first rotational direction when high lesion passability is required, and the medical device (100) may be rotated in the second rotational direction when high torque is required. Accordingly, the rotational directions of the medical device (100) can be selected depending on the lesion passability and torque required.
(26) In the above medical device (100), a spiral groove (18) may be formed on an outer peripheral surface (17) of the main body part (10). According to this configuration, the presence of the spiral groove (18) enables different lesion passabilities when the medical device (100) is rotated in the first rotational direction or rotated in the second rotational direction.
(27) In the above medical device (100), the main body part (10) may include a coil (50), and the spiral groove (18) may be formed on an outer peripheral surface (52) of the coil (50). According to this configuration, the presence of the spiral groove (18) formed on the outer peripheral surface (52) of the coil (50) enables different lesion passabilities when the medical device (100) is rotated in the first rotational direction or rotated in the second rotational direction.
(28) In the above medical device (100), the first rotational direction may be a clockwise direction as centered around its rotation axis extending from a proximal end to a distal end of the main body part, and the spiral groove (18) may be formed on an outer peripheral surface (17) of the main body part (10), and the spiral groove (18) may be Z-wound, and the lesion passability when the medical device (100) is rotated in the first rotational direction may be higher than the lesion passability when the medical device (100) is rotated in the second rotational direction. According to this configuration, the presence of the spiral groove (18) enables the lesion passability when the medical device (100) is rotated in the first rotational direction to be higher than the lesion passability when the medical device (100) is rotated in the second rotational direction.
(29) In the above medical device (100), the torque generated when the medical device (100) is rotated in the first rotational direction may be lower than the torque generated when the medical device (100) is rotated in the second rotational direction. According to this configuration, the presence of the spiral groove (18) enables the torque generated when the medical device (100) is rotated in the first rotational direction to be lower than the torque generated when the medical device (100) is rotated in the second rotational direction.
(30) In the above medical device (100), the first rotational direction may be a clockwise direction as centered around its rotation axis extending from the proximal end to the distal end of the main body part, and the spiral groove (18) may be formed on the outer peripheral surface (17) of the main body part (10), and the spiral groove (18) may be S-wound, and the lesion passability when the medical device (100) is rotated in the first rotational direction may be lower than the lesion passability when the medical device (100) is rotated in the second rotational direction. According to this configuration, the presence of the spiral groove (18) enables the lesion passability when the medical device (100) is rotated in the first rotational direction to be lower than the lesion passability when rotated in the second rotational direction.
(31) In the above medical device (100), the torque generated when the medical device (100) is rotated in the first rotational direction may be higher than the torque generated when the medical device (100) is rotated in the second rotational direction. According to this configuration, the presence of the spiral groove (18) enables the torque generated when the medical device (100) is rotated in the first rotational direction to be higher than the torque generated when rotated in the second rotational direction.
(32) In the above medical device (100), the first rotational direction may be the clockwise direction as centered around its rotation axis extending from the proximal end to the distal end of the main body part, and the main body part (10) may include the coil (50), and the coil (50) may be Z-wound, and a pitch of the coil (50) may be widened when the coil (50) is rotated in the first rotational direction, and the lesion passability when the medical device (100) is rotated in the first rotational direction may be higher than the lesion passability when the medical device (100) is rotated in the second rotational direction. According to this configuration, the presence of the spiral coil (50) enables the lesion passability when the medical device (100) is rotated in the first rotational direction to be higher than the lesion passability when rotated in the second rotational direction.
(33) In the above medical device (100), the torque generated when the medical device (100) is rotated in the first rotational direction may be lower than the torque generated when the medical device (100) is rotated in the second rotational direction. According to this configuration, the presence of the spiral coil (50) enables the torque generated when the medical device (100) is rotated in the first rotational direction to be lower than the torque generated when rotated in the second rotational direction.
(34) In the above medical device (100), the first rotational direction may be the clockwise direction as centered around its rotation axis extending from the proximal end to the distal end of the main body part, and the main body part (10) may include the coil (50), and the coil (50) may be S-wound, and a pitch of the coil (50) may be narrowed when the coil (50) is rotated in the first rotational direction, and the lesion passability when the medical device (100) is rotated in the first rotational direction may be lower than the lesion passability when the medical device (100) is rotated in the second rotational direction. According to this configuration, the presence of the spiral coil (50) enables the lesion passability when the medical device (100) is rotated in the first rotational direction to be lower than the lesion passability when rotated in the second rotational direction.
(35) In the above medical device (100), the torque generated when the medical device (100) is rotated in the first rotational direction may be higher than the torque generated when the medical device (100) is rotated in the second rotational direction. According to this configuration, the presence of the spiral coil (50) enables the torque generated when the medical device (100) is rotated in the first rotational direction to be higher than the torque generated when rotated in the second rotational direction.
(36) In the above medical device (100), the leading portion (20) may have an edge shaped so as to scrape the lesion (220) when rotated around its axis while pressed against the lesion (220). According to this configuration, the lesion (220) can be efficiently drilled with the edge of the leading portion (20). This can improve the passability of the medical device (100) through the lesion (220).
(37) In the above medical device (100), the leading portion may enter into a space created by scraping the lesion (220) with the edge of the leading portion (20) to form a through-hole in the lesion (220). According to this configuration, the lesion (220) can be efficiently drilled with the edge of the leading portion (20), enabling the leading portion (20) to enter into the distal end side. This can further improve the passability of the medical device (100) through the lesion (220).
(38) A method of treatment as disclosed herein including: inserting a medical device (100) including an elongated main body part (10) extending along an axis (Ax) and a loop-shaped leading portion (20) connected to a distal end (16) of the main body part (10) into a living body lumen (200) from a side of the leading portion (20); and advancing the leading portion (20) though a lesion (220) while rotating the medical device (100) around the axis (Ax) within a living body lumen (200). According to this method of treatment, the lesion (220) can be efficiently drilled with the leading portion (20) which is rotating. This enables the lesion (220) to be treated efficiently.
(39) In the method of treatment, the living body lumen (200) may be a blood vessel of a human subject, and the lesion (220) may be located in the blood vessel at one of the legs of the human subject, and the insertion of the medical device (100) may include inserting the medical device (100) into the blood vessel from the leg in which the lesion (220) is located and advancing the medical device (100) in a direction of a flow of blood in the blood vessel. According to this method of treatment, the lesion (220) can be treated efficiently.
(40) In the method of treatment, the living body lumen (200) may be a blood vessel of a human subject, and the lesion (220) may be located in the blood vessel at one of the legs of the human subject, and the insertion of the medical device (100) may include inserting the medical device (100) into the blood vessel from either the ankle or the instep of the leg in which the lesion (220) is located, and advancing the medical device (100) in a direction opposite to the flow of blood in the blood vessel. According to this method of treating, the lesion (220) can be treated efficiently.
(41) In the above method of treatment, the living body lumen (200) may be a blood vessel of a human subject, and the lesion (220) may be located in the blood vessel at one of the legs of the human subject, and the insertion of the medical device (100) may include inserting the medical device (100) into the blood vessel from the other leg in which the lesion (220) is not located. According to this method of treatment, the lesion (220) can be treated efficiently.
(42) In the above method of treatment, the living body lumen (200) may be a blood vessel of a human subject, and the lesion (220) may be located in the blood vessel at one of the legs of the human subject, and the insertion of the medical device (100) may include inserting the medical device (100) into the blood vessel from an arm of the human subject. According to this method of treatment, the lesion (220) can be treated efficiently.
(43) In the above method of treatment, the advancement of the medical device (100) may include advancing the medical device (100) while rotating the medical device (100) in a first rotational direction and advancing the medical device (100) while rotating the medical device (100) in a second rotational direction opposite to the first rotational direction. According to this method of treatment, the lesion (220) can be more efficiently drilled with the leading portion (20) which is rotating. This enables the lesion (220) to be treated efficiently.
(44) In the above method of treatment, the advancement of the medical device (100) may include advancing the medical device (100) with the rotational direction of the guide wire 100 fixed to the first rotational direction. According to this method of treatment, the lesion can be treated by the medical device (100) with a fixed passability.
(45) In the above method of treatment, a length of the lesion (220) along an extension direction of the living body lumen (200) may be 100 mm or more. According to this method of treatment, the medical device (100) can be passed through the lesion (220) that would be otherwise relatively difficult for the medical device (100) to be passed through.
(46) In the above method of treatment, the advancement of the medical device (100) may include enabling a professional to grip the medical device (100) and rotate the medical device (100). According to this method of treatment, the professional can use the medical device (100) with her or his intended passability when performing treatment.
(47) In the above method of treatment, the advancement of the medical device (100) may include rotating the medical device (100) using a driving force from an actuator (182). According to this method of treatment, the medical device (100) can be stably rotated, effectively improving the passability of the medical device (100) through the lesion (220). According to this method of treatment, the professional does not need to rotate the medical device (100), allowing her or him to concentrate on other procedures. This can improve the ease of the procedures.
(48) In the above method of treatment, the lesion (220) may be a highly calcified lesion. According to this method of treatment, the medical device (100) can be passed through the lesion (220) that would be otherwise relatively difficult for the medical device (100) to be passed through.
(49) In the above method of treatment, prior to the insertion of the medical device (100), a sheath may be inserted into the living body lumen (200), and the medical device (100) may be then passed through inside the sheath to allow the medical device (100) to be inserted into the living body lumen (200). According to this method of treatment, the medical device (100) can be smoothly inserted into the living body lumen (200).
(50) In the above method of treatment, prior to the insertion of the medical device (100), a guide wire (110) may be inserted into the living body lumen (200), and a catheter (120) may be inserted into the living body lumen (200) along the guidewire (110), and the guidewire (110) may be then removed from the living body lumen (200), and then the medical device (100) may be inserted into the catheter (120) which remains inserted into the living body lumen (200). According to this method of treatment, treatments with the medical device (100) can be efficiently performed.
(51) In the above method of treatment, the advancement of the medical device (100) may be performed in a situation where none of other medical devices are passed through the lesion (220). According to this method of treatment, the medical device (100) can be passed through the lesion (220) that would be otherwise relatively difficult for the medical device (100) to be passed through.
(52) In the above method of treatment, after the medical device (100) is passed through the lesion (220), a guide wire may be passed through the lesion (220) and an atherectomy device may be then inserted along the guide wire. According to this method of treatment, treatments with the atherectomy device can be efficiently performed.
(53) In the above method of treatment, after the medical device (100) is passed through the lesion (220), a guide wire may be passed through the lesion (220), and a balloon catheter may be then inserted along the guide wire. According to this method of treatment, treatments with a balloon catheter can be efficiently performed.
(54) In the above method of treatment, after the medical device (100) is passed through the lesion (220), a guide wire may be passed through the lesion (220), and a stent may be then inserted along the guide wire. According to this method of treatment, treatments with the stent can be efficiently performed.
(55) In the above method of treatment, the leading portion (20) may include an edge shaped so as to scrape the lesion (220) when rotated around its axis while pressed against the lesion (220). According to this method of treating, the lesion (220) can be efficiently drilled with the edge of the leading portion (20). This enables the lesion (220) to be treated efficiently.
(56) In the above method of treatment, the medical device (100) may have the leading portion (20) for forming a through-hole in the lesion (220) by the leading portion (20) entering into a space created by scraping the lesion (220) with the edge of the leading portion (20). According to this method of treatment, the lesion (220) can be efficiently drilled with the edge of the leading portion (20), enabling the leading portion to enter into the distal end side. This enables the lesion (220) to be treated efficiently.
(57) A medical device (100) as disclosed herein includes an elongated main body part (10) and a leading portion (20). The leading portion (20) is connected to a distal end (16) of the main body part (10), and enters into a lesion (220). The leading portion (20) has a loop-shaped structure formed with a portion of a wire (40). The main body part (10) includes another portion of the wire (40). The wire (40) has a first portion (P1) having a first thickness and a second portion (P2) having a second thickness thinner than the first thickness. A thickness direction of the wire (40) is orthogonal to a direction of a largest outer diameter (Dx) of the leading portion (20). According to this medical device (100), a desired property can be given to each of the portions of the wire (40) which form the leading portion (20) and the main body part (10).
(58) In the above medical device (100), the first portion (P1) and the second portion (P2) may be present in the leading portion (20). According to this configuration, a desired property can be given to each of the portions of the wire (40) which form the leading portion (20).
(59) In the above medical device (100), the second portion (P2) may be a portion in which a groove (26) concave in the thickness direction is formed. According to this configuration, the drillability of the leading portion (20) can be improved by arranging the second portion (P2) which is relatively thin in a region of the wire (40) which forms the leading portion (20). As a result, the passability of the medical device (100) through the lesion (220) can be improved. According to this configuration, when a tensile force is applied to the leading portion (20), the second portion (P2) which is relatively thin is preferentially subjected to fracturing, thereby preventing detachment of the leading portion (20) from the main body part (10).
(60) In the above medical device (100), the second portion (P2) may correspond to at least one end portion of the leading portion (20) in the direction of the largest outer diameter (Dx) of the leading portion (20). According to this configuration, the drillability of the leading portion (20) can be effectively improved because of the presence of the second portion (P2) which is relatively thin at an end portion of the leading portion (20) in the direction of the largest outer diameter of the leading portion (20). As a result, the passability of the medical device (100) through the lesion (220) can be improved.
(61) In the above medical device (100), the second portion (P2) may correspond to a distal end portion of the leading portion (20). According to this configuration, when a tensile force is applied to the leading portion (20), the distal end portion of the leading portion (20) is preferentially subjected to fracturing, thereby more reliably preventing detachment of the leading portion (20) from the main body part (10).
(62) A medical device (100) as disclosed herein includes an elongated main body part (10) and a leading portion (20). The leading portion (20) is connected to a distal end (16) of the main body part (10), and enters into a lesion (220). The leading portion (20) has a loop-shaped structure formed with a portion of a wire (40). The main body part (10) includes another portion of the wire (40). The wire (40) has a first portion (P1) having a first cross section (CS1) and a second portion (P2) having a second cross section (CS2) different in shape from the first cross section (CS1). According to this medical device (100), a desired property can be given to each of the portions of the wire (40) which form the leading portion (20) and the main body part (10).
(63) In the above medical device (100), the area of the first cross section (CS1) may be different from that of the second cross section (CS2). According to this configuration, a desired property can be given to each of the portions of the wire (40) which form the leading portion (20) and the main body part (10).
(64) In the above medical device (100), a cross section of the wire (40) included in the main body part (10) may be circular in shape, and the circularity of the first cross section (CS1) may be different from that of the second cross section (CS2). According to this configuration, a desired property can be given to each of the portions of the wire (40) which form the leading portion (20) and the main body part (10).
(65) In the above medical device (100), the first portion (P1) may be provided in the main body part (10), and the second portion (P2) may be provided in the leading portion (20). According to this configuration, a desired property can be given to each of the portions of the wire (40) which form the leading portion (20) and the main body part (10).
(66) In the above medical device (100), the cross section of the wire (40) included in the main body part (10) may be circular in shape, and the first portion (P1) may be present in the leading portion (20), and a curvature of an outer peripheral side and a curvature of an inner peripheral side are different from each other along an outline of the first cross section (CS1). According to this configuration, a desired property can be given to each of the portions of the wire (40) which form the leading portion (20).
(67) In the above medical device (100), the curvature of the outer peripheral side along the outline of the first cross section (CS1) may be smaller than that of the inner peripheral side. According to this configuration, the first cross section (CS1) can have a highly drillable shape. This effectively improve the passability of the medical device (100) through the lesion (220).
(68) In the above medical device (100), an outline of a cross section of the wire included in the main body part (10) may consist of an arc line and a line segment connecting the end portions of the arc line, and with respect to a ratio of a height in a direction orthogonal a direction of a largest width of the cross section to the largest width, the ratio in the first cross-section (CS1) may be different from that in the second cross section (CS2). According to this configuration, an area occupied by the wire (40) in a cross section of the medical device (100) can be increased. This can increase a distal end load of the medical device (100), effectively improving the passability of the medical device (100) through the lesion (220).
(69) In the above medical device (100), an outline of the cross section of the wire (40) included in the main body part (10) may include a first straight line portion, and a portion corresponding to the first straight line portion may be a curved line in an outline of a cross section of the wire (40) included in the leading portion (20). According to this configuration, the cross section of the leading portion (20) can have a highly drillable shape. This can effectively improve the passability of the medical device (100) through the lesion (220).
(70) In the above medical device (100), the outline of the cross section of the wire (40) included in the main body part (10) may comprise a second straight line portion. According to this configuration, a desired property can be given to each of the portions of the wire (40) which form the main body part (10).
(71) In the above medical device (100), the first straight line portion and the second straight line portion may be opposite to each other across the center of gravity of the cross section of the wire (40). According to this configuration, a desired property can be given to each of the portions of the wire (40) which form the main body part (10).
(72) In the above medical device (100), a portion of an outer peripheral side is concave, and a portion of an inner peripheral side may be convex in the outline of the cross section of the above wire (40) included in the leading portion (20). According to this configuration, the cross section of the leading portion (20) can have a highly drillable shape. This can effectively improve the passability of the medical device (100) through the lesion (220).
(73) A medical device (100) as disclosed herein includes an elongated main body part (10) and a leading portion (20). The leading portion (20) is connected to a distal end (16) of the main body part (10), and enters the lesion (220). The leading portion (20) has a loop-shaped structure formed with a portion of a wire (40). The main body part (10) includes another portion of the wire (40). The wire (40) has a specific portion (P3, P1). An outline of a cross section of the specific portion (P3, P1) consists of an arc line and a line segment connecting the end portions of the arc line. According to this medical device (100), an area occupied by the wire (40) in a cross section of the medical device (100) can be increased. This can increase a distal end load of the medical device (100), effectively improving the passability of the medical device (100) through the lesion (220).
(74) In the above medical device (100), the specific portion (P3) may be present in the main body part (10). According to this configuration, an area occupied by the wire (40) in a cross section of the main body part (10) can be increased. This can increase a distal end load of the medical device (100), effectively improving the passability of the medical device (100) through the lesion (220).
(75) In the above medical device (100), the specific portion (P1) may be present in the leading portion (20). According to this configuration, the cross section of the leading portion (20) can have a highly drillable shape. This can effectively improve the passability of the medical device (100) through the lesion (220).
(76) In the above medical device (100), a largest width of the cross section of the specific portion (P1) may be larger than a length of the line segment. According to this configuration, the cross section of the leading portion (20) can have a highly drillable shape. This can effectively improve the passability of the medical device (100) through the lesion (220).
(77) In the above medical device (100), the largest width of the cross section of the specific portion (P1) may be substantially identical to the length of the line segment. According to this configuration, the cross section of the leading portion (20) can have a highly drillable shape. This can effectively improve the passability of the medical device (100) through the lesion (220).
(78) A medical device (100) as disclosed herein includes an elongated main body part (10) and a leading portion (20). The main body part (10) has a first rigid portion (11) having a first rigidity and a second rigid portion (12) located at a proximal end side of the first rigid portion (11) and having a second rigidity lower than the first rigidity. The leading portion (20) is connected to a distal end (16) of the main body part (10), and enters into a lesion (220). According to this medical device (100), the presence of the second rigid portion (12) having relatively lower rigidity can ensure softness of the main body part (10) while the presence of the first rigid portion (11) located at a distal end side of the second rigid portion (12) can improve the rotation transmissibility from the main body part (10) to the leading portion (20). This can improve the passability of the medical device (100) through the lesion (220).
(79) In the above medical device (100), the main body part (10) may have a third rigid portion (13) located at a proximal end side of the second rigid portion (12) and having a third rigidity higher than the second rigidity. According to this configuration, the rotation transmissibility can be improved from the proximal end portion toward the distal end portion of the main body part (10).
(80) In the above medical device (100), the first rigidity may be equal to or lower than the third rigidity. According to this configuration, an excessive rigidity of the distal end portion of the main body part (10) can be prevented, which in turn can prevent the occurrence of kinking at the distal end portion of the main body part (10).
(81) In the above medical device (100), the first rigidity may be higher than the third rigidity. According to this configuration, the rotation transmissibility from the main body part (10) to the leading portion (20) can be increased effectively. This can efficiently improve the passability of the medical device (100) through the lesion (220).
(82) In the above medical device (100), the leading portion (20) may have a loop-shaped structure formed with a part of a base portion (40B) and a part of a folded-back portion (40A) of a wire (40) including the base portion (40B) and the folded-back portion (40A) folded back from the base portion (40B), and the main body part (10) may include a part of the base portion (40B) and a part of the folded-back portion (40A) of the wire (40), and the first rigid portion (11) may be a portion of the main body part (10) which includes the folded-back portion (40A) of the wire (40). According to this configuration, the main body part (10) having the first rigid portion (11) and the second rigid portion (12), which have different rigidity each other, can be obtained while avoiding structural complexity.
(83) In the above medical device (100), the base portion (40B) may have a tapered portion (42, 44) having a largest width decreased toward a distal end side of the main body part (10). According to this configuration, the rigidity of the medical device (100) can be gradually reduced toward the distal end side.
(84) In the above medical device (100), the tapered portion may be a first tapered portion (42), and the base portion (40B) may have a second tapered portion (44) provided at a distal end side of the first tapered portion (42) and having a largest width decreased toward the distal end side of the main body part (10) and having a slope different from that of the first tapered portion (42). According to this configuration, the medical device (100) can be designed to have a more preferred rigidity at each position along an axis (Ax) of the medical device (100).
(85) In the above medical device (100), the main body part (10) may have a parallel portion (11) in which the base portion (40B) and the folded-back portion (40A) extend along the axis (Ax) of the main body part (10), and the base portion (40B) and the folded-back portion (40A) are spaced in the parallel section (11). According to this configuration, the presence of the parallel portion (11) enables the medical device (100) to have a more preferred rigidity at each position along the axis (Ax) of the medical device (100).
(86) A medical device (100) as disclosed herein includes an elongated main body part (10) and a leading portion (20). The leading portion (20) is connected to a distal end (16) of the main body part (10), and enters into a lesion (220). The leading portion (20) has a reinforcing portion (28) located at a connection site with the main body part (10). According to this medical device (100), detachment of the leading portion (20) from the main body part (10) can be prevented.
(87) In the above medical device (100), the leading portion (20) may include a wire (40), and the main body part (10) may include a coil (50) joined to the wire (40) via a joining material (61), and the reinforcing portion (28) may be formed with a portion of the joining material (61). According to this configuration, detachment of the leading portion (20) from the main body part (10) can be prevented while avoiding structural complexity.
(88) In the above medical device (100), the joining material (61) may be provided inside the coil (50). According to this configuration, the reinforcement effect by the joining material (61) can be improved, and detachment of the leading portion (20) from the main body part (10) can be effectively prevented.
(89) In the above medical device (100), the leading portion (20) may have a loop-shaped structure formed with a part of a base portion (40B) and a part of a folded-back portion (40A) of a wire (40) including the base portion (40B) and the folded-back portion (40A) folded back from the base portion (40B), and the main body part (10) may include a part of the base portion (40B) and a part of the folded-back portion (40A) of the wire (40), and the joining portion (61) may be provided between the base portion (40B) and the folded-back portion (40A). According to this configuration, the base portion (40B) and the folded-back portion (40A) can be joined via the joining material (61) as the reinforcing portion (28). This can simplify the structure.
(90) In the above medical device (100), the main body part (10) may include a joining region (R1) in which a part of the base portion (40B), a part of the folded-back portion (40A), and the main body part (10) are joined, and a non-joining region (R2) in which a part of the base portion (40B), a part of the folded-back portion (40A), and the main body part (10) are not joined. According to this configuration, the presence of the non-joining region (R2) can ensure the softness of the main body part (10) while the presence of the joining region (R1) can ensure the joining between the wire (40) and a tubular body (50).
(91) In the above medical device (100), a width of the reinforcing portion (28) in a direction orthogonal to an axis (Ax) of the main body part (10) may be decreased toward a distal end of the reinforcing portion (28). According to this configuration, an excessive rigidity of the connection site between the main body part (10) and the leading portion (20) can be prevented, which in turn can prevent the occurrence of kinking at that site.
(92) In the above medical device (100), a width of the reinforcing portion (28) in a direction orthogonal to the axis (Ax) of the main body part (10) may be increased toward the distal end of the reinforcing portion (28). According to this configuration, detachment of the leading portion (20) from the main body part (10) can be effectively prevented.
(93) In the above medical device (100), an outer peripheral surface of the reinforcing portion (28) may have a curved surface recessed toward a radially inward side of the reinforcing portion (28). According to this configuration, excessive rigidity at the connection site between the main body part (10) and the leading portion (20) can be prevented while detachment of the leading portion (20) from the main body part (10) can be effectively prevented.
(94) In the above medical device, the reinforcing portion (28) may be formed with a welded portion of the leading portion (20) and the main body part (10). According to this configuration, detachment of the leading portion (20) from the main body part (10) can be effectively prevented.
(95) A medical device (100) as disclosed herein includes a main body part (10) and a leading portion (20). The main body part (10) is an elongated along an axis (Ax), and has a curved portion (19). The leading portion (20) is connected to a distal end (16) of the main body part (10), and enters into a lesion (220). According to this medical device (100), the leading portion (20) can be easily delivered to a location of a lesion (220) within a living body lumen (200).
(96) In the above medical device (100), the above leading portion (20) may have a largest outer diameter (Dx) in a first direction orthogonal to the axis (Ax), and an angle between a first virtual line (VL1) parallel to the first direction and a first virtual plane (VP) encompassing the axis (Ax) in the curved portion (19) of the main body part (10) may be 80 degrees or less. According to this configuration, the drillability with the leading portion (20) can be improved. This can improve the passability of the medical device (100) through the lesion (220).
(97) In the above medical device (100), the angle may be 10 degrees or less. According to this configuration, the drillability with the leading portion (20) can be further improved. This can further improve the passability of the medical device (100) through the lesion (220).
(98) In the above medical device (100), the first virtual line (VL1) and the first virtual plane (VP) may be substantially parallel. According to this configuration, the drillability with the leading portion (20) can be further improved. This can further improve the passability of the medical device (100) through the lesion (220).
(99) In the above medical device (100), the first virtual line (VL1) and the first virtual plane (VP) may be orthogonal. According to this configuration, the controllability of the medical device (100) within the lesion (220) can be effectively improved.
(100) A medical device (100) as disclosed herein includes an elongated main body part (10), a leading portion (20), a first coating (31), and a second coating (32). The leading portion (20) is connected to a distal end (16) of the main body part (10), and enters into a lesion (220). The first coating (31) has a first slidability, and covers at least a part of the leading portion (20). The second coating (32) has a second slidability different from the first slidability, and covers at least a part of the main body part (10). According to this medical device (100), an appropriate slidability can be given to each of the portions of the medical device (100).
(101) In the above medical device (100), the first slidability may be lower than the second slidability. According to this configuration, the slidability of the leading portion (20) can be relatively low while ensuring the slidability of the main body part (10). This can increase the drillability with the leading portion (20), and can improve the passability of the medical device (100) through the lesion (220).
(102) In the above medical device (100), the first coating (31) may be hydrophobic, and the second coating (32) may be hydrophilic. According to this configuration, the first slidability of the first coating (31) can be lower than the second slidability of the second coating (32).
(103) In the above medical device (100), a distal end portion of the main body part (10) may be covered with the first coating (31). According to this configuration, the slidability of the distal end portion of the main body part (10) which enters into the lesion (220) when performing a procedure can be relatively lower. This can effectively improve the passability of the medical device (100) through the lesion (220).
(104) A medical device (100) as disclosed herein includes an elongated main body part (10) and a leading portion (20). The main body part (10) includes a tubular body (50). The leading portion (20) is connected to a distal end (16) of the main body part (10), and includes a loop-shaped wire (40), and enters into a lesion (220). A method of manufacturing the medical device (100) as disclosed herein includes: performing a bending work of pressing the wire (40) against a pin to bend the wire (40) into a loop-shaped structure; and joining the wire (40) to the tubular body (50). According to this method of manufacture, the wire (40) can be processed stably into a desired loop-shaped structure, enabling effective manufacturing of the medical device (100) having the leading portion (20) with a loop-shaped structure.
(105) The above method of manufacture may comprise inserting the wire (40) into a hollow space of the tubular body (50) prior to the bending work. According to this method of manufacture, an operation of inserting the wire (40) into the hollow space of the tubular body (50) can be efficiently performed, which in turn enables the medical device (100) to be efficiently manufactured.
(106) In the above method of manufacture, the joining may include using a soldering material. According to this method of manufacture, the wire (40) can be reliably joined to the tubular body (50).
(107) In the above method of manufacture, the tubular body (50) may be a coil (50), and the joining may include feeding the soldering material into a hollow space of the coil (50) through a pitch portion of the coil (50). According to this method of manufacture, the wire (40) can be more reliably joined to the coil (50).
(108) In the above method of manufacture, the leading portion (20) may have a reinforcing portion (28) located at a connection site with the main body part (10), and the joining may include forming the reinforcing portion (28) with the soldering material. According to this method of manufacture, the reinforcing portion (28) integrated with the joining portion can be formed, effectively preventing the detachment of the leading portion (20) from the main body part (10).
(109) In the above method of manufacture, the bending work may include deforming a cross section of the wire (40). According to this method of manufacture, a desired property can be given to each of the portions of the wire (40) which form the leading portion (20) by changing a shape of a cross section at each portion of the wire (40).
(110) In the above method of manufacture, a narrowed portion (29) may be formed at a proximal end portion of the leading portion (20), and the bending work may include forming the narrowed portion (29). According to this method of manufacture, an excessive rigidity of the connection site between the main body part (10) and the leading portion (20) can be prevented, which in turn can prevent the occurrence of kinking at that site.
(111) The above method of manufacture may comprise performing grinding work of the wire (40) prior to the bending work. According to this method of manufacture, a desired property can be given to each of the portions of the wire (40) which form the leading portion (20) by changing an outer diameter and/or a shape of a cross section at each of the portions of the wire (40).
(112) The above method of manufacture may comprise bending a distal end portion of the main body part (10) after the joining. According to this method of manufacture, the medical device (100) which can easily deliver the leading portion (20) to a location of the lesion (220) in a living body lumen (200) can be easily manufactured.
(113) A medical device (100) as disclosed herein includes an elongated main body part (10) and a leading portion (20). The leading portion (20) is connected to a distal end (16) of the main body part (10), and enters into a lesion (220). A leading portion (20) has a loop-shaped structure formed with a portion of a wire (40), and includes an intersecting portion (49) in a proximal end portion in which the wire (40) intersects. According to this medical device (100), the rigidity of the proximal end portion of the leading portion (20) can be increased, enabling the leading portion (20) to drill the lesion (220) efficiently. This can improve the passability of the medical device (100) through the lesion (220).
(114) In the above medical device (100), the leading portion (20) may have a reinforcing portion (28) located at a connection site with the main body part (10), and a part of the reinforcing portion (28) may be a joining material covering an outer periphery of the intersecting portion (49). According to this configuration, the rigidity of the proximal end portion of the leading portion (20) can be effectively increased. This can improve the passability of the medical device (100) through the lesion (220).

The technology as disclosed herein can be embodied according to various aspects, for example, according to the aspects of medical devices, methods of manufacturing the medical devices, and methods of treatment using the medical devices.

### BRIEF DESCRIPTION OF THE DRAWINGS

FIG. 1 shows a diagram schematically illustrating a configuration of a guide wire according to a first embodiment.
FIG. 2 shows a diagram schematically illustrating a configuration of the guide wire according to the first embodiment.
FIG. 3 shows a diagram schematically illustrating a configuration of the guide wire according to the first embodiment.
FIG. 4 shows a diagram schematically illustrating a configuration of the guide wire according to the first embodiment.
FIG. 5 shows a diagram schematically illustrating a configuration of the guide wire according to the first embodiment.
FIG. 6 shows a diagram schematically illustrating a configuration of the guide wire according to the first embodiment.
FIG. 7 shows a flow chart of an example of a method of treatment using the guide wire.
FIG. 8 shows a diagram illustrating an example of the method of treatment using the guide wire.
FIG. 9 shows a diagram illustrating an example of the method of treatment using the guide wire.
FIG. 10 shows a diagram illustrating an example of the method of treatment using the guide wire.
FIG. 11 shows a diagram illustrating an example of the method of treatment using the guide wire.
FIG. 12 shows a diagram illustrating an example of the method of treatment using the guide wire.
FIG. 13 shows a diagram illustrating an example of the method of treatment using the guide wire.
FIG. 14 shows a diagram illustrating an example of the method of treatment using the guide wire.
FIG. 15 shows a diagram illustrating an example of the method of treatment using the guide wire.
FIG. 16 shows a diagram illustrating a cross section of a wire.
FIG. 17 shows a diagram illustrating a cross section of the wire.
FIG. 18 shows a flow chart of an example of the method manufacturing of the guide wire.
FIG. 19 shows a diagram illustrating an example of the method manufacturing of the guide wire.
FIG. 20 shows a diagram illustrating an example of the method manufacturing of the guide wire.
FIG. 21 shows a diagram illustrating an example of the method manufacturing of the guide wire.
FIG. 22 shows a diagram schematically illustrating a configuration of a guide wire according to a second embodiment.
FIG. 23 shows a diagram schematically illustrating a configuration of a guide wire according to a third embodiment.
FIG. 24 shows a diagram schematically illustrating a configuration of a guide wire according to a fourth embodiment.
FIG. 25 shows a diagram schematically illustrating a configuration of a guide wire according to a fifth embodiment.
FIG. 26 shows a diagram schematically illustrating a configuration of a guide wire according to a sixth embodiment.
FIG. 27 shows a diagram schematically illustrating a configuration of a guide wire according to a seventh embodiment.
FIG. 28 shows a diagram schematically illustrating a configuration of a guide wire according to an eighth embodiment.
FIG. 29 shows a diagram schematically illustrating a configuration of the guide wire according to the eighth embodiment.
FIG. 30 shows a diagram schematically illustrating a configuration of a guide wire according to a variation of the eighth embodiment.
FIG. 31 shows a diagram schematically illustrating a configuration of a guide wire according to a variation of the eighth embodiment.
FIG. 32 shows a diagram schematically illustrating a configuration of a guide wire according to another variation of the eighth embodiment.
FIG. 33 shows a diagram schematically illustrating a configuration of a guide wire according to another variation of the eighth embodiment.
FIG. 34 shows a diagram schematically illustrating a configuration of a guide wire according to the ninth embodiment.
FIG. 35 shows a diagram schematically illustrating a configuration of the guide wire according to the ninth embodiment.
FIG. 36 shows a diagram schematically illustrating a cross section of a wire of the guide wire according to the ninth embodiment.
FIG. 37 shows a diagram schematically illustrating a cross section of a wire of the guide wire according to the ninth embodiment.
FIG. 38 shows a diagram schematically illustrating a cross section of a wire of a variation of the guide wire according to the ninth embodiment.
FIG. 39 shows a diagram schematically illustrating a cross section of a wire of another variation of the guide wire according to the ninth embodiment.
FIG. 40 shows a diagram schematically illustrating a configuration of a guide wire according to a tenth embodiment.
FIG. 41 shows a diagram schematically illustrating a configuration of the guide wire according to the tenth embodiment.
FIG. 42 shows a diagram illustrating a cross section of a wire of the guide wire according to the tenth embodiment.
FIG. 43 shows a diagram illustrating a cross section of a wire of the guide wire according to the tenth embodiment.
FIG. 44 shows a diagram schematically illustrating a configuration of a guide wire according to an eleventh embodiment.
FIG. 45 shows a diagram schematically illustrating a configuration of the guide wire according to the eleventh embodiment.
FIG. 46 shows a diagram illustrating a method of treatment using a guide wire according to a twelfth embodiment.
FIG. 47 shows a diagram schematically illustrating a configuration of a guide wire according to a thirteenth embodiment.
FIG. 48 shows a diagram schematically illustrating a configuration of a guide wire according to a fourteenth embodiment.
FIG. 49 shows a diagram illustrating a method of evaluation.
Fig. 50 shows a plot illustrating results from evaluation.
Fig. 51 shows a plot illustrating results from evaluation.

### DESCRIPTION OF EMBODIMENTS

### (General configuration of guide wire 100)

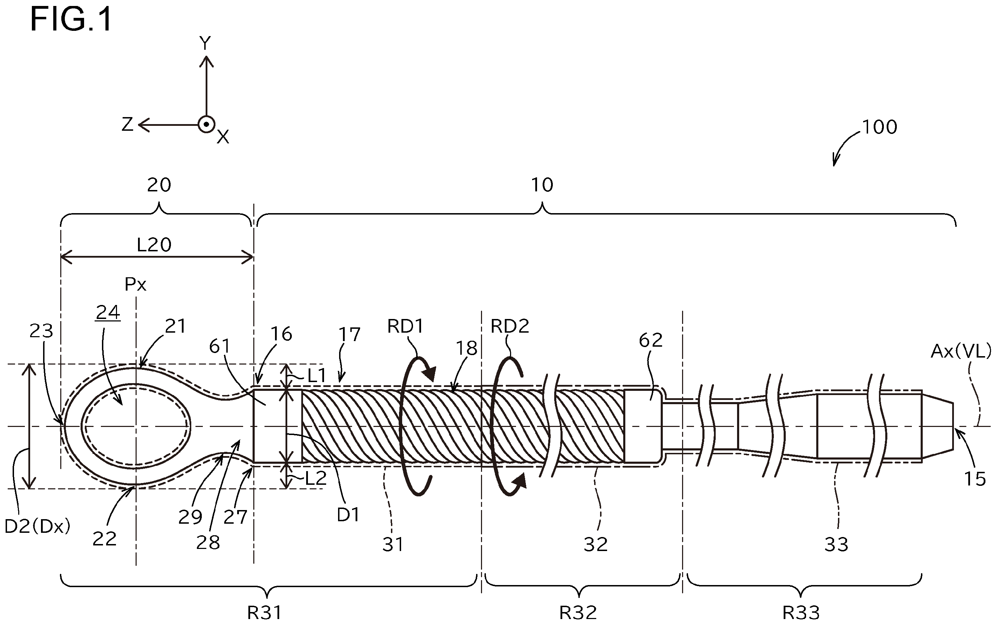

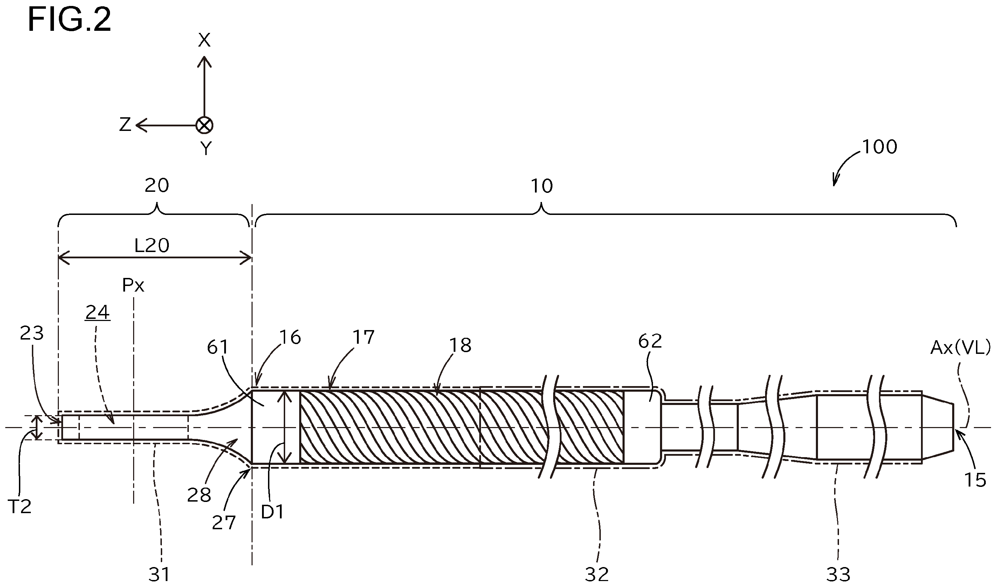

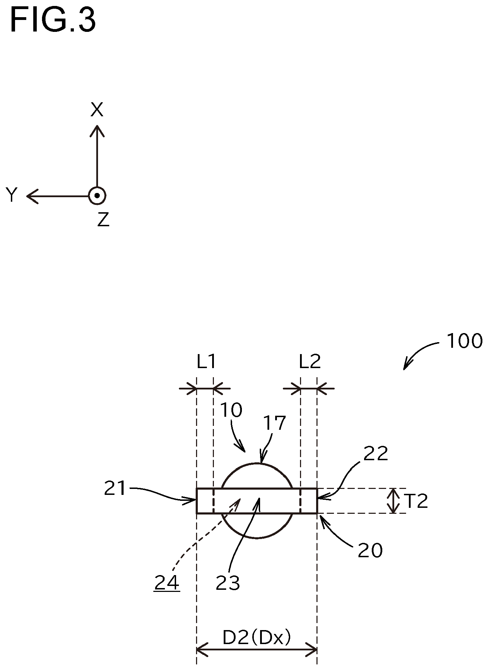

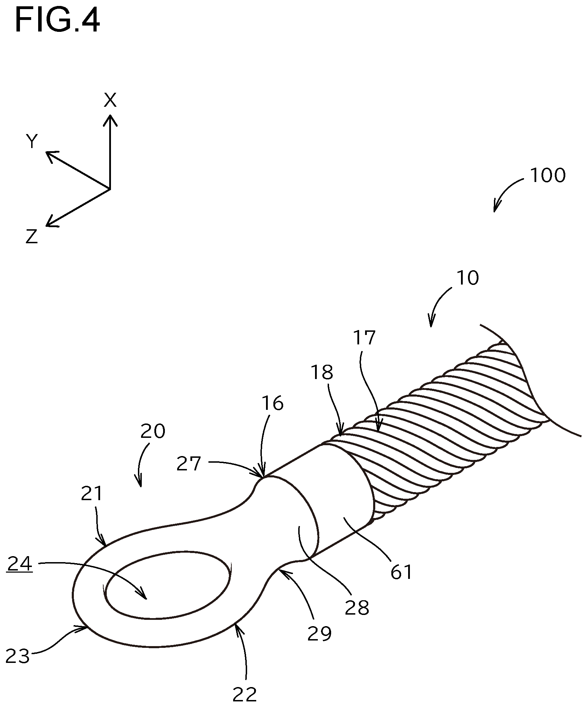

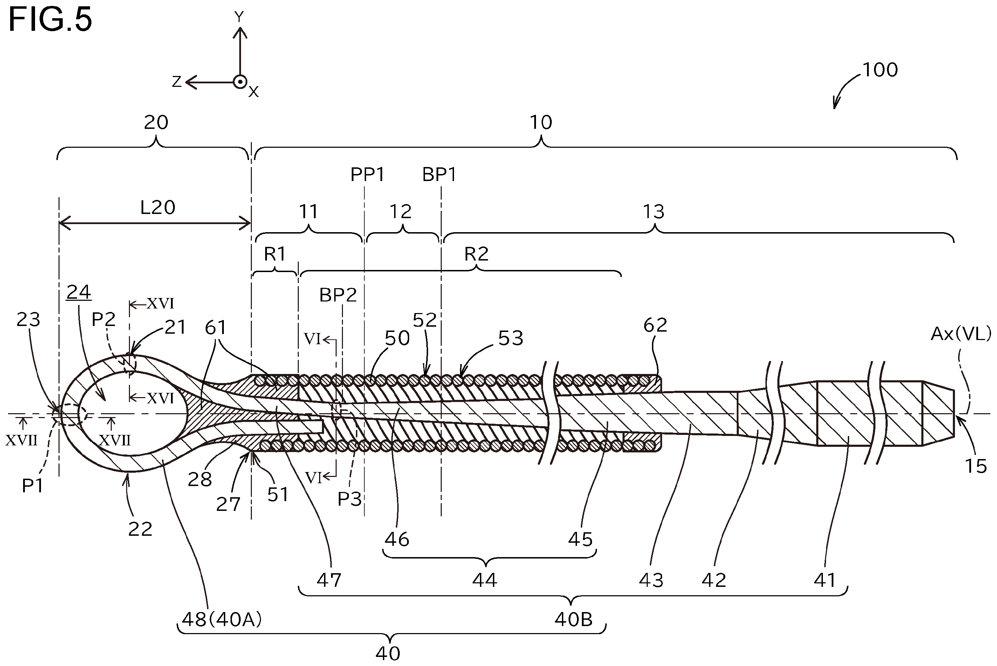

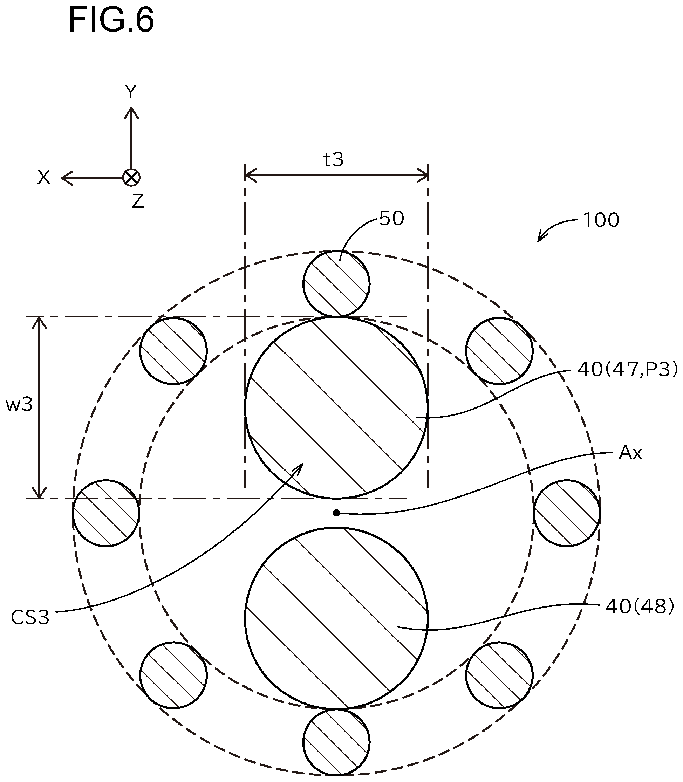

FIGs. 1 to 6 show diagrams schematically illustrating configurations of a guide wire 100 according to a first embodiment. FIG. 1 shows an appearance of the guide wire 100 viewed from a direction of the X-axis. FIG. 2 shows an appearance of the guide wire 100 viewed from a direction of the Y-axis. FIG. 3 shows an appearance of the guide wire 100 viewed from a direction of the Z-axis. FIG. 4 shows a perspective view of an appearance of a distal end portion of the guide wire 100. FIG. 5 shows a Y-Z longitudinal section of the guide wire 100. FIG. 6 shows an X-Y cross section of the guide wire 100 along a VI-VI position in FIG. 5. In the guide wire 100, the positive side of the Z-axis corresponds to a distal end side (far side) that will be inserted into the body, and the negative side of the Z-axis corresponds to a proximal end side (near side) that will be manipulated by a professional. In each view, a portion of the guide wire 100 may be omitted. FIGs. 1, 2, and 5 show the guide wire 100 lies linearly extended in a direction parallel to the Z-axis. The guide wire 100 is flexible enough to be curved. These will apply to the following figures.

As used herein, in the guide wire 100 and component members thereof, an end of the distal end side is referred to as a "distal end", and the distal end and the vicinity thereof are referred to as a "distal end portion", and an end of the proximal end side is referred to as a "proximal end", and the proximal end and the vicinity thereof are referred to as a "distal end portion". Cross sections of the guide wire 100 and component members thereof are meant to be sections orthogonal to the longitudinal direction. Longitudinal sections of the guide wire 100 and component members thereof are meant to be sections parallel to the center axis in the longitudinal direction. In the guide wire 100 and component members thereof, a direction orthogonal to the longitudinal direction is called a radial direction. Outer diameters of the guide wire 100 and component members thereof are meant to be widths along the radial direction.

The guide wire 100 is an elongated medical device to be inserted into a blood vessel. The entire length of the guide wire 100 is, for example, 1000 mm or more and 3000 mm or less.

The guide wire 100 has a main body part 10 and a leading portion 20.

The main body part 10 is an elongated portion extending along a center axis Ax. In this embodiment, the main body part 10 includes a wire 40 and a coil 50 (see FIG. 5). In this embodiment, the center axis Ax of the main body part 10 coincides with the center axis of the guide wire 100. A proximal end 15 of the main body part 10 coincides with a proximal end of the guide wire 100. A spiral groove 18 is formed on an outer peripheral surface 17 of a distal end portion of the main body part 10.

The leading portion 20 is connected to a distal end 16 of the main body part 10. The leading portion 20 can be expressed as a guiding portion, a drilling portion, a fracturing portion, a peeling portion, an entering portion, a peeler, a shaver, and the like. A distal end 23 of the leading portion 20 coincides with a distal end of the guide wire 100. The leading portion 20 has a loop-shaped structure which encloses a through-hole 24 extending in a direction of the X axis. In the following descriptions, a surface visible when viewing the leading portion 20 from the positive direction of the X axis is, for convenience, referred to as an upper surface, and a surface visible when viewing the leading portion 20 from the negative direction of the X axis direction is referred to as a lower surface. Surfaces of the leading portion 20 may or may not have an edge. The term "edge" refers to a boundary (a ridge line) of two surfaces. The leading portion 20 enters into a lesion while rotating around the center axis Ax. Entering of the leading portion 20 into the lesion may be expressed as crossing/passing through the lesion, drilling the lesion, fracturing the lesion, peeling the lesion, pushing through the lesion, diving into the lesion, advancing into the lesion, and the like. A length L20 of the leading portion 20 along with the center axis Ax is, for example, 0.2 mm or more and 2.0 mm or less. The length L20 of the leading portion 20 may be 0.3 mm or more and 1.5 mm or less, or may be 0.4 mm or more and 1.0 mm or less.

### (Method of treatment using guide wire 100)

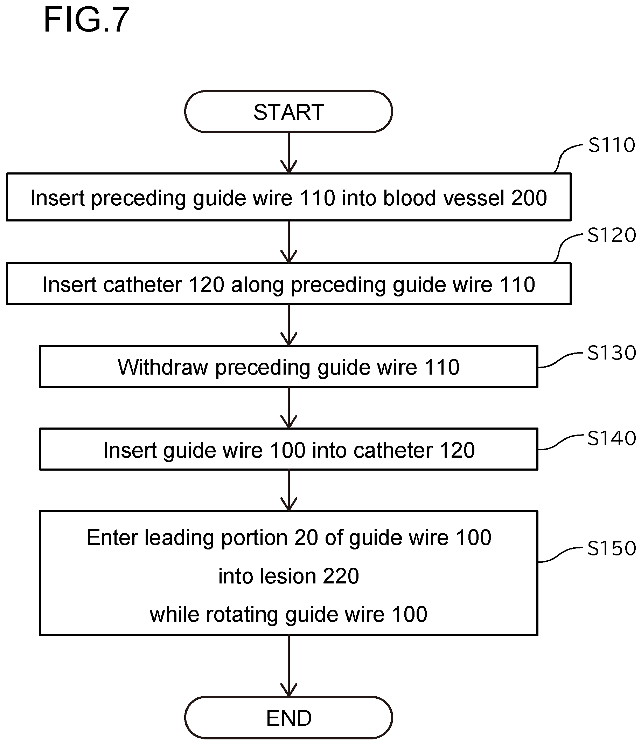

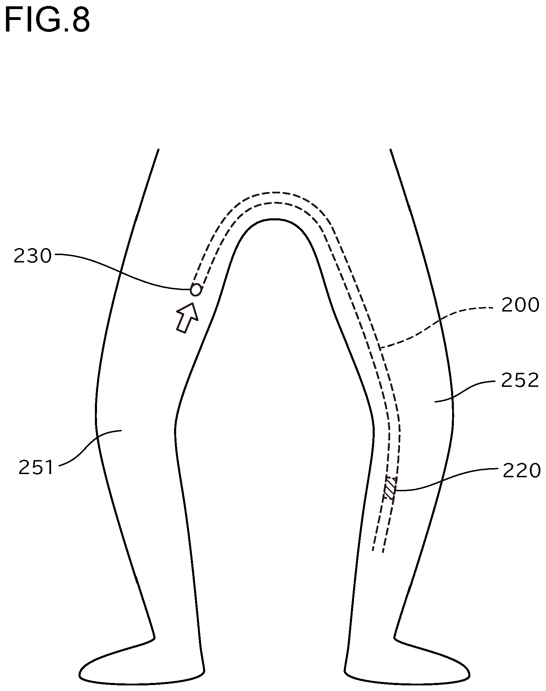

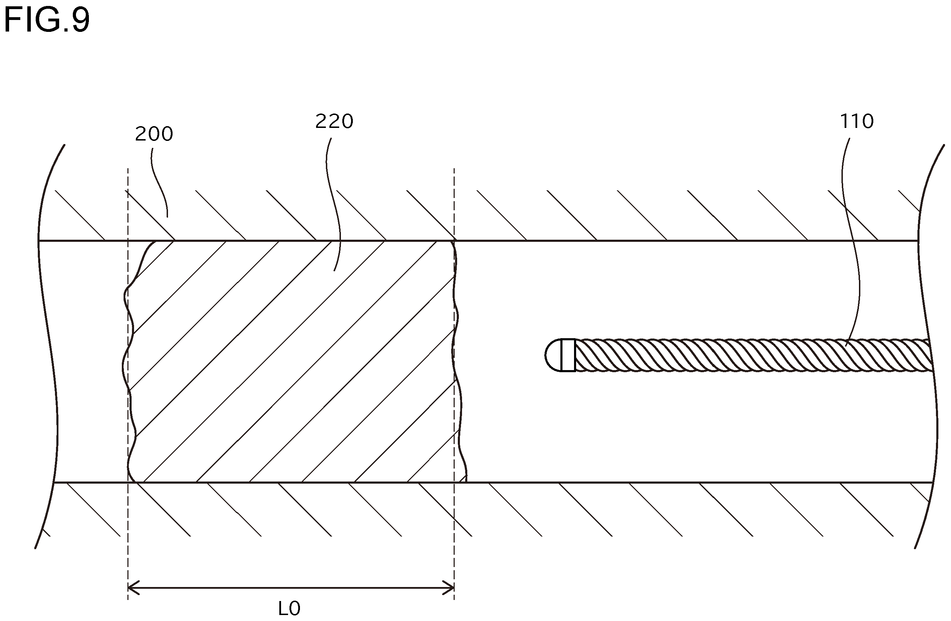

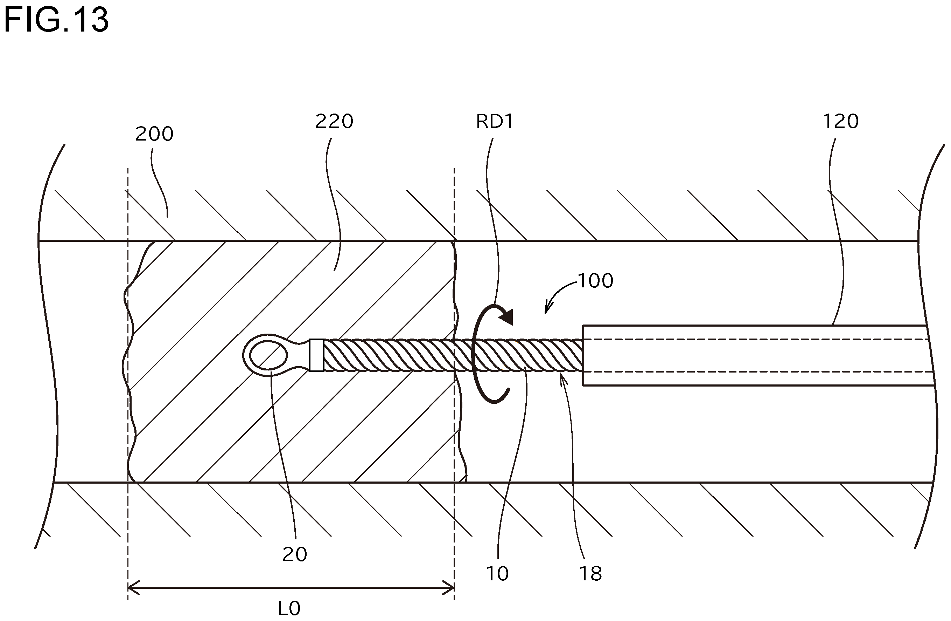

FIG. 7 shows a flow chart of an example of a method of treatment using the guide wire 100. FIGs. 8 to 15 show diagrams illustrating an example of the method of treatment using the guide wire 100. In the method of treatment using the guide wire 100, the leading portion 20 of the guide wire 100 is entered into a lesion 220 in a blood vessel 200, as shown in FIGs. 9 to 15. The lesion 220 is, for example, a highly calcified lesion. The lesion 220 is, for example, a lesion of chronic total occlusion in nature. A length L0 of the lesion 220 along an extension direction of the blood vessel 200 is, for example, 100 mm or more and 500 mm or less. The length L0 of the lesion 220 may be 150 mm or more and 450 mm or less, or may be 200 mm or more and 400 mm or less.

As shown in FIG. 8, the lesion 220 to be treated is located, for example, in a blood vessel 200 at a lower limb portion of a human subject. FIG. 8 shows a lesion 220 which occurred in a below-knee region. In this method of treatment, a procedure referred to as a so-called crossover approach may be used, for example. In this approach, access to the blood vessel 200 is made through the inguinal portion of an opposite leg 251 opposite to a target leg 252, in which the lesion 220 is located, to approach the lesion 220 in the target leg 252. As a method of approaching the lesion 220 occurred at the below-knee region by inserting the guide wire 100 into the blood vessel 200, the following methods other than the crossover approach can be used: an antegrade approach of inserting the guidewire 100 into the blood vessel 200 at the inguinal portion of the target leg 252 in which the lesion 220 is located, and advancing the guide wire 100 along the flow of the blood stream; an antegrade approach of inserting the guide wire 100 into the blood vessel 200 at an arm, and advancing the guide wire 100 along the flow of the blood stream; a retrograde approach of inserting the guide wire 100 into the blood vessel 200 at either the ankle or the instep of the target leg 252 in which the lesion 220 is located, and advancing the guide wire 100 against the flow of the blood stream; and the like. A position at which the guide wire 100 is inserted into the blood vessel 200 is not limited to those position as described above, but the blood vessel 200 at a wrist may also be selected when inserting the guide wire 100 into the blood vessel 200 at an arm. When inserting the guide wire 100 into the blood vessel 200 at a leg, the superficial aorta and the popliteal artery may be selected. Use of the guide wire 100 is not limited to the treatment of the lesion 220 occurred at the below-knee region, but may also be for the treatments of the lesions 220 occurred at other sites, for example, the iliac artery, and the like. The blood vessel 200 is an example of living body lumens. The living body lumens include tubular organs in the human body, such as digestive tract, urinary duct, organs, bile duct, and the like.

A professional inserts a preceding guide wire 110 into the blood vessel 200 (S110, FIG. 9). The preceding guide wire 110 is a known guide wire which differs from the guide wire 100 of this embodiment in terms of not having the leading portion 20. The professional inserts the preceding guide wire 110 into the vessel 200 via a sheath (not shown) positioned at a puncture position 230 (FIG. 8) at the opposite leg 251, and advances the preceding guide wire 110 up to the proximal side of the lesion 220 in the blood vessel 200 at the target leg 252.

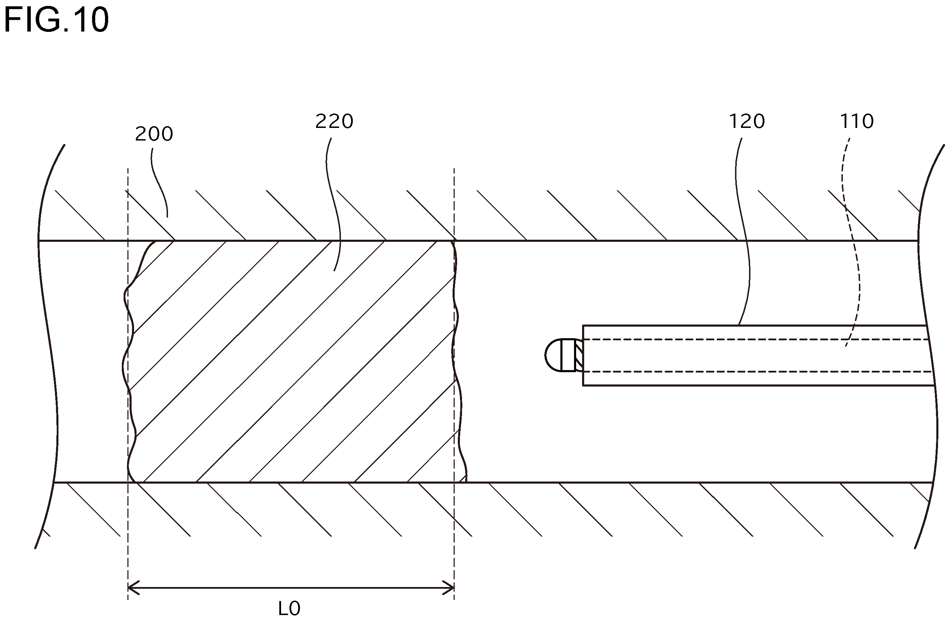

Next, the professional inserts a catheter 120 into the blood vessel 200 along the preceding guide wire 110 (S120, FIG. 10). The professional then advances the catheter 120 up to the proximal side of the lesion 220 in the blood vessel 200.

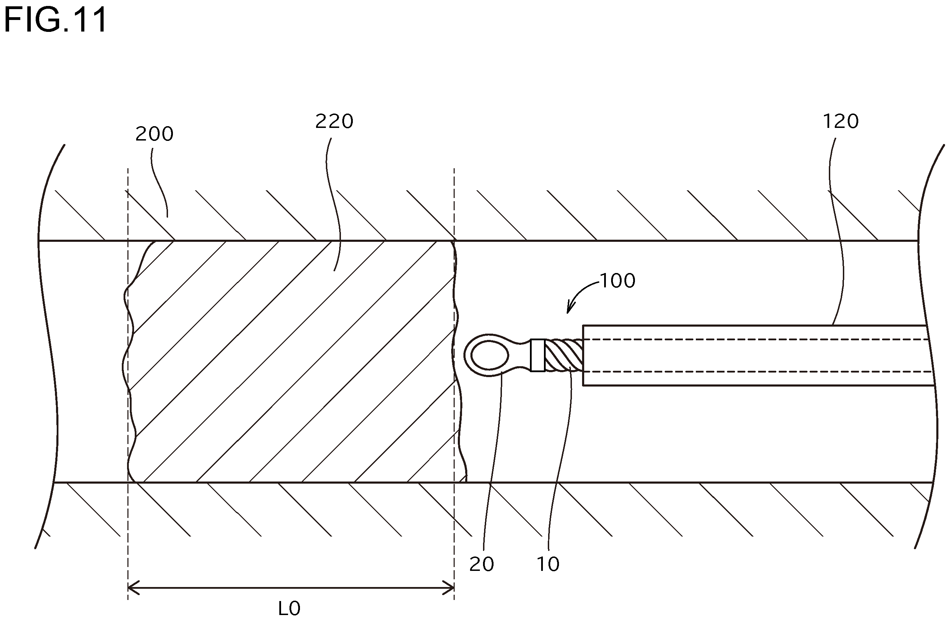

Next, the professional withdraws the preceding guide wire 110 out of the blood vessel 200 (S130). Subsequently, the professional inserts the guide wire 100 from the side of the leading portion 20 into the catheter 120 which remains inserted into the blood vessel 200 (S140, FIG. 11). The professional then advances the guide wire 100 up to the proximal side of the lesion 220 in the blood vessel 200. The guide wire 100 may or may not be rotated around the center axis Ax during advancement of the guide wire 100.

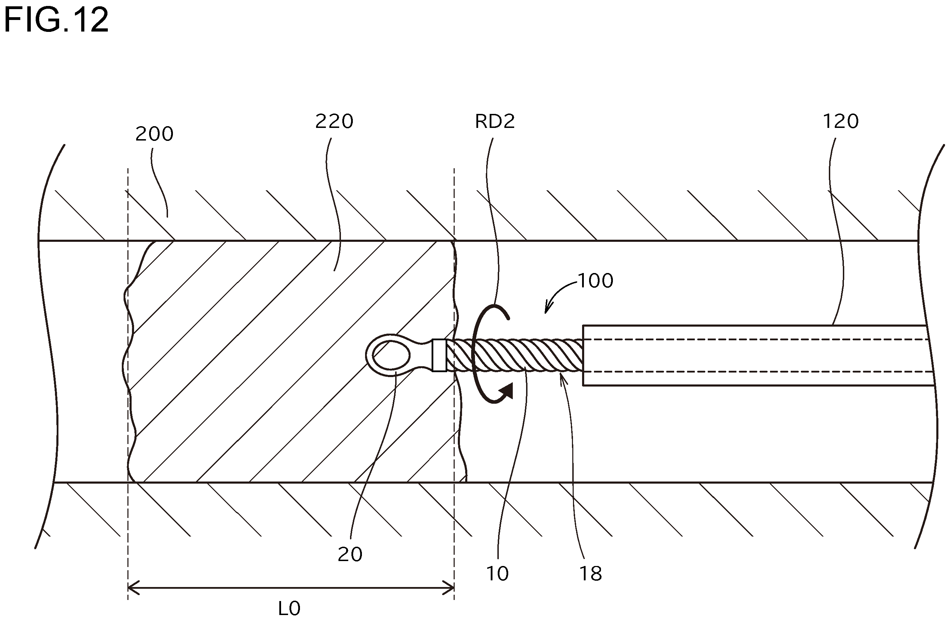

Next, the professional advances the leading portion 20 though the lesion 220 by advancing the guide wire 100 toward the distal end side while rotating the guide wire 100 (S150, FIGs. 12 to 14). When the professional holds the proximal end portion of the guide wire 100, and rotates the guide wire 100 around the center axis Ax, the leading portion 20 located at the distal end portion of the guide wire 100 also rotates around the center axis Ax. The leading portion 20 which rotates inside the lesion 220 can drill the lesion 220 in a manner of tearing apart the lesion 220. In this embodiment, the leading portion 20 is allowed to advance until the leading portion 20 passes through the lesion 220 at a step of advancing the guide wire 100 (S150). When the leading portion 20 has an edge, rotating the leading portion 20 around its axis with the edge pressed against the lesion 220 enables the edge to scrape the lesion 220. A through-hole is formed in the lesion 220 by forcing the leading portion 20 to enter into a space created by scraping the lesion 220 with the edge. The spiral groove 18 is formed on the outer peripheral surface 17 of the main body part 10, as described above. By virtue of the presence of the groove 18, the main body part 10 gains a function of discharging small pieces of the lesion 220 generated by contact between the leading portion 20 which is rotating and the lesion 220 from the distal end side toward the proximal end side of the main body part 10 (hereinafter referred to as a "lesion discharge function"). The step of advancing the guide wire 100 (S150) is performed in a situation where no other medical devices are passed through the lesion 220.

In the step of advancing the guide wire 100 (S150), the professional may switch the rotational direction of the guide wire 100 between a first rotational direction RD1 and a second rotational direction RD2 opposite to the first rotational direction RD1. Alternatively, the professional may fix the rotational direction of the guide wire 100 in the first rotational direction RD1. In this embodiment, the coil 50 is Z-wound, and the first rotational direction RD1 is the clockwise direction as centered around the axis Ax extending from the proximal end to the distal end of the main body part 10 (that is, when the main body part 10 is viewed from the negative direction of the Z-axis toward the positive direction of the Z-axis). The term "Z-wound" as used in the content of spiral articles such as the coil 50 and the glove 53 formed on the coil 50 refers to a manner of winding so that when a strand is wound around an opaque cylinder standing vertically, the right upper side is connected with the left lower side in a visible portion, i.e., a portion of the strand closer to an observer. On the contrary, the term "S-wound" refers to a manner of winding so that when a strand is wound around an opaque cylinder standing vertically, the left upper side is connected with the right lower side in a visible portion, i.e., a portion of the strand closer to an observer.

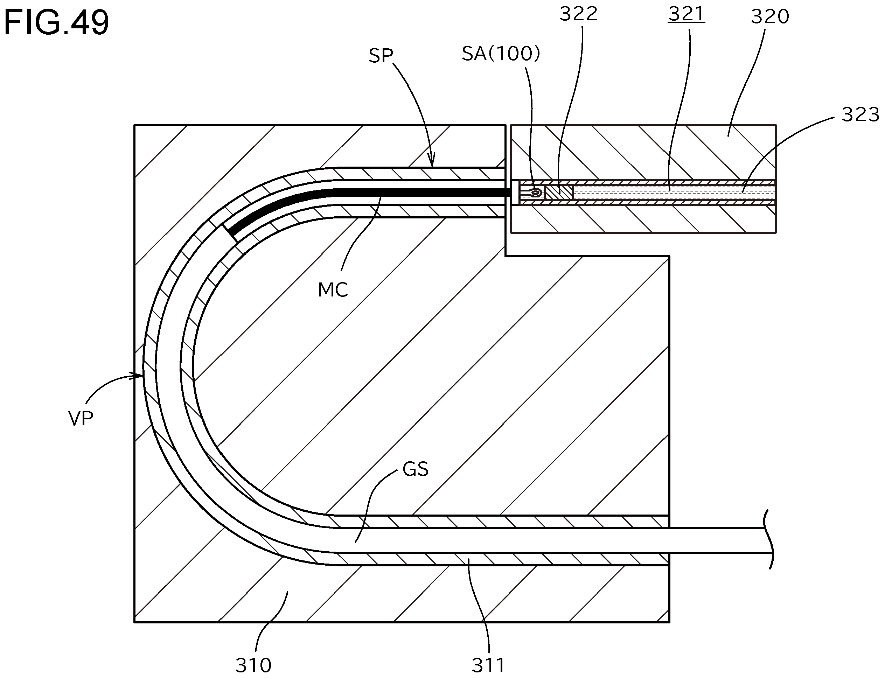

In this embodiment, the lesion passability when the guide wire 100 is rotated in the first rotational direction RD1 differs from the lesion passability when the guide wire 100 is rotated in the second rotational direction RD2 opposite to the first rotational direction. More specifically, when the guide wire 100 is rotated in the first rotational direction RD1, the coil 50 loosens so as to widen a pitch, resulting in a relatively low torque, but the lesion passability becomes relatively high due to the widened pitch of the coil 50. On the contrary, when the guide wire 100 is rotated in the second rotational direction RD2, the coil 50 becomes tighter so as to narrow the pitch, resulting in a relatively high torque, but the lesion passability becomes relatively low due to the narrowed pitch of the coil 50. The professional can select the rotation direction of the guide wire 100 depending on a required lesion passability. The lesion passability of the guide wire 100 can be compared, for example, using a method as described below (see FIG. 49). The term "torque" as used herein means a torque generated at the distal end side of the guide wire 100 when the proximal end side of the guide wire (medical device) 100 is rotated.

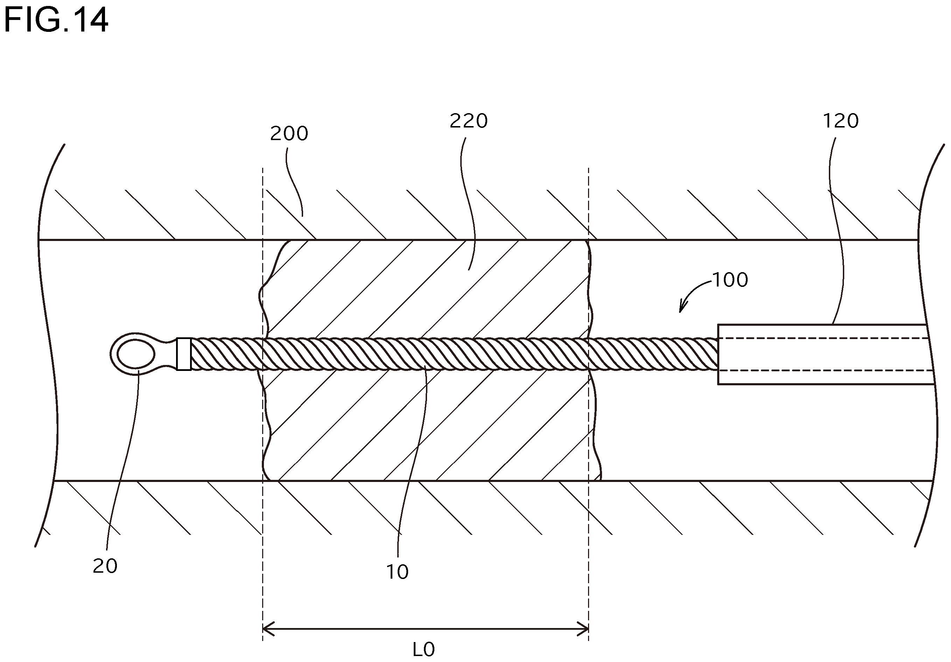

After the leading portion 20 of the guide wire 100 passes through the lesion 220 (FIG. 14), the professional advances a catheter (not shown) to a location of the lesion 220 along the guide wire 100. Subsequently, the professional removes the guide wire 100. The guide wire 100 may or may not be rotated around the center axis Ax when the guide wire 100 is removed.

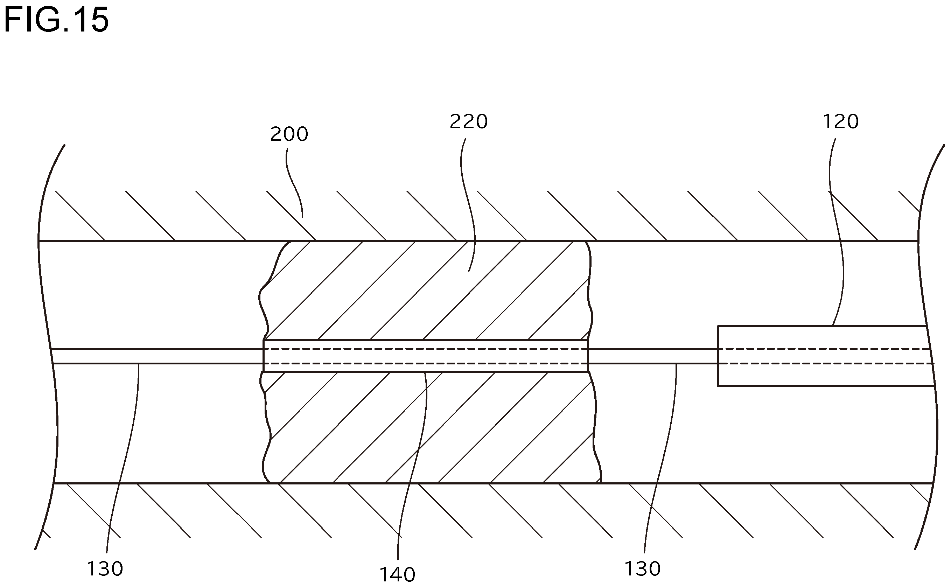

Subsequently, the professional inserts a guide wire 130 for a concurrent device (not shown) into the blood vessel 200, and advances it until the distal end of the guide wire 130 for a concurrent device passes through the lesion 220 (FIG. 15). The professional advances a concurrent device 140 to the location of the lesion 220 along the guide wire 130 for a concurrent device. The concurrent device 140 is, for example, a device for atherectomy, a balloon catheter, a stent, and the like.

### (Detailed configuration of guide wire 100)

As shown in FIGs. 5 and 6, the guide wire 100 includes a wire 40 and a coil 50.

The coil 50 is a hollow cylindrical member around which one or more wire rod are spirally wound. The outer diameter of the coil 50 is, for example, 0.1 mm or more and 0.6 mm or less. The outer diameter of the coil 50 may be 0.2 mm or more and 0.5 mm or less, or may be 0.3 mm or more and 0.4 mm or less. The outer diameter of the coil 50 may be 1.00 mm or more and 2.00 mm or less, may be 1.10 mm or more and 1.65 mm or less, or may be 1.20 mm or more and 1.35 mm or less. In this embodiment, the outer diameter of the coil 50 is constant over the entire length of the coil 50. The coil 50 may be tapered so that the outer diameter of the coil 50 gradually decreases from the proximal end toward the distal end, or may be tapered so that the outer diameter of the coil 50 gradually decreases from the distal end toward the proximal end. A spiral groove 53 is formed on an outer peripheral surface 52 of the coil 50. In this embodiment, the coil 50 is Z-wound, and the running direction of the groove 53 is also in a direction of Z-winding. A spiral groove 18 is formed on an outer peripheral surface 17 of the main body part 10 by virtue of the presence of the spiral groove 53. A distal end 51 of the coil 50 coincides with the distal end 16 of the main body part 10. The coil 50 is an example of a tubular body.

The wire rod of the coil 50 may be a single strand, or may be a twisted wire in which a plurality of strands are twisted. In this embodiment, the coil 50 is a multi-thread coil around which two or more wire rods are wound. In this embodiment, each wire rod of the coil 50 is a twisted wire.

As a material of the coil 50, for example, metal is used. More specifically, the followings may be used, for example, radiation permeable materials such as stainless steel (SUS302, SUS304, SUS316, and the like), Ni-Ti alloys, piano wire, and radiopaque materials such as platinum, gold, tungsten, and any of alloys thereof. The coil 50 may be formed entirely of the same material, or different portions may be made from materials different from each other.

The wire 40 is a linear member. The wire 40 includes a large-diameter portion 41, a first tapered portion 42, an intermediate-diameter portion 43, a second tapered portion 44, a first small-diameter portion 47, and a second small-diameter portion 48.

The large-diameter portion 41 of the wire 40 is a rod-shaped portion having a substantially consist outer diameter. The outer diameter (largest width) of the large-diameter portion 41 is, for example, about 0.2 mm to 3.0 mm. The intermediate-diameter portion 43 is a rod-shaped portion located in the distal end side of the large-diameter portion 41 and having a substantially constant outer diameter smaller than the outer diameter of the large-diameter portion 41. The first tapered portion 42 is a portion located between the large-diameter portion 41 and the intermediate-diameter portion 43, in which the diameter gradually becomes smaller from the boundary with the large-diameter portion 41 toward the boundary with the intermediate-diameter portion 43. The first small-diameter portion 47 is a rod-shaped portion located in the distal end side of the intermediate-diameter portion 43 and having a substantially constant outer diameter smaller than the outer diameter of the intermediate-diameter portion 43. The second tapered portion 44 is a portion located between the intermediate-diameter portion 43 and the first small-diameter portion 47, in which the diameter gradually becomes smaller from the boundary with the intermediate-diameter portion 43 toward the boundary with the first small-diameter portion 47. In this embodiment, the second tapered portion 44 includes a proximal end side-second tapered portion 45 and a distal end side-second tapered portion 46 located in the distal end side of the proximal end side-second tapered portion 45. In this embodiment, the rates of change (hereinafter referred to as "slopes") in the outer diameter along the longitudinal direction in the proximal end side-second tapered portion 45 and the distal end side-second tapered portion 46 are different from each other. For example, the slope of the proximal end side-second tapered portion 45 is steeper than that of the distal end side-second tapered portion 46. The slope of the proximal end side-second tapered portion 45 may be less steep than that of the distal end side-second tapered portion 46, or may be identical to that of the distal end side-second tapered portion 46. In this embodiment, the slopes of the first tapered portion 42 and the second tapered portion 44 are different from each other. For example, the slope of the first tapered portion 42 is steeper than that of the second tapered portion 44. The slope of the first tapered portion 42 may be less steep than that of the second tapered portion 44, or may be identical to that of the second tapered portion 44. The second small-diameter portion 48 is a rod-shaped portion having a substantially constant outer diameter.

The first small-diameter portion 47 of the wire 40 extends from the distal end side of a position BP2 at the boundary with the distal end side-second tapered portion 46 to the distal end 23 of the guide wire 100. The second small-diameter portion 48 extends to the proximal end side of the distal end 23 of the guide wire 100. That is, the second small-diameter portion 48 is folded back to the proximal end side in the wire 40. Hereinafter, a portion folded back to the proximal end side in the wire 40 is called a folded-back portion 40A, and a portion other than the folded-back portion 40A in the wire 40 is called a base portion 40B. The folded-back portion 40A is formed with the second small-diameter portion 48. The base portion 40B is formed with a portion other than the second small-diameter portion 48 in the wire 40. In this embodiment, the folded-back portion 40A and the base portion 40B do not make contact with each other in a radial direction, and a gap is present between them.

For example, a position PP1 of the proximal end of the second small-diameter portion 48 is in the distal end side of the position BP1 of the boundary between the proximal end side-second tapered portion 45 and the distal end side-second tapered portion 46, and is in the proximal end side of the position BP2 of the boundary between the distal end side-second tapered portion 46 and the first small-diameter portion 47.

The shape of a cross section in each position of the wire 40 can take any shapes. The shape of a cross section at each position of the wire 40 may be circular, partially circular (e.g., semi-circular, segmentally circular, arched), oval, rectangular, parallelogram, trapezoidal, rhombic, and the like. A partial circle corresponds to a shape of one of two parts of a circle divided by a chord. The outer rim of a partial circle consists of an arc line and a line segment connecting the two end portions of the arc line. A semi-circle corresponds to a shape of one of two equal parts of a circle divided by a chord passing through the center of the circle. A segmental circle corresponds to a shape of the larger one of two parts of a circle divided by a chord not passing through the center of the circle. An arch corresponds to a shape of the smaller one of two parts of a circle divided by a chord not passing through the center of the circle. The cross section of the wire 40 may take any shapes substantially similar to those described above in addition to the exactly same shapes as those described above. The shape of a cross section may differ at each position along the longitudinal direction of the wire 40.

As a material of the coil 40, for example, metal is used. More specifically, used are, for example, stainless steel (SUS302, SUS304, SUS316, and the like), Ni-Ti alloys, piano wire, and the like. The wire 40 may be formed entirely of the same material, or different portions may be made from materials different from each other.

The wire 40 is inserted into a hollow space of the coil 50. As a result, the wire 40 is partially covered with the coil 50. In this embodiment, the second tapered portion 44, the first small-diameter portion 47, and a portion of the proximal end side of the second small-diameter portion 48 among the wires 40 are covered with the coil 50.

The coil 50 is joined to the wire 40 via a distal end side-jointing material 61 formed at the distal end portion of the coil 50 and a proximal end side-jointing material 62 formed at the proximal end portion of the coil 50. The coil 50 may be joined to the wire 40 via a jointing material formed at another position. The distal end side-jointing material 61 is also present inside the coil 50, and joins the folded-back portion 40A with the base portion 40B of the wire 40. The distal end side-jointing material 61 is extruded from the distal end 51 of the coil 50 to the distal end side, and this extruded portion also joins the folded-back portion 40A with the base portion 40B of the wire 40. Examples of materials used for forming the distal end side-jointing material 61 and the proximal end side-jointing material 62 include, for example, metal solder (Au-Sn alloy, Sn-Ag alloy, Sn-Pb alloy, Pb-Ag alloy, and the like), brazing materials (aluminum alloy braze, silver braze, gold braze, and the like), adhesives (epoxy adhesives and the like), and the like.

The main body part 10 of the guide wire 100 is formed with the entire of the coil 50 and a portion of the wires 40. In this embodiment, the main body part 10 is formed with the large-diameter portion 41, the first tapered portion 42, the intermediate-diameter portion 43, the second tapered portion 44, and a portion inserted into the hollow space of the coil 50 of the first small-diameter portion 47 and the second small-diameter portion 48 in the wires 40.

As shown in FIG. 5, the main body part 10 includes a first rigid portion 11, a second rigid portion 12 located at the proximal end side of the first rigid portion 11 and having a rigidity lower than that of the first rigid portion 11, and a third rigid portion 13 located at the proximal end side of the second rigid portion 12 and having a rigidity higher than that of the second rigid portion 12. The first rigid portion 11 is a portion including the folded-back portion 40A of the wire 40 in the main body part 10. At the first rigid portion 11, the base portion 40B and the folded-back portion 40A of the wire 40 extend along the center axis Ax of the main body part 10 with spaced to each other. The first rigid portion 11 is an example of the parallel portion. The second rigid portion 12 is a portion from the proximal end of the first rigid portion 11, i.e., the proximal end of the folded-back portion 40A (the position PP1 in FIG. 5) to the proximal end of the distal end side-second tapered portion 46 (the position BP1 in FIG. 5) in the main body part 10. The first rigid portion 11, which includes the folded-back portion 40A in addition to the base portion 40B of the wire 40, has a higher rigidity than the second rigid portion 12 including only the base portion 40B. The third rigid portion 13 is a portion in the proximal end side of the proximal end of the distal end side-second tapered portion 46. The proximal end side-second tapered portion 45 and the like as a portion of the wire 40 included in the third rigid portion 13 has a larger diameter than the distal end side-second tapered portion 46 as a portion of the wire 40 included in the second rigid portion 12. Therefore, the third rigid portion 13 has a rigidity higher than the second rigid portion 12. The rigidity of the first rigid portion 11 may be equal to or less than that of the third rigid portion 13, or may be higher than that of the third rigid portion 13.

As shown in FIG. 5, the main body part 10 has a joining region R1 in which a part of the base portion 40B and a part of the folded-back portion 40A in the wire 40, and the main body part 10 are joined, and a non-joining region R2 in which a part of the base portion 40B is not joined to a part of the folded-back portion 40A in the wire 40, and the main body part 10 are not joined. The joining region R1 is located at the distal end portion of the main body part 10, and a part of the base portion 40B and a part of the folded-back portion 40A in the wire 40, and the main body part 10 are joined via the distal end side-jointing material 61. The non-joining region R2 is located in the proximal end side of the joining region R1. A part of folded-back portion 40A is included in the joining region R1. A part of the second small-diameter portion 48 which forms the folded-back portion 40A is joined to the coil 50 along with the first small-diameter portion 47. A part of folded-back portion 40A is included in the non-joining region R2. A part of the second small-diameter portion 48 which forms the folded-back portion 40A is not joined to the coil 50. In the main body part 10, a region in which the wire 40 and the coil 50 are joined via the proximal end side-jointing material 62 is present in the proximal end side of the non-joining region R2.

The leading portion 20 is formed with a portion of the wire 40. In this embodiment, the leading portion 20 is formed with, among the wire 40, a portion protruded to the distal end side from the distal end 51 of the coil 50 at the first small-diameter portion 47 and a portion protruded to the distal end side from the distal end 51 of the coil 50 at the second small-diameter portion 48.

The leading portion 20 has a reinforcing portion 28 located at a connection site with the main body part 10. The connection site with the main body part 10 in the leading portion 20 is reinforced by the reinforcing portion 28. In this embodiment, the reinforcing portion 28 is formed with a portion extruded to the distal end side from the distal end 51 of the coil 50 in the distal end side-jointing material 61. The reinforcing portion 28 may be formed with a welded portion between the leading portion 20 and the main body part 10. A narrowed portion 29 is formed at the outer peripheral surface of the reinforcing portion 28. That is, in a portion of the reinforcing portion 28 (e.g., the proximal end portion), the width of the reinforcing portion 28 in a direction orthogonal to the axis Ax decreases toward the distal end of the reinforcing portion 28, and in another portion of the reinforcing 28 (e.g., the distal end portion), the width of the reinforcing 28 in a direction orthogonal to the axis Ax increases toward the distal end of the reinforcing portion 28, and the outer peripheral surface of the reinforcing portion 28 has a curved surface recessed toward a radially inward side of the reinforcing portion 28.

As shown in FIG. 1, an outer diameter D2 of the leading portion 20 varies along the center axis Ax, as viewed from a direction of the X-axis. Specifically, the outer diameter D2 of the leading portion 20 is substantially identical to a largest outer diameter D1 of the distal end 16 of the main body part 10 in a proximal end 27 of the leading portion 20. The outer diameter D2 of the leading portion 20 gradually decreases from the proximal end 27 of the leading portion 20 toward the distal end side, and takes the smallest value at the bottom portion of the narrowed portion 29, and then gradually increases to a largest outer diameter Dx at a largest outer diameter position Px, and gradually decreases from the largest outer diameter position Px to the distal end 23 of the leading portion 20. The largest outer diameter Dx of the leading portion 20 is, for example, 0.2 mm or more and 1.0 mm or less. The largest outer diameter Dx of the leading portion 20 may be 0.3 mm or more and 0.8 mm or less, or may be 0.4 mm or more and 0.6 mm or less. The largest outer diameter Dx of the leading portion 20 may be 1.00 mm or more and 3.00 mm or less, or may be 1.20 mm or more and 2.50 mm or less, or may be 1.50 mm or more and 2.00 mm or less.

The largest outer diameter Dx of the leading portion 20 may be measured as follows. A measurer observes the guide wire 100 from a side. The lateral side corresponds to a direction where the first small-diameter portion 47 and the second small-diameter portion 48 of the wire 40 overlap, and the Y-axis direction in this embodiment. The measurer search for an angle where a portion in the front side (e.g., the first small-diameter portion 47) and a portion in the back side (e.g., the second small-diameter portion 48) of the wire 40 overlap so that the portion in the back side is not visible. A viewpoint at this time is hereinafter referred to as a "first viewpoint". The image of the guide wire 100 is captured under a microscope along a viewpoint rotated 90 degrees around the center axis Ax (hereinafter referred to as a "second viewpoint") from the first viewpoint. The magnification of the microscope will be at least 200 times. On the captured image, the measurer measures the outer diameter D2 of the leading portion 20 at three measurement points where the leading portion 20 appears to have the largest outer diameter Dx. Specifically, at each of the measurement points, a pair of straight lines parallel to each other is drawn which pass through a pair of end portions of the leading portion 20 in a direction of the outer diameter, and are orthogonal to the direction of the outer diameter, and then the distance between these pairs of straight lines is measured. The measurer takes the largest value of the measurement results at the three measurement points as the largest outer diameter Dx of the leading portion 20.

As viewed from a direction of the X-axis, an outer peripheral line of an outline of the leading portion 20 has a curved portion. In this embodiment, substantially all of the outline of the outer peripheral line of the leading portion 20 is substantially arc-shaped. As viewed from a direction of the X-axis, an inner peripheral line of the outline of the leading portion 20, i.e., a line of the outline of the through-hole 24, has a curved portion. In this embodiment, substantially all of the outline of the inner peripheral line of the leading portion 20 is substantially arc-shaped.

A thickness T2 of the leading portion 20 as used herein means an outer diameter of the leading portion 20 in a direction orthogonal to a direction of the largest outer diameter Dx of the leading portion 20. That is, the thickness T2 of the leading portion 20 means the outer dimension of the leading portion 20 in the direction orthogonal to the direction of the largest outer diameter Dx of the leading portion 20 and also orthogonal to the center axis Ax. As shown in FIGs. 2 and 3, in this embodiment, the thickness T2 of the leading portion 20 corresponds to the outer diameter of the leading portion 20 along a direction of the X axis. The thickness T2 of the leading portion 20 varies along the center axis Ax. Specifically, the thickness T2 of the leading portion 20 is substantially identical to the largest outer diameter D1 at the distal end 16 of the main body part 10 at the proximal end 27 of the leading portion 20. The thickness T2 of the leading portion 20 gradually decreases from the proximal end 27 of the leading portion 20 toward the distal end side, and then substantially constant until the distal end 23 of the leading portion 20.

The largest outer diameter Dx of the leading portion 20 is larger than the thickness T2 of the leading portion 20 at the largest-outer diameter position Px. That is, the leading portion 20 is flattened in shape as a whole. The thickness T2 of the leading portion 20 at the largest-outer diameter position Px is smaller than the largest outer diameter D1 of the distal end 16 of the main body part 10. The thickness T2 of the leading position 20 at the largest-outer diameter position Px is, for example, 0.02 mm or more and 0.3 mm or less. The thickness T2 of the leading portion 20 at the largest-outer diameter position Px may be 0.04 mm or more and 0.2 mm or less, or may be 0.06 mm or more and 0.1 mm or less. The largest outer diameter Dx of the leading portion 20 is, for example, 1.2 times or more of the thickness T2 of the leading portion 20 at the largest-outer diameter position Px. The largest outer diameter Dx of the leading portion 20 may be 1.5 times or more of the thickness T2 of the leading portion 20 at the largest-outer diameter position Px, or may be 1.8 times or more.

The largest outer diameter D1 of the distal end 16 of the main body part 10 is smaller than the largest outer diameter Dx of the leading portion 20. A diameter of a cavity of the lesion 220 formed by the leading portion 20 is larger than the largest outer diameter D1 of the distal end 16 of the main body part 10, and when the main body part 10 passes through the cavity formed in the lesion 220, a gap is present between the outer peripheral surface 17 of the body part 10 and an inner wall of the lesion 220. The small pieces of the lesion 220 scraped with the leading portion 20 pass through the gap between the outer peripheral surface 17 of the main body part 10 and the inner wall of the lesion 220, and are discharged toward the proximal end side of the main body part 10. Retention of the small pieces of the lesion 220 can be prevented between the largest-outer diameter position Px of the leading portion 20 and the distal end 16 of the main body part 10. Thereby, the main body part 10 has a lesion discharge function of discharging the small pieces of the lesion 220 generated by contact between the leading portion 20 which is rotating and the lesion 220 from the distal end side toward the proximal end side of the main body part 10.

The largest outer diameter D1 of the distal end 16 of the main body part 10 is measured as follows. A measurer captures an image of the distal end portion of the guide wire 100 under a microscope. The magnification of the microscope will be at least 200 times. The measurer identifies positions 0.3 mm, 0.6 mm, 0.9 mm away from the distal end 51 toward the proximal end side of the coil 50 on the captured image as three measurement points. At each measurement point, the measurer draws a pair of straight lines parallel to each other that pass through a pair of end portions of the coil 50 in a radial direction and are orthogonal to a radial direction, and measures the distance between these pairs of straight lines. The measurer takes the smallest value of the measurement results at the three measurement points as the outer diameter D1 of the distal end 51 of the coil 50.

A difference Δd (= Dx - D1) between the largest outer diameter Dx of the leading portion 20 and the largest outer diameter D1 of the distal end 16 of the main body part 10 is, for example, 0.06 mm or more and 0.15 mm or less. The difference Δd may be 0.07 mm or more and 0.15 mm or less, or may be 0.08 mm or more and 0.15 mm or less, or may be 0.09 mm or more and 0.14 mm or less, or may be 0.10 mm or more and 0.13 mm or less, or may be 0.11 mm or more and 0.12 mm or less. The difference Δd may be 0.20 mm or more and 1.00 mm or less, or may be 0.30 mm or more and 0.85 mm or less, or may be 0.40 mm or more and 0.65 mm or less.

A ratio Cd (= Dx/D1) of the largest outer diameter Dx of the leading portion 20 to the largest outer diameter D1 of the distal end 16 of the main body part 10 is, for example, 1.15 or more and 1.50 or less. The ratio Cd may be 1.19 or more and 1.50 or less, or may be 1.23 or more and 1.50 or less. The ratio Cd may be 1.16 or more and 1.45 or less, or may be 1.17 or more and 1.40 or less, or may be 1.18 or more and 1.39 or less, or may be 1.20 or more and 1.38 or less, or may be 1.21 or more and 1.37 or less, or may be 1.22 or more and 1.36 or less, or may be 1.24 or more and 1.35 or less, or may be 1.25 or more and 1.30 or less.

As shown in FIGs. 1 and 3, the leading portion 20 has a first end portion 21 and a second end portion 22 located outside the outer peripheral surface 17 of the main body part 10 in a Y-axis direction where the leading portion 20 has the largest outer diameter Dx. The first end portion 21 and the second end portion 22 are protruded from the outer peripheral surface 17 of the main body part 10 to the outer peripheral side in a radial direction. The first end portion 21 and the second end portion 22 are opposite to each other across a virtual line VL extended from the center axis Ax of the main body part 10. In this embodiment, a protrusion amount L1 from the outer peripheral surface 17 of the main body part 10 at the first end portion 21 is identical to a protrusion amount L2 from the outer peripheral surface 17 of the main body part 10 at the second end portion 22. The Y-axis direction is an example of the first direction and the second direction. In this embodiment, a direction where the first end portion 21 and the second end portion 22 of the leading portion 20 protruded outward from the outer peripheral surface 17 of the main body part 10 coincides with a direction where the leading portion 20 has the largest outer diameter Dx. In other words, the outer diameter D2 of the leading portion 20 in a direction orthogonal to the center axis Ax of the main body part 10 is largest at a position of the first end portion 21 along a direction (the Z-axis direction) where the center axis Ax extends.

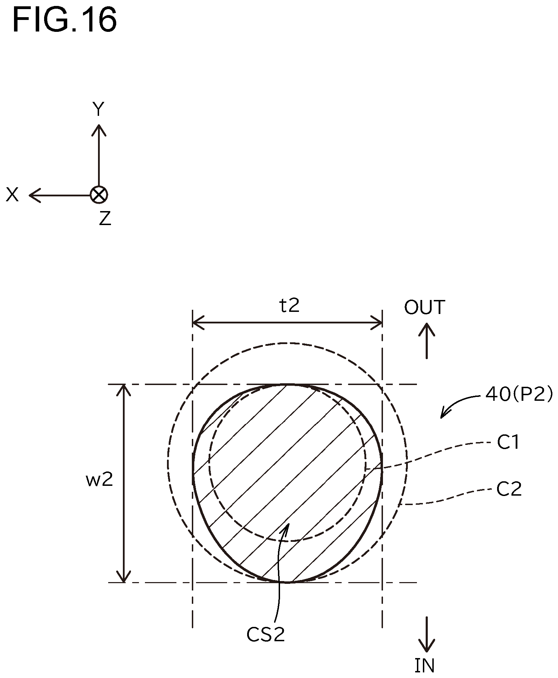

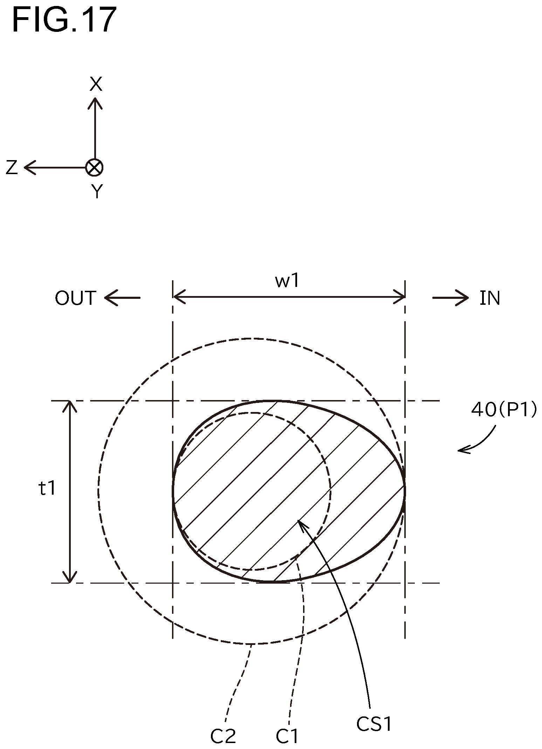

FIGs. 16 and 17 show diagrams showing cross sections of the wire 40. FIG. 16 shows a cross section of the wire 40 at the position of XVI-XVI in FIG. 5. FIG. 17 shows a cross section of the wire 40 at the position of XVII-XVII in FIG. 5. Symbols indicating an outer peripheral side (OUT) and a central side (IN) at a loop of the leading portion 20 are shown in FIGs. 16 and 17.

As shown in FIG. 6, a cross section of a third portion P3 of the first small-diameter portion 47 of the wire 40, which is a portion included in the main body part 10 (hereinafter referred to as a "third cross section CS3"), is substantially circular in shape. As shown in FIG. 16, a cross section of a second portion P2 of the first small-diameter portion 47 of the wire 40, which is a portion included in the leading portion 20 (hereinafter referred to as a "second cross section CS2") is not circular but collapsed circular in shape. As shown in FIG. 5, the second portion P2 is one end portion (the first end portion 21) of the leading portion 20 in a direction of the largest outer diameter Dx of the leading portion 20. In this embodiment, an end portion of the leading portion 20 (the second end portion 22) opposite to the second portion P2 across the virtual line VL also has a similar cross section as the second portion P2. As shown in FIG. 17, a cross section of the first portion P1, which is a boundary between the first small-diameter portion 47 and the second small-diameter portion 48 in the wire 40 (hereinafter referred to as a "first cross section CS1") is not circular, but collapsed circular in shape. The first portion P1 corresponds to the distal end portion of the leading portion 20.

In the wire 40, the shape of the first cross section CS1 of the first portion P1 included in the leading portion 20 is different from that of the second cross section CS2 of the second portion P2 also included in the leading portion 20. The area of the first cross section CS1 is different from that of the second cross section CS2. For example, the area of the first cross section CS1 is larger than that of the second cross section CS2. The area of the second cross section CS2 may be smaller than that of the third cross section CS3. The circularity of the first cross section CS1 is different from that of the second cross section CS2. For example, the circularity of the first cross section CS1 is lower than that of the second cross section CS2. The circularity is the degree of closeness to the perfect circle, and is the difference between the radii of two circles C1 and C2 when the distance between two circles C1 and C2 is minimized if the outlines of the cross sections are sandwiched between two concentric geometric circles C1 and C2.

In the outline of the first cross section CS1, the curvatures of the outer peripheral side and the inner peripheral side are different from each other. For example, in the outline of a cross section of the first cross section CS1, the curvature of the outer peripheral side is smaller than that of the inner peripheral side. In the outline of the second cross section CS2, the curvatures of the outer peripheral side and the inner peripheral side are different from each other. For example, in the outline of the second cross section CS2, the curvature of the outer peripheral side is smaller than that of the inner peripheral side.

In the present description, a thickness direction in a cross section of each portion of the wire 40 corresponds to a direction orthogonal to the largest outer diameter Dx of the leading portion 20, and is the X-axis direction in this embodiment. A thickness of the first cross section CS1 (hereinafter referred to as a "first thickness t1") differs from a thickness of the second cross section CS2 (hereinafter referred to as a "second thickness t2"). For example, the second thickness t2 is thinner than the first thickness t1. The first thickness t1 may be, for example, thicker than a thickness of the third cross section CS3 (hereinafter referred to as a "third thickness t3").

In the present description, a width direction in a cross section of each portion of the wire 40 corresponds to a direction orthogonal to the thickness direction. A width of the first cross section CS1 (hereinafter referred to as a "first width w1") differs from a width of the second cross section CS2 (hereinafter referred to as a "second width w2"). For example, the second width w2 is larger than the first width w1. The first width w1 may be, for example, smaller than a width of the third cross section CS3 (hereinafter referred to as a "third width w3").

In the wire 40, the first portion P1 having the first cross section CS1 shown in FIG. 17 may be provided in the main body part 10, and the second part P2 having the second cross section CS2 shown in FIG. 16 may be provided in the leading portion 20.

As shown in FIG. 1, the guide wire 100 includes a first coating 31 and a second coating 32. The first coating 31 covers at least a part of the leading portion 20. The first coating 31 may cover a part of the main body part 10. In this embodiment, the first coating 31 covers a region R31 formed with the entire of the leading portion 20 and the distal end portion of the main body part 10. The second coating 32 covers at least a part of the main body part 10. In this embodiment, the second coating 32 covers a region R32 as a middle portion except for the distal end portion and the proximal end portion in the main body part 10. The region R32 is located in the proximal end side of the region R31. The slidability of the first coating 31 differs from that of the second coating 32. For example, the slidability of the first coating 31 is lower than that of the second coating 32. The first coating 31 is, for example, hydrophobic, and made from silicone. The second coating 32 is, for example, hydrophilic, and is made from polyvinylpyrrolidone, polyacrylic acid, polyacrylamide, polyvinyl alcohol, maleic anhydride copolymer, hyaluronic acid, or the like.

The guide wire 100 further includes a third coating 33. The third coating 33 covers at least a part of main body part 10. In this embodiment, the third coating 33 covers a region R33 as the proximal end portion of the main body part 10. The region R33 is located in the proximal end side of the region R32. The third coating 33 is made from, for example, PTFE.

### (Method of manufacture guide wire 100)

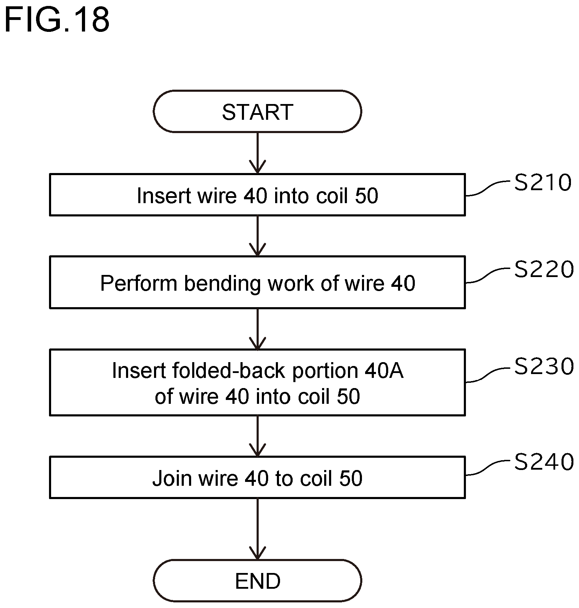

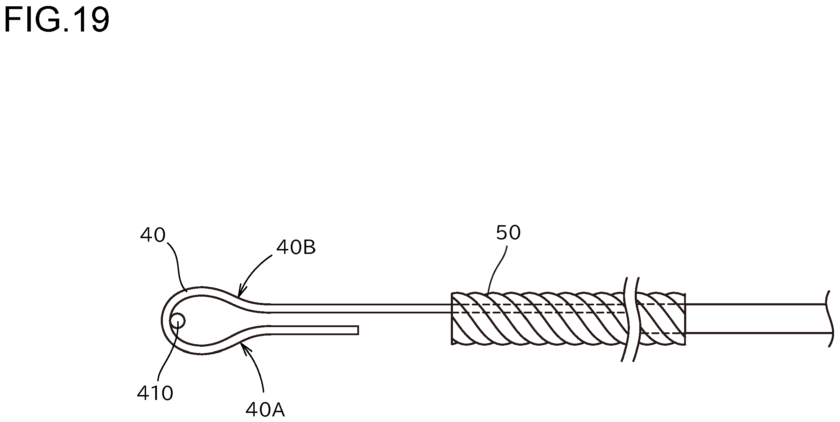

FIG. 18 shows a flow chart of an example of a method of manufacturing the guide wire 100. FIGs. 19 to 21 show diagrams illustrating an example of the method of manufacturing the guide wire 100. First, a worker prepares the wire 40 and inserts the wire 40 into the hollow space of the coil 50 (S210, FIG. 19). Next, the worker performs a bending work of bending a protruded portion of the wire 40 from the distal end of the coil 50 into a loop shape by pressing it against a pin 410 (S220, FIG. 19). The distal end side of the wire 40 relative to the pin 410 serves as the folded-back portion 40A (see FIG. 5), and the proximal end side of the wire 40 relative to the pin 410 serves as the base portion 40B. Upon the bending work, the cross section in each portion of the wire 40 is deformed. As a result, the shape of a cross section at each portion of the wire 40 is shaped as described above (see FIGs. 6, 16, 17).

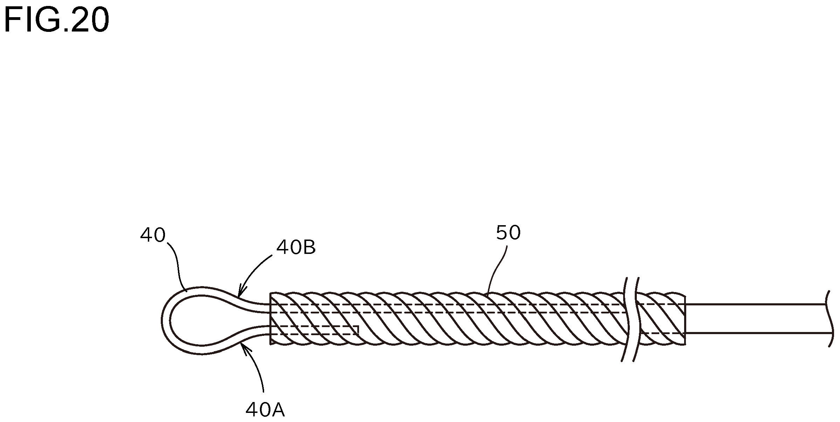

Next, the worker elastically deforms the folded-back portion 40A of the wire 40, and inserts it into the hollow space of the coil 50 (S230, FIG. 20). At this time, the folded-back portion 40A and the base portion 40B do not make contact with each other in a radial direction, leaving a gap between them by virtue of the springiness of the wire 40.

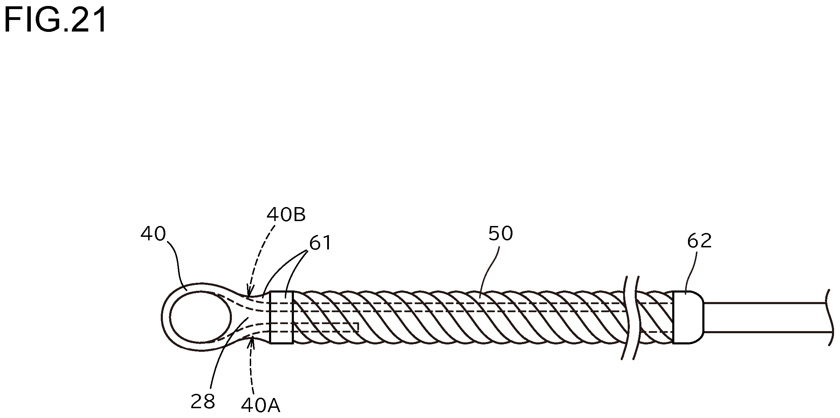

Next, the worker joins the wire 40 to the coil 50 (S240, FIG. 21). Specifically, the worker forms a distal end side-joining material 61 and a proximal end side-joining material 62, for example, with a joining material such as a soldering material to join the wire 40 to the coil 50. The jointing material is fed into the inside of the coil 50, for example, through a pitch portion of the coil 50. The reinforcing portion 28 is formed with the distal end side-jointing material 61 extruded to the distal end side of the distal end of the coil 50. The guide wire 100 having the leading portion 20 having the reinforcing portion 28; and the main body part 10 is produced by the joining step. The guide wire 100 according to this embodiment is, for example, manufactured by the above step.

### (Functional effects of this embodiment)

As described above, the guide wire 100 of the present embodiment includes the elongated main body part 10, and the leading portion 20 which is connected to the main body part 10 at the distal end 16 and enters the lesion 220. The leading portion 20 has the first end portion 21 located outside the outer peripheral surface 17 of the main body part 10. According to the guide wire 100 of the present embodiment, the leading portion 20 having the first end portion 21 located outside the outer peripheral surface 17 of the main body part 10 may be rotated to efficiently drill the lesion 220. This can improve the passability through the lesion 220.

In the present embodiment, the main body part 10 extends along the center axis Ax, and the first end portion 21 is located outside the outer peripheral surface 17 of the main body part 10 in the Y-axis direction orthogonal to the center axis Ax of the main body part 10. According to the guide wire 100 of the present embodiment, the leading portion 20 having the first end portion 21 located outside the outer peripheral surface 17 of the main body part 10 in a direction orthogonal to the center axis Ax of the main body part 10 may be rotated to effectively drill the lesion 220. This can improve the passability through the lesion 220.

In the present embodiment, the leading portion 20 has the second end portion 22 located outside the outer peripheral surface 17 of the main body part 10 in the Y-axis direction orthogonal to the center axis Ax in addition to the first end portion 21. According to the guide wire 100 of the present embodiment, the leading portion 20 having the second end portion 22 located outside the outer peripheral surface 17 of the main body part 10 in addition to the first end portion 21 may be rotated to more effectively drill the lesion 220. This can further improve the passability of the lesion 220.

In the present embodiment, the first end portion 21 and the second end portion 22 of the leading portion 20 are opposite to each other across the virtual line VL extended from the center axis Ax of the main body part 10. According to the guide wire 100 of the present embodiment, the leading portion 20 having the first end portion 21 and the second end portion 22 which are opposite to each other across the virtual line VL may be rotated to more effectively drill the lesion 220. This can further improve the passability through the lesion 220.

In the present embodiment, a direction where the first end portion 21 and the second end portion 22 of the leading portion 20 protruded outside the outer peripheral surface 17 of the main body part 10 coincides with a direction where the leading portion 20 has the largest outer diameter Dx. In other words, the outer diameter D2 of the leading portion 20 in a direction orthogonal to the center axis Ax of the main body part 10 is largest at a position of the first end portion 21 along a direction (the Z-axis direction) where the center axis Ax extends. According to the guide wire 100 of the present embodiment, the leading portion 20 having the first end portion 21 and the second end portion 22 protruded in the direction having the largest-outer diameter Dx may be rotated to more effectively drill the lesion 220. This can further improve the passability through the lesion 220.

In the present embodiment, the largest-outer diameter Dx of the leading portion 20 is larger than the largest-outer diameter D1 of the distal end 16 of the main body part 10, and the thickness T2, which is an outer diameter in a direction orthogonal to a direction of the largest-outer diameter Dx of the leading portion 20, is smaller than the largest-outer diameter D1 of the distal end 16 of the main body part 10. According to the guide wire 100 of the present embodiment, a flattened shape of the leading portion 20 enables the leading portion 20 to efficiently drill the lesion 220. This can improve the passability through the lesion 220.

According to the present embodiment, the main body part 10 has a lesion discharge function. According to the guide wire 100 of the present embodiment, the small pieces of the lesion 220 generated by contact between the leading portion 20 and the lesion 220 are smoothly discharged from the distal end side toward the proximal end side of the main body part 10. This can prevent the small pieces from interfering with drilling by the leading portion 20, improving the passability through the lesion 220.

In the present embodiment, the lesion discharge function of the main body part 10 can be achieved by the spiral groove 18 formed on the outer peripheral surface 17 of the main body part 10. According to the guide wire 100 of the present embodiment, the small pieces of the lesion 220 generated by contact between the leading portion 20 and the lesion 220 are more smoothly discharged along the groove 18 from the distal end side toward the proximal end side of the main body part 10. This can more effectively prevent the small pieces from interfering with drilling by the leading portion 20, effectively improving the passability through the lesion 220.

In the present embodiment, the main body part 10 includes the coil 50, and the spiral groove 18 of the main body part 10 is formed on the outer peripheral surface 52 of the coil 50. According to the guide wire 100 of the present embodiment, a structure with a relatively simple configuration in which the spiral groove 18 is formed on the outer peripheral surface 17 of the main body part 10 can be provided.

In the present embodiment, the lesion discharge function of the main body part 10 can be achieved by the largest-outer diameter D1 of the distal end 16 of the main body part 10 being smaller than the largest-outer diameter Dx of the leading portion 20. According to the guide wire 100 of the present embodiment, the small pieces of the lesion 220 generated by contact between the leading portion 20 and the lesion 220 are smoothly discharged from the distal end side toward the proximal end side of the main body part 10. This can prevent the small pieces from interfering with drilling by the leading portion 20, improving the passability through the lesion 220.

In the present embodiment, the lesion passability when the guide wire 100 is rotated around the center axis Ax in the first rotational direction RD1 is different from the lesion passability when the guide wire 100 is rotated in the second rotational direction RD2 opposite to the first rotational direction. According to the guide wire 100 of the present embodiment, the rotational directions of the guide wire 100 can be selected depending on required lesion passability, allowing a procedure to be proceeded carefully.

In the present embodiment, the lesion passability when the guide wire 100 is rotated in the first rotational direction RD1 is higher than the lesion passability when the guide wire 100 is rotated in the second rotational direction RD2, and the torque generated when the guide wire 100 is rotated in the first rotational direction RD1 is lower than the torque generated when the guide wire 100 is rotated in the second rotational direction RD2. According to the guide wire 100 of the present embodiment, the rotational directions of the guide wire 100 can be selected depending on required lesion passability and torque.

In the present embodiment, the spiral groove 18 is formed on the outer peripheral surface 17 of the main body part 10. According to the guide wire 100 of the present embodiment, the guide wire 100 can be provided in which lesion passability and torque are different depending on rotational directions.

In the present embodiment, the main body part 10 includes the coil 50, the spiral groove 18 is formed on the outer peripheral surface 52 of the coil 50. According to the guide wire 100 of the present embodiment, the guide wire 100 with a relatively simple configuration in which lesion passability and torque are different depending on rotational directions can be provided.

In the present embodiment, the first rotational direction RD1 is the clockwise direction as centered around its rotation axis extending from the proximal end to the distal end of the main body part 10, and the spiral groove 18 is formed on the outer peripheral surface 17 of the main body part 10, and the spiral groove 18 is Z-wound, the lesion passability when the guide wire 100 is rotated in the first rotational direction RD1 is higher than the lesion passability when the guide wire 100 is rotated in the second rotational direction. According to the guide wire 100 of the present embodiment, the lesion passability can be changed depending on a rotational direction without having a complex configuration.

In the method of treatment according to the present embodiment, the guide wire 100 is inserted into the blood vessel 200 from the side of the leading portion 20, and the leading portion 20 is then allowed to advance through the lesion 220 while the guide wire 100 is rotated around the center axis Ax within the blood vessel 100. In the method of treatment according to the present invention, the lesion 220 can be efficiently drilled with the leading portion 20 which is rotating, enabling the lesion 220 to be treated efficiently.

In the method of treatment according to the present embodiment, the blood vessel 200 to be treated is a blood vessel 200 of a human subject, and the lesion 220 is located in a target leg 252 of the human subject, and the guide wire 100 is inserted into the blood vessel 200 from an opposite leg 251 opposite to the target leg 252 in which the lesion 220 is located. In the method of treatment according to the present embodiment, the backup capacity upon treatment can be improved.

In the method of treatment according to the present embodiment, the blood vessel 200 to be treated may be a blood vessel 200 of a human subject, and the lesion 220 may be located in a target leg 252 of the human subject, and the guide wire 100 may be inserted in the blood vessel 200 from the target leg 252 in which the lesion 220 is located, and then may be allowed to advance in a direction of the flow of blood in the blood vessel. The method of treatment as described above can also effectively treat the lesion 220.

In the method of treatment according to the present embodiment, the blood vessel 200 to be treated may be a blood vessel 200 of a human subject, the lesion 220 may be located at a target leg 252 of the human subject, and the guide wire 100 may be inserted into the blood vessel 200 from either of the ankle or the instep of the leg in which the lesion 220 is located, and then allowed to advance in a direction opposite to the direction of the flow of blood in the blood vessel 200. The method of treatment as described above can also effectively treat the lesion 220.

In the method of treatment according to the present embodiment, the blood vessel 200 to be treated may be a blood vessel 200 of a human subject, and the lesion 220 may be located at a target leg 252 of the human subject, and the guide wire 100 may be inserted into the blood vessel 200 from an arm of the human subject. The method of treatment as described above can also effectively treat the lesion 220.

In the method of treatment according to the present embodiment, the guide wire 100 is allowed to advance while the guide wire 100 is rotated in the first rotational direction RD1, and the guide wire 100 is allowed to advance while the guide wire 100 is rotated in the second rotational direction RD2 which is opposite to the first rotational direction RD1. In the method of treatment according to the present invention, the lesion 220 can be more efficiently drilled with the leading portion 20 which is rotating, enabling the lesion 220 to be treated efficiently.

In the method of treatment according to the present embodiment, prior to insertion of the guide wire 100, a sheath may be inserted into the blood vessel 200, and the guide wire 100 may be passed though inside the sheath and then inserted into the blood vessel 200. According to such a method of treatment, the guide wire 100 may be smoothly inserted into the blood vessel 200.

In the method of treatment according to the present embodiment, a professional holds the guide wire 100 and advance the guide wire 100 while rotating the guide wire 100. In the method of treatment according to the present embodiment, treatment can be performed with the lesion passability of the guide wire 100 as intended by the professional.

In the method of treatment according the present embodiment, the preceding guide wire 110 is inserted into the blood vessel 200, and the catheter 120 is inserted into the blood vessel 200 along the preceding guide wire 110, and the preceding guide wire 110 is withdrawn from the blood vessel 200, and then the guide wire 100 is inserted into the catheter 120 which remains inserted in the blood vessel 200. In the method of treatment of the present embodiment, treatment can be efficiently performed with the guide wire 100.

In the method of treatment according to the present embodiment, advancement of the guide wire 100 is performed in a situation where no other medical devices have passed through the lesion 220. In the method of treatment according to the present embodiment, the guide wire 100 can be passed through the lesion 220 that would be otherwise relatively difficult for a medical device to be passed through.

In the method of treatment according to the present embodiment, the guide wire 100 may be allowed to advance with the rotational direction of the guide wire 100 fixed to the first rotational direction RD1. In this way, treatment can be performed with the lesion passability of the guide wire 100 fixed.

In the method of treatment according to the present embodiment, the length of the lesion 220 along the extension direction of the blood vessel 200 may be 100 mm or more. In the method of treatment according to the present embodiment, the guide wire 100 can be passed through the lesion 220 that would be otherwise relatively difficult for a medical device to be passed through.

In the method of treatment according to the present embodiment, the lesion 220 may be a highly calcified lesion. In the method of treatment according to the present embodiment, the guide wire 100 can be passed through the lesion 220 that would be otherwise relatively difficult for a medical device to be passed through.

In the method of treatment according to the present embodiment, after the guide wire 100 passes through the lesion 220, the guide wire 130 for concurrent devices may be passed through the lesion 220, and the concurrent device 140 may be then inserted along the guide wire 130 for concurrent devices. In the method of treatment according to the present embodiment, treatment can be efficiently performed with the concurrent device 140. Examples of the concurrent device 140 include, for example, atherectomy devices, balloon catheters, stents, and the like.

In the present embodiment, the leading portion 20 has a loop-shaped structure formed with a portion of the wire 40. More specifically, the leading portion 20 is formed with, among the wire 40, a portion protruded to the distal end side from the distal end 51 of the coil 50 at the first small-diameter portion 47 and the second small-diameter portion 48. The main body part 10 includes another portion of the wire 40. More specifically, the main body part 10 includes, among the wire 40, a portion inserted into the hollow space of the coil 50 at the first small-diameter portion 47 and the second small-diameter portion 48. When the thickness direction of the wire 40 is defined as a direction orthogonal to a direction of the largest-outer diameter Dx of the leading portion 20, the wire 40 has the first portion P1 having the first thickness t1 and the second portion P2 having the second thickness t2 thinner than the first thickness t1. According to the guide wire 100 of the present embodiment, a desired property can be given to each of the portions of the wire 40 which form the leading portion 20 and the main body part 10. For example, by arranging the second portion P2 having the second thickness t2 which is relatively thin in a region of the wire 40 where the leading portion 20 is formed, the drillability of the leading portion 20 can be improved, resulting in improved passability through the lesion 220. Moreover, when a tensile force is applied to the leading portion 20, the second portion P2 having the second thickness t2 which is relatively thin is preferentially subjected to fracturing, thereby more reliably preventing detachment of the leading portion 20 from the main body part 10. Alternatively, by arranging the first portion P1 having the first thickness t1 which is relatively thick at a region of the wire 40 where the main body part 10 is formed, the durability of the leading portion 20 can be improved.

In the present embodiment, the first portion P1 and the second portion P2 are present in the leading portion 20. According to the guide wire 100 of the present embodiment, the presence of the second portion P2 having the second thickness t2 which is relatively thin can improve the drillability of the leading portion 20, resulting in improved passability through the lesion 220. Moreover, when a tensile force is applied to the leading portion 20, the second portion P2 having the second thickness t2 which is relatively thin may be preferentially subjected to fracturing, thereby more reliably preventing detachment of the leading portion 20 from the main body part 10.

In the present embodiment, the second portion P2 corresponds to at least one end portion of the leading portion 20 in a direction of the largest outer diameter Dx of the leading portion 20. According to the guide wire 100 of the present embodiment, the presence of the second portion P2 having the second thickness t2 which is relatively thin at an end portion of the leading portion 20 in a direction of the largest outer diameter Dx of the leading portion 20 can efficiently improve the drillability of the leading portion 20, resulting in effectively improved passability though the lesion 220.

In the present embodiment, the wire 40 has the first portion P1 having the first cross section CS1, and the second portion P2 having the second cross section CS2 which differs from the first cross section CS1 in shape. According to the guide wire 100 of the present embodiment, a desired property can be given to each of the portions of the wire 40 which form the leading portion 20 and the main body part 10. For example, the cross section of a portion of the wire 40 which forms the leading portion 20 can be made into a shape having high drillability, and the cross section of a portion which forms the main body part 10 can be made into a shape having high processability.

In the present embodiment, the area of the first cross section CS1 of the first portion P1 of the wire 40 differs from the area of the second cross section CS2 of the second portion P2. According to the guide wire 100 of the present embodiment, a desired property can be given to each of the portions of the wire 40 which form the leading portion 20 and the main body part 10.

In the present embodiment, a cross section of the wire 40 included in the main body part 10 (for example, the third cross section CS3 of the third portion P3) is circular in shape, and the circuitry of the first cross section CS1 of the first portion P1 differs from the circuitry of the second cross section CS2 of the second portion P2. According to the guide wire 100 of the present embodiment, a desired property can be given to each of the portions of the wire 40 which form the leading portion 20 and the main body part 10.

In the present embodiment, the first portion P1 of the wire 40 is present in the leading portion 20, and the curvatures of the outer peripheral side and the inner peripheral side in the outline of the first cross section CS1 of the first portion P1 are different from each other. According to the guide wire 100 of the present embodiment, a desired property can be given to the first portion P1 of the wire 40 which forms the leading portion 20. For example, the first cross section CS1 of the first portion P1 can be shaped so as to have high drillability.

In the present embodiment, the curvature of the outer peripheral side in the outline of the first cross section CS1 of the first portion P1 is smaller than that of the inner peripheral side. According to the guide wire 100 of the present embodiment, the first cross section CS1 can be shaped so as to have high drillability by a relatively small curvature of the outer peripheral side in the outline of the first cross section CS1 of the first portion P1.

In the present embodiment, among the wire 40, the first portion P1 having the first cross section CS1 may be provided in the main body part 10, and the second portion P2 having the second cross section CS2 may be provided in the leading portion 20. According to this configuration, a desired property can be given to each of the portions of the wire 40 which form the leading portion 20 and the main body part 10.

In the present embodiment, the main body part 10 has the first rigid portion 11 having the first rigidity, and the second rigid portion 12 located in the proximal end side of the first rigid portion 11 and having the second rigidity lower than the first rigidity. According to the guide wire 100 of the present embodiment, the presence of the second rigid portion 12 having relatively low rigidity can ensure softness of the main body part 10 while the presence of the first rigid portion 11 located in the distal end side of the second rigid portion 12 and having relatively high rigidity can improve the rotation transmissibility from the main body part 10 to the leading portion 20, improving the passability though the lesion 220.

In the present embodiment, the main body part 10 includes the third rigid portion 13 located in the proximal end side of the second rigid portion 12 and having the third rigidity higher than the second rigidity. According to the guide wire 100 of the present embodiment, the rotation transmissibility from the proximal end portion to the distal end portion of the main body part 10 can be improved.

In the present embodiment, the first rigidity of the first rigid portion 11 may be equal to or lower than the third rigidity of the third rigid portion 13. In this way, an excessive rigidity of the distal end portion of the main body part 10 can be prevented, which in turn can prevent the occurrence of kinking at the distal end portion of the main body part 10.

In the present embodiment, the first rigidity of the first rigid portion 11 may be higher than the third rigidity of the third rigid portion 13. In this way, the rotation transmissibility from the main body part 10 to the leading portion 20 can be effectively increased, thereby efficiently improving the passability through the lesion 220.

In the present embodiment, the leading portion 20 has a loop-shaped structure formed with a part of the base portion 40B and a part of the folded-back portion 40A of the wire 40. The main body part 10 includes a part of the base portion 40B and a part of the folded-back portion 40A. The first rigid portion 11 of the main body part 10 is a portion including the folded-back portion 40A of the wire 40. According to the guide wire 100 of the present embodiment, the main body part 10 having the first rigid portion 11 and the second rigid portion 12, which have different rigidity from each other, can be obtained while avoiding structural complexity.

In the present embodiment, the base portion 40B of the wire 40 has the tapered portions 42, 44 in which the largest width decreases toward the distal end side of the main body part 10. According to the guide wire 100 of the present embodiment, the rigidity of the guide wire 100 can be gradually reduced toward the distal end side.

In the present embodiment, the base portion 40B of the wire 40 includes the first tapered portion 42, and the second tapered portion 44 provided in the distal end side of the first tapered portion 42 and having a slope different from that of the first tapered portion 42. According to the guide wire 100 of the present embodiment, the guide wire 100 can be designed to have a more preferred rigidity at each position along the center axis Ax of the guide wire 100.

In the present embodiment, the main body part 10 has the parallel portion 11 in which the base portion 40B and the folded-back portion 40A extend along the axis Ax of the main body part 10, and the base portion 40B and the folded-back portion 40A are spaced in the parallel portion 11. According to the guide wire 100 of the present embodiment, the presence of the parallel portion 11 enables the guide wire 100 to have a more preferred rigidity at each position along the center axis Ax of the guide wire 100.

In the present embodiment, the leading portion 20 has the reinforcing portion 28 located at a connection site with the main body part 10. According to the guide wire 100 of the present embodiment, detachment of the leading portion 20 from the main body part 10 can be prevented.

In the present embodiment, the leading portion 20 includes the wire 40, and the main body part 10 includes the coil 50 joined to the wire 40 via the distal end side-jointing material 61. The reinforcing portion 28 is formed with a portion of the distal end side-jointing material 61. According to the guide wire 100 of the present embodiment, detachment of the leading portion 20 from the main body part 10 can be prevented while avoiding structural complexity.

In the present embodiment, the distal end side-jointing material 61 is provided inside the coil 50. According to the guide wire 100 of the present embodiment, the reinforcing effect of the distal end side-jointing material 61 can be improved, effectively preventing detachment of the leading portion 20 from the main body part 10.

In the present embodiment, the leading portion 20 has a loop-shaped structure formed with a part of the base portion 40B and a part of the folded-back portion 40A of the wire 40, and the main body part 10 includes a part of the base portion 40B and a part of the folded-back portion 40A of the wire 40, and the distal end side-jointing material 61 is provided between the base portion 40B and the folded-back portion 40A. According to the guide wire 100 of the present embodiment, the base portion 40B and the folded-back portion 40A can be joined via the distal end side-jointing material 61 as the reinforcing portion 28, leading to a simplified structure.

In the present embodiment, the main body part 10 has the joining region R1 in which a part of the base portion 40B and a part of the folded-back portion 40A of the wire 40 are joined to the main body part 10, and the non-joining region R2 in which a part of the base portion 40B and a part of folded-back portion 40A are not joined to the main body part 10. According to the guide wire 100 of the present embodiment, the presence of the non-joining region R2 can ensure the softness of the main body part 10 while the presence of the joining region R1 can ensure the joining between the wire 40 and the coil 50.

In the present embodiment, the width of a part of the reinforcing portion 28 in a direction orthogonal to the axis Ax of the reinforcing portion 28 decreases toward the distal end of the reinforcing portion 28. According to the guide wire 100 of the present embodiment, an excessive rigidity of the connection site between the main body part 10 and the leading portion 20 can be prevented, which in turn can prevent the occurrence of kinking at that site.

In the present embodiment, the width of another part of the reinforcing portion 28 in a direction orthogonal to the axis Ax of the reinforcing portion 28 increases toward the distal end of the reinforcing portion 28. According to the guide wire 100 of the present embodiment, detachment of the leading portion 20 from the main body part 10 can be effectively prevented.

In the present embodiment, the outer peripheral surface of the reinforcing portion 28 has a curved surface recessed toward a radially inward side of the reinforcing portion 28. According to the guide wire 100 of the present embodiment, an excessive rigidity of the connection site between the main body part 10 and the leading portion 20 can be prevented while detachment of the leaving portion 20 from the main body part 10 can be effectively prevented.

In the present embodiment, the reinforcing section 28 may be formed with a welded portion of the leading portion 20 and the main body part 10. According to this configuration, detachment of the leading portion 20 from the main body part 10 can be effectively prevented.

The guide wire 100 of the present embodiment comprises the first coating 31 having a first slidability, which covers at least a part of the leading portion 20, and the second coating 32 having a second slidability different from the first slidability, which covers at least a part of the main body part 10. According to the guide wire 100 of the present embodiment, an appropriate slidability can be given to each part of the guide wire 100.

In the present embodiment, the first slidability of the first coating 31 is lower than the second slidability of the second coating 32. According to the guide wire 100 of the present embodiment, the slidability of the main body part 10 can be ensured while the slidability of the leading portion 20 can be relatively lowered to increase the drillability of the leading portion 20. This can improve the passability through the lesion 220.

In the present embodiment, the first coating 31 is hydrophobic, and the second coating 32 is hydrophilic. According to the guide wire 100 of the present embodiment, the first slidability of the first coating 31 can be lowered relative to the second slidability of the second coating 32.

In the present embodiment, the distal end portion of the main body part 10 is covered with the first coating 31. According to the guide wire 100 of the present embodiment, the slidability of the distal end portion of the main body part 10 which enters into the lesion 220 during a procedure can be relatively lowered to effectively improve the passability through the lesion 220.

In the method of manufacturing the guide wire 100 of the present embodiment, a bending work of pressing the wire 40 against the pin 410 to bend the wire 40 into a loop-shaped structure is performed, and the wire 40 is then joined to the coil 50. According to the method of manufacture according to the present embodiment, the wire 40 can be processed stably into a desired loop-shaped structure, enabling effective manufacturing of the guide wire 100 having the leading portion 20 having a loop-shaped structure.

In the present embodiment, the wire 40 is inserted into the hollow space of the coil 50 prior to the above bending work. According to the method of manufacture of the present embodiment, an operation of inserting the wire 40 into the hollow space of the coil 50 can be efficiently conducted. This, in turn, enables the guide wire 100 to be manufactured efficiently.

In the present embodiment, a soldering material is used to join the wire 40 to the coil 50. According to the method of manufacture of the present embodiment, the wire 40 can be more reliably joined to the coil 50.

In the present embodiment, for joining the wire 40 to the coil 50, a soldering material is fed to the hollow space of the coil 50 through a pitch portion of the coil 50. According to the method of manufacture of the present embodiment, the wire 40 can be more reliably joined to the coil 50.

In the present embodiment, the reinforcing portion 28 is formed with a soldering material upon joining of the wire 40 to the coil 50. According to the method of manufacture of the present embodiment, the reinforcing portion 28 integrated with the joining portion can be formed, effectively preventing the detachment of the leading portion 20 from the main body part 10.

In the present embodiment, the bending work of bending the wire 40 into a loop-shaped structure includes deforming a cross section of the wire 40. According to the method of manufacture of the present embodiment, a desired property can be given to each of the portions of the wire 40 which form the leading portion 20 by deforming a cross sectional shape at each portion of the wire 40.

In the present embodiment, the bending work of bending the wire 40 into a loop-shaped structure includes forming the narrowed portion 29. According to the method of manufacture of the present embodiment, an excessive rigidity of the connection site between the main body part 10 and the leading portion 20 can be prevented, which in turn can prevent the occurrence of kinking at that site.

In the present embodiment, grinding work of the wire 40 may be performed prior to the bending work of bending the wire 40 into a loop-shaped structure. According to the method of manufacture of the present embodiment, a desired property can be given to each of the portions of the wire 40 which form the leading portion 20 by adjusting an outer diameter and/or a cross sectional shape at each portion of the wire 40.

In the present embodiment, the above grinding work may include mechanical grinding work, electrolytic grinding work, or chemical grinding work. According to this method of manufacture, a desired surface condition can be achieved for the wire 40 which forms the leading portion 20.

### (Second embodiment)

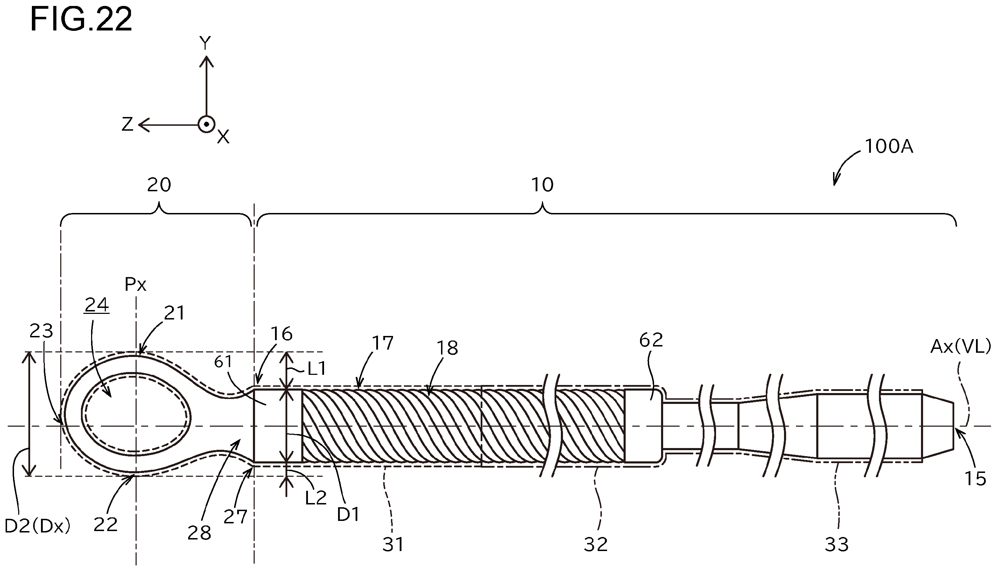

FIG. 22 shows a diagram schematically illustrating a configuration of a guide wire 100A according to a second embodiment. FIG. 22 shows an appearance of the guide wire 100A as viewed from a direction of the X-axis. Hereinafter, among the configurations of the guide wire 100A according to the second embodiment, the descriptions of the same configurations as those of the guide wire 100 according to the first embodiment as described above will be approximately omitted, and the same symbols will be assigned.

In the guide wire 100A according to the second embodiment, the configuration of the leading portion 20 differs from the configuration of the leading portion 20 of the guide wire 100 according to the first embodiment. More specifically, the protrusion amount L1 from the outer peripheral surface 17 of the main body part 10 at the first end portion 21 of the leading portion 20 is different from the protrusion amount L2 from the outer peripheral surface 17 of the main body part 10 at the second end portion 22. Specifically, the protrusion amount L1 is larger than the protrusion amount L2.

According to the guide wire 100A of the second embodiment, when the leading portion 20 rotates to drill the lesion 220, the first end portion 21 having a larger protrusion amount L1 can well drill the lesion 220, effectively improving the passability through the lesion 220.

### (Third embodiment)

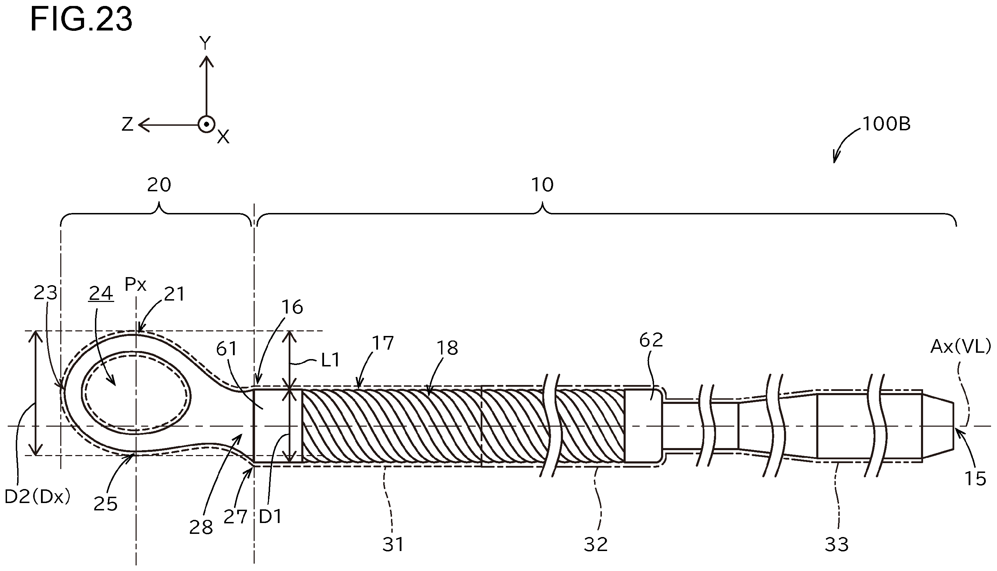

FIG. 23 shows a diagram schematically illustrating a configuration of a guide wire 100B according to a third embodiment. FIG. 23 shows an appearance of the guide wire 100B as viewed from a direction of the X-axis. Hereinafter, among the configurations of the guide wire 100B according to the third embodiment, the descriptions of the same configurations as those of the guide wire 100 according to the first embodiment as described above will be approximately omitted, and the same symbols will be assigned.

In the guide wire 100B according to the third embodiment, the configuration of the leading portion 20 differs from the configuration of the leading portion 20 of the guide wire 100 according to the first embodiment. More specifically, the leading portion 20 has the first end portion 21 located outside the outer peripheral surface 17 of the main body part 10 in the Y-axis direction, a direction in which the leading portion 20 has the largest outer diameter Dx. In the leading portion 20, the third end portion 25 opposite to the first end portion 21 across the virtual line VL extended from the center axis Ax of the main body part 10 is located inside the outer peripheral surface 17 of the main body part 10.

According to the guide wire 100B of the third embodiment, when the leading portion 20 rotates to drill the lesion 220, the first end portion 21 protruded from the outer peripheral surface 17 of the main body part 10 enables good drilling by the lesion 220, effectively improving the passability through the lesion 220. Moreover, the third end portion 25 opposite to the first end portion 21 in the leading portion 20 is located inside the outer peripheral surface 17 of the main body part 10, thereby preventing the outer diameter of the leading portion 20 from being excessively large. This in turn can prevent the leading portion 20 from unintentionally making contact with the blood vessel 200 to damage the blood vessel 200.

### (Fourth embodiment)

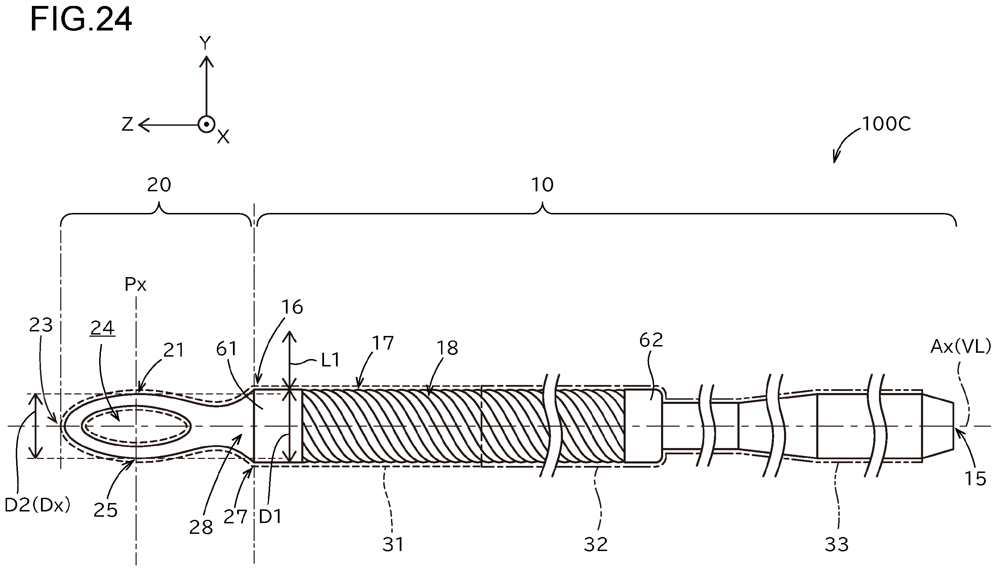

FIG. 24 shows a diagram schematically illustrating a configuration of a guide wire 100C according to a fourth embodiment. FIG. 24 shows an appearance of the guide wire 100C as viewed from a direction of the X-axis. Hereinafter, among the configurations of the guide wire 100C according to the fourth embodiment, the descriptions of the same configurations as those of the guide wire 100B according to the third embodiment as described above will be approximately omitted, and the same symbols will be assigned.

In the guide wire 100C according to the fourth embodiment, the configuration of the leading portion 20 differs from the configuration of the leading portion 20 of the guide wire 100B according to the third embodiment. More specifically, in the Y-axis direction, a direction in which the leading portion 20 has the largest outer diameter Dx, the two end portions of the leading portion 20 (the first end portion 21 and the third end portion 25) are both located inside the outer peripheral surface 17 of the main body part 10.

According to the guide wire 100C of the fourth embodiment, the outer diameter of the leading portion 20 can be prevented from being excessively large, which in turn can prevent the leading portion 20 from unintentionally making contact with the blood vessel 200 to damage the blood vessel 200.

### (Fifth embodiment)

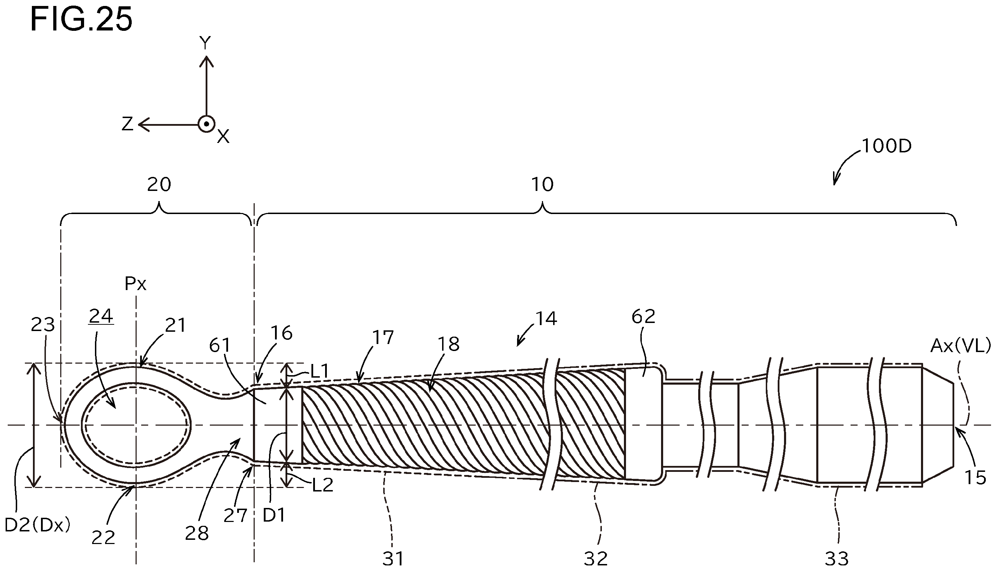

FIG. 25 shows a diagram schematically illustrating a configuration of a guide wire 100D according to a fifth embodiment. FIG. 25 shows an appearance of the guide wire 100D as viewed from a direction of the X-axis. Hereinafter, among the configurations of the guide wire 100D according to the fifth embodiment, the descriptions of the same configurations as those of the guide wire 100 according to the first embodiment as described above will be approximately omitted, and the same symbols will be assigned.

In the guide wire 100D according to the fifth embodiment, the configuration of the main body part 10 differs from the configuration of the main body part 10 of the guide wire 100 according to the first embodiment. More specifically, the main body part 10 has a tapered portion 14 having a diameter reduced from the proximal end side toward the distal end side. In the present embodiment, the tapered portion 14 is formed as a result of the coil 50 (see FIG. 5) having a diameter reduced from the proximal end side toward the distal end side. The presence of the tapered portion 14 can confer a lesion discharge function on the main body part 10, the lesion discharge function comprising discharging the small pieces of the lesion 220 generated by contact between the leading portion 20 which is rotating and the lesion 220 from the distal end side toward the proximal end side of the main body part 10.

According to the guide wire 100D of the fifth embodiment, the small pieces of the lesion 220 generated by contact between the leading portion 20 and the lesion 220 are more smoothly discharged from the distal end side toward the proximal end side of the main body part 10. This can more effectively present the small pieces from interfering with drilling by the leading portion 20, effectively improving the passability through the lesion 220.

### (Sixth embodiment)

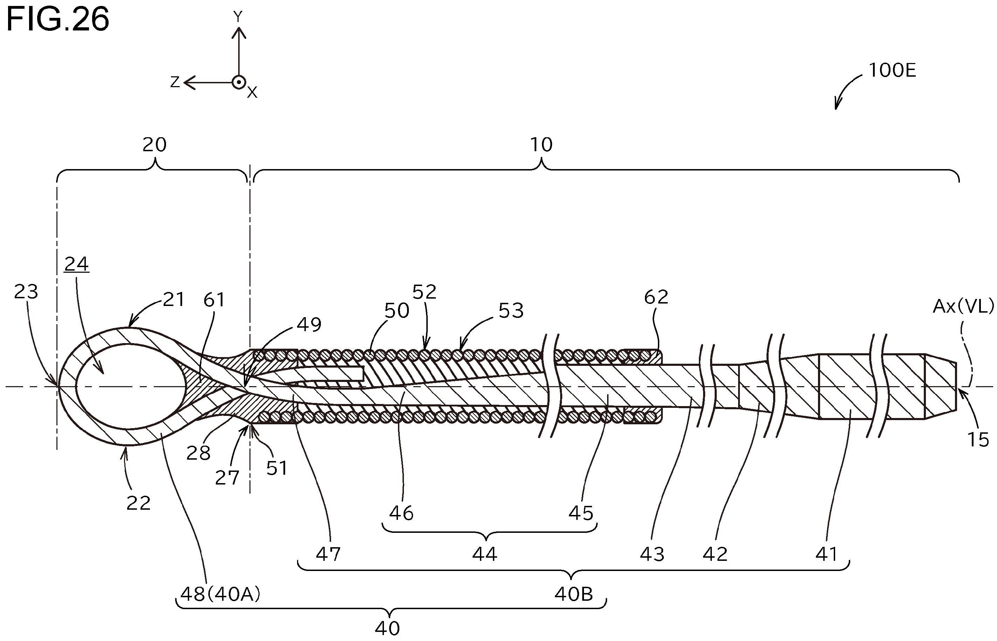

FIG. 26 shows a diagram schematically illustrating a configuration of a guide wire 100E according to a sixth embodiment. FIG. 26 shows a Y-Z longitudinal section of the guide wire 100E. Hereinafter, among the configurations of the guide wire 100E according to the sixth embodiment, the descriptions of the same configurations as those of the guide wire 100 according to the first embodiment as described above will be approximately omitted, and the same symbols will be assigned.

In the guide wire 100E according to the sixth embodiment, the configuration of the leading portion 20 differs from the configuration of the leading portion 20 of the guide wire 100 according to the first embodiment. More specifically, the leading portion 20 has a loop-shaped structure formed with a portion of the wire 40 and comprises an intersecting portion 49 in which the wire 40 intersects at the proximal end portion. The intersecting portion 49 is a portion in which the first small-diameter portion 47 forming the base portion 40B and the second small-diameter portion 48 forming the folded-back portion 40A of the wire intersect. The intersecting portion 49 is a portion in which a portion of the wire 40 overlaps with another portion of the wire 40 as viewed from a direction orthogonal to the center axis Ax of the main body part 10, and the two portions may be in contact with each other, or may be spaced. In the present embodiment, a part of the reinforcing portion 28 is formed with the distal end side-jointing material 61 which covers the outer periphery of the intersecting portion 49.

In the guide wire 100E according to the sixth embodiment, the intersecting portion 49 in which the wire 40 intersects provided at the proximal end portion of the leading portion 20 can improve the rigidity of the distal end side of the leading portion 20. This enables the leading portion 20 to efficiently drill the lesion 220, improving the passability though the lesion 220. In the guide wire 100E according to the sixth embodiment, a part of the reinforcing portion 28 is formed with the distal end side-jointing material 61 which covers the outer periphery of the intersecting portion 49. This can effectively improve the rigidity of the proximal end portion of the leading portion 20, improving the passability though the lesion 220.

### (Seventh embodiment)

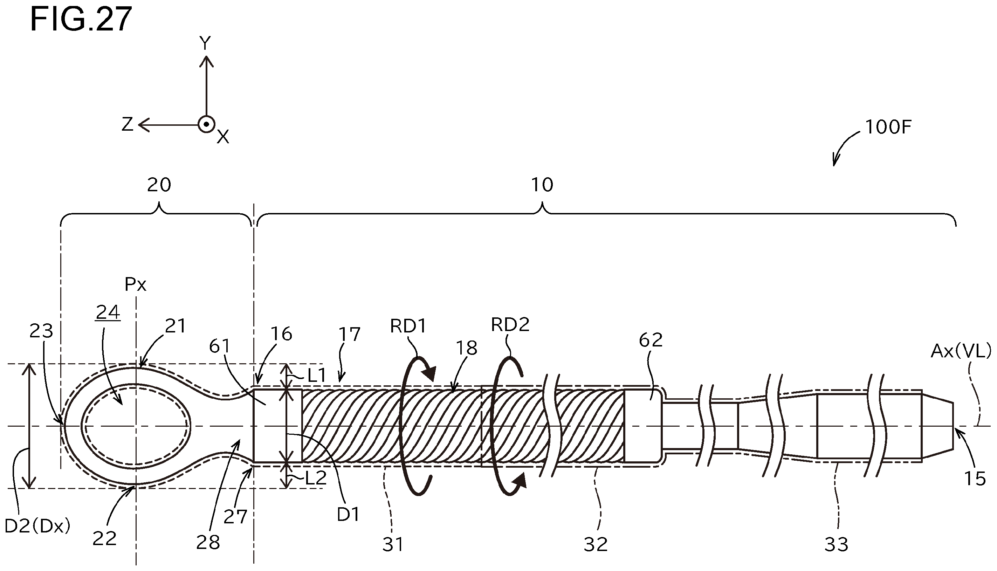

FIG. 27 shows a diagram schematically illustrating a configuration of a guide wire 100F according to a seventh embodiment. FIG. 27 shows an appearance of the guide wire 100F as viewed from a direction of the X-axis. Hereinafter, among the configurations of the guide wire 100F according to the seventh embodiment, the descriptions of the same configurations as those of the guide wire 100 according to the first embodiment as described above will be approximately omitted, and the same symbols will be assigned.

In the guide wire 100F according to the seventh embodiment, the configuration of the main body part 10 differs from the configuration of the main body part 10 of the guide wire 100 according to the first embodiment. More specifically, a running direction of the groove 18 formed on the outer peripheral surface 17 of the main body part 10 is a direction of S-winding, instead of Z-winding. In the present embodiment, the coil 50 (see FIG. 5) is S-wound, and the running direction of the groove 53 formed on the outer peripheral surface 52 of the coil 50 is also a direction of S-winding. Therefore, the running direction of the groove 18 is also a direction of S-winding.

When performing a treatment using the guide wire 100F according to the seventh embodiment, a professional may switch a rotational direction of the guide wire 100F between the first rotational direction RD1 and the second rotational direction RD2 opposite to the first rotational direction RD1. Alternatively, the professional may fix the rotational direction of the guide wire 100F in the first rotational direction RD1. In the present embodiment, the first rotational direction RD1 is the clockwise direction as centered around the axis Ax extending from the proximal end to the distal end of the main body part 10. When the coil 50 is rotated in the first rotational direction RD1, the pitch of the coil 50 becomes narrower. The lesion passability when the guide wire 100F is rotated in the first rotational direction RD1 is lower than the lesion passability when the guide wire 100F is rotated in the second rotational direction RD2. The torque generated when the guide wire 100F is rotated in the first rotational direction RD1 is higher than the torque generated when the guide wire 100F is rotated in the second rotational direction RD2.

According to the guide wire 100F of the seventh embodiment, the lesion passability can be changed according to the rotational directions, without having a complex configuration.

### (Eighth embodiment)

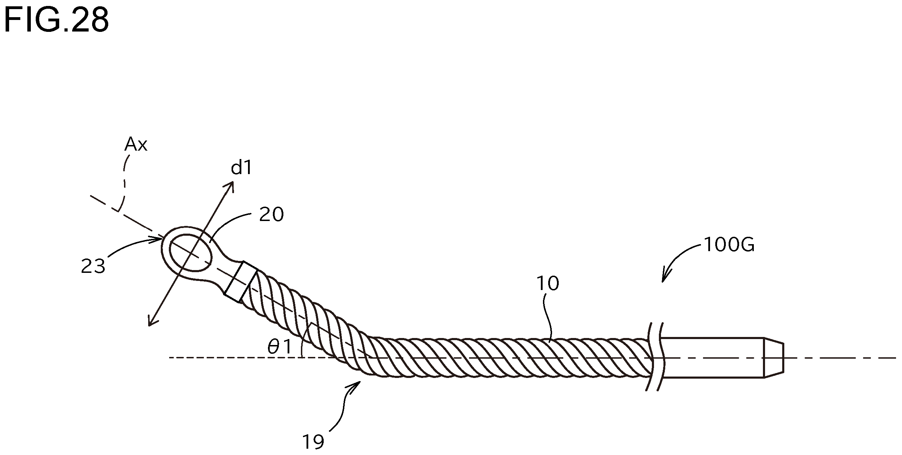

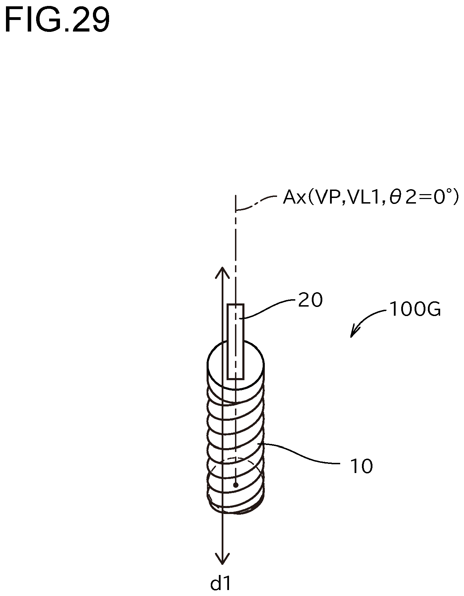

FIGs. 28 and 29 show diagrams schematically illustrating a configuration of a guide wire 100G according to an eighth embodiment. FIG. 28 shows a side view of the guide wire 100G. FIG. 28 shows a front view of the guide wire 100G. Hereinafter, among the configurations of the guide wire 100G according to the eighth embodiment, the descriptions of the same configurations as those of the guide wire 100 according to the first embodiment as described above will be approximately omitted, and the same symbols will be assigned.

In the guide wire 100G according to the eighth embodiment, the configuration of the main body part 10 differs from the configuration of the main body part 10 of the guide wire 100 according to the first embodiment. More specifically, the main body part 10 has a curved portion 19 which is pre-bent. The curved portion 19 is a portion which is pre-bent at a predetermined bending angle θ1. In the method of manufacturing the guide wire 100 (FIG. 18), the curved portion 19 is formed by performing bending work on the distal end portion of the main body part 10 after joining the wire 40 to the coil 50. The bending angle θ1 is, for example, 3 degrees or more and 30 degrees or less. The bending angle θ1 may be 5 degrees or more and 25 degrees or less, or may be 10 degrees or more and 20 degrees or less. The distance from the distal end 23 of the guide wire 100D to the curved portion 19 is, for example, 0.8 mm or more and 3.0 mm or less. The distance may be 1.0 mm or more and 2.5 mm or less, or may be 1.2 mm or more and 2.0 mm or less.

As shown in FIG. 28, the leading portion 20 has the largest outer diameter Dx in a direction orthogonal to the center axis Ax (hereinafter referred to as a "largest-outer diameter direction d1"). At this time, as shown in FIG. 29, an angle between the first virtual line VL1 parallel to the largest-outer diameter direction d1 and a first virtual plane VP encompassing the center axis Ax at the curved portion 19 of the main body part 10 (hereinafter referred to as a "specific angle 02") is, for example, 80 degrees or less. The specific angle θ2 may be 10 degrees or less. In the present embodiment, the specific angle θ2 is 0 degree. In other words, the first virtual line VL1 is parallel to the first virtual plane VP. That is, the curved portion 19 is bent within a virtual plane including a straight line parallel to the largest-outer diameter direction d1.

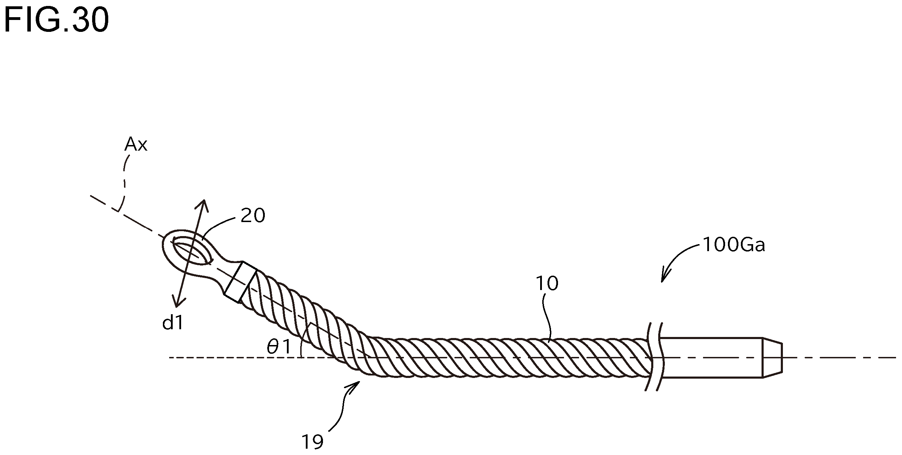

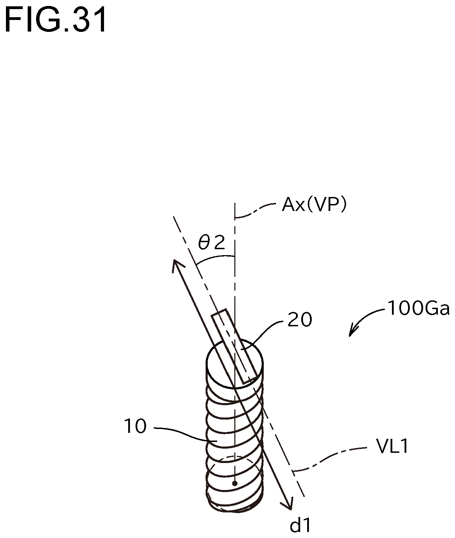

FIGs. 30 and 31 show diagrams schematically illustrating configurations of a guide wire 100Ga according to a variation of the eighth embodiment. FIG. 30 shows a side view of the guide wire 100Ga. FIG. 31 shows a front view of the guide wire 100Ga. In the guide wire 100Ga according a variation of the eighth embodiment, the specific angle θ2, an angle between the first virtual line VL1 parallel to the largest-outer diameter direction d1 and the first virtual plane VP encompassing the center axis Ax at the curved portion 19 of the main body part 10 is not 0 degree, but, for example, 10 degrees.

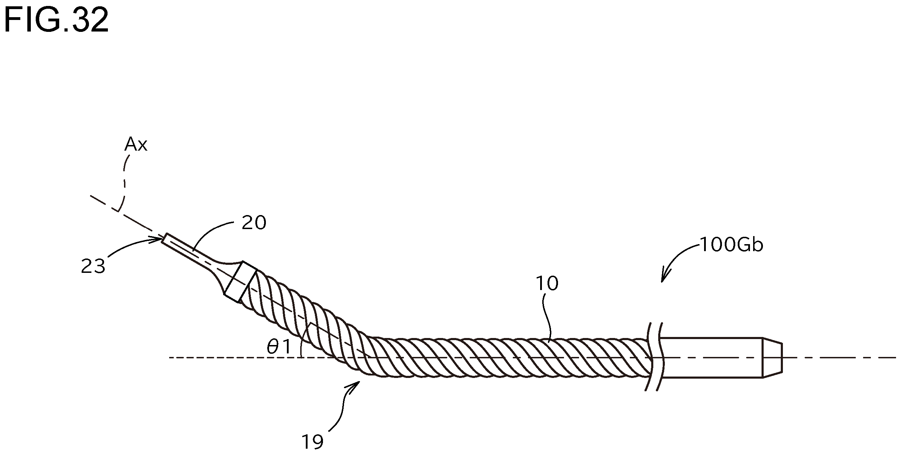

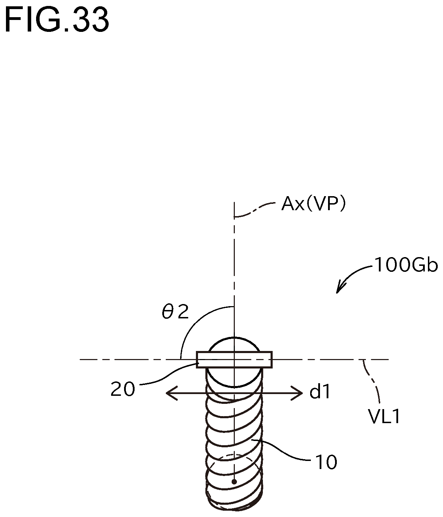

FIGs. 32 and 33 show diagrams schematically illustrating configurations of a guide wire 100Gb according to another variation of the eighth embodiment. FIG. 32 shows a side view of the guide wire 100Gb. FIG. 33 shows a front view of the guide wire 100Gb. In the guide wire 100Gb according to another variation of the eighth embodiment, the specific angle θ2 which is an angle between the first virtual line VL1 parallel to the largest-outer diameter direction d1 and the first virtual plane VP encompassing the center axis Ax at the curved portion 19 of the main body part 10 is more than 80 degrees or, for example, 90 degrees. In other words, the first virtual line VL1 is orthogonal to the first virtual plane VP.

According to the guide wires 100G, 100Ga, 100Gb of the eighth embodiment and variations thereof, the main body part 10 has the curved portion 19, and thus the leading portion 20 can be easily delivered to a position of the lesion 220 within the blood vessel 200. Moreover, the above specific angle θ2 of 80 degrees or less can improve the drillability of the leading portion 20, improving the passability through the lesion 220. The above specific angle θ2 of 10 degrees or less can further improve the drillability of the leading portion 20, further improving the passability through the lesion 220. The first virtual line VL1 being parallel to the first virtual plane VP can further improve the drillability of the leading portion 20, further improving the passability through the lesion 220. The lesion 220 may have both hard and soft portions. If the guide wire 100 is passed through a soft portion avoiding a hard portion, the guide wire 100 can be passed smoothly. When the distal end 23 of the guide wire 100 hits a hard lesion, and experiences resistance to bend toward a soft portion, then the guide wire 100 may advance so as to follow soft portions. To this end, the distal end 23 of the guide wire 100 needs to experience some amount of resistance from a hard portion. In a case where the above specified angle θ2 is more than 80 degrees, the upper and lower surfaces of the leading portion 20 are more likely to hit a hard portion of the lesion, facilitating change in the direction of advancing due to the resistance they experience. This can improve controllability of the guide wire 100 within the lesion 220. The first virtual line VL1 being orthogonal to the first virtual plane VP can effectively improve the controllability of the guide wire 100 within the lesion 220.

### (Ninth embodiment)

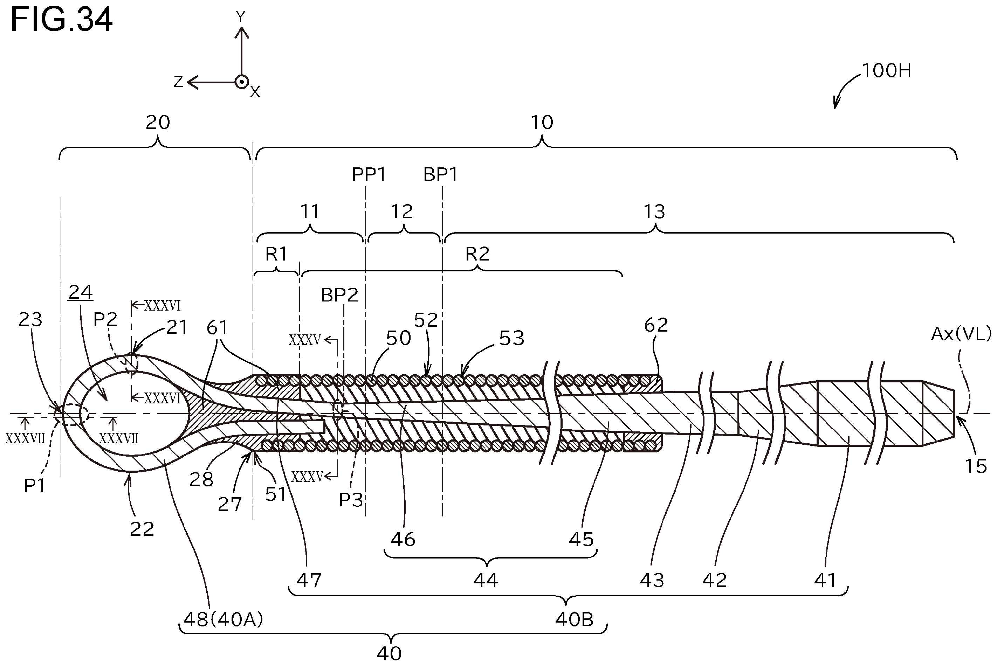

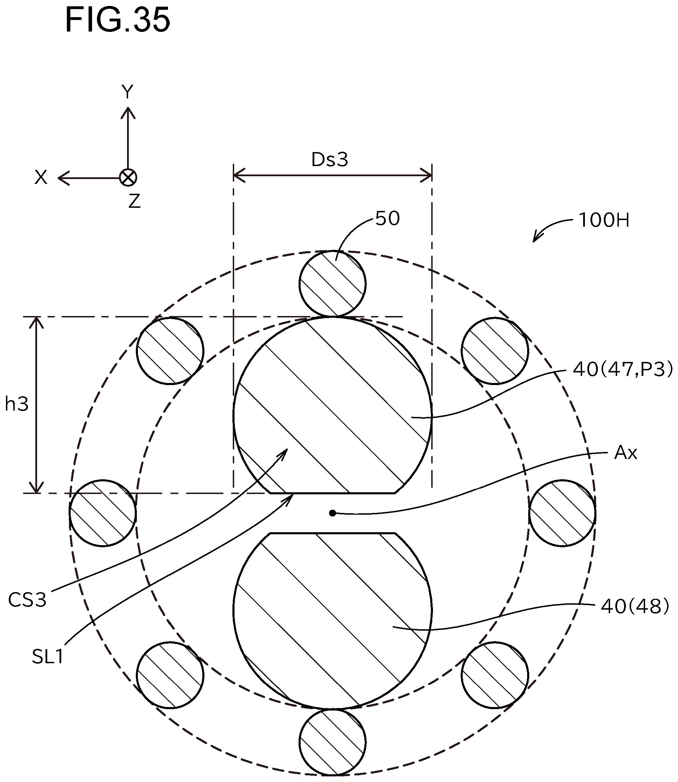

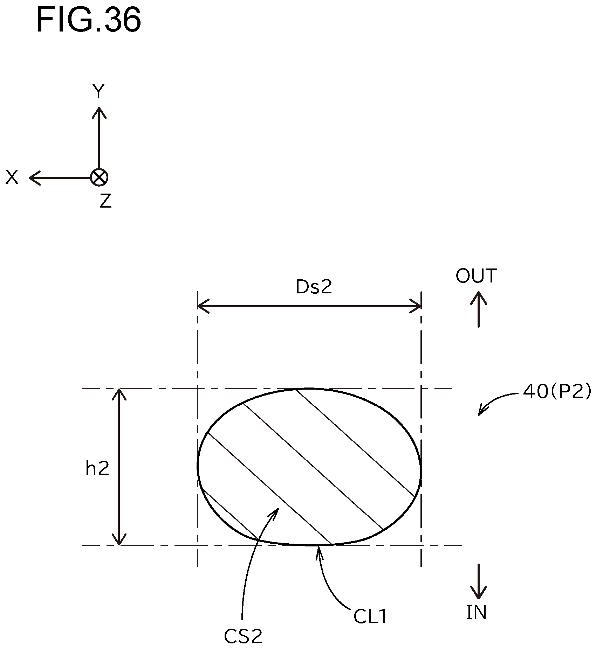

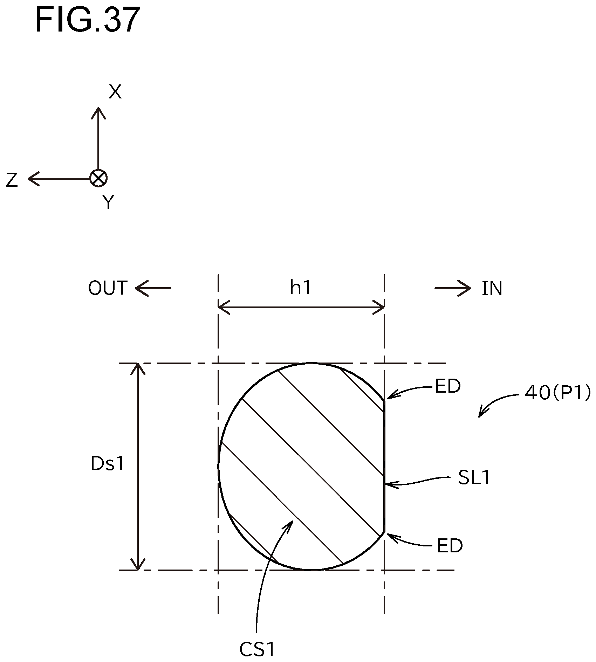

FIGs. 34 and 35 show diagrams schematically illustrating configurations of the guide wire 100H according to the ninth embodiment. FIG. 34 shows a Y-Z longitudinal section of the guide wire 100H. Fig. 35 shows an X-Y cross section of the guide wire 100H at the position of XXXV-XXXV in FIG. 34. FIGs. 36 and 37 show diagrams illustrating cross sections of the wire 40 in the guide wire 100H according to the ninth embodiment. Fig. 36 shows a cross section of the wire 40 at the position of XXXVI-XXXVI in FIG. 34. Fig. 37 shows a cross section of the wire 40 at the position of XXXVII-XXXVII in FIG. 34. Symbols indicating the outer peripheral side (OUT) and the central side (IN) in a loop of the leading portion 20 are shown in FIGs. 36 and 37. Hereinafter, among the configurations of the guide wire 100H according to the ninth embodiment, the descriptions of the same configurations as those of the guide wire 100 according to the first embodiment as described above will be approximately omitted, and the same symbols will be assigned.

In the guide wire 100H according to the ninth embodiment, the configuration of the wire 40 differs from the configuration of the wire 40 of the guide wire 100 according to the first embodiment. As shown in FIG. 35, the third cross section CS3 of the third portion P3 which is a portion included in the main body part 10 among the first small-diameter portion 47 of the wire 40 is segmentally circular in shape. That is, an outer rim of the third cross section CS3 consists of an arc line and a line segment connecting the two end portions of the arc line. A largest width Ds3 of the third cross section CS3 is larger than the length of a first straight line portion SL1 which corresponds to the above line segment. In the present embodiment, the cross section of a portion included in the main body part 10 among the second small-diameter portions 48 of the wire 40 is also segmentally circular in shape. The outer rim (outline) of the third cross section CS3 includes the first straight line portion SL1. In the present embodiment, a cross section of the proximal end side-second tapered portion 45 in the wire 40 is substantially circular in shape, and the shape of the cross section changes from a substantially circular form to a segmentally circular form at the distal end side-second tapered portion 46. The third portion P3 is an example of the specific portion.

As shown in FIG. 37, the first cross section CS1 of the first portion P1, which is a boundary portion between the first small-diameter portion 47 and the second small-diameter portion 48 in the wire 40 is not segmentally circular, but collapsed segmentally circular in shape. The outline of the first cross section CS1 includes the first straight line portion SL1. A largest width Ds1 of the first cross section CS1 is larger than the length of the first straight line portion SL1 which corresponds to the above line segment.

As shown in FIG. 36, the second cross section CS2 of the second portion P2 which is a portion included in the leading portion 20 among the first small-diameter portion 47 of the wire 40 is not segmentally circular, but collapsed segmentally circular in shape. The outline of the second cross section CS2 does not include a straight line portion. Among the outline of the second cross section CS2, a portion corresponding to the first straight line portion SL1 in the third cross section CS3 and the first cross section CS1 serves as a curved portion CL1. In the present embodiment, among the leading portion 20, an end portion opposite to the second portion P2 across the virtual line VL also has a cross section similar to that of the second portion P2.

The shape of the first cross section CS1 of the first portion P1 included in the leading portion 20 among the wire 40 is different from that of the second cross section CS2 of the second portion P2 also included in the leading portion 20. The area of the first cross section CS1 is different from that of the second cross section CS2. For example, the area of the first cross section CS1 is larger than that of the second cross section CS2. The area of the second cross section CS2 may be smaller than that of the third cross section CS3.

In the present embodiment, with regard to a ratio (hereinafter referred to as a "specific ratio") of a height h in a direction orthogonal to the largest width Ds to the largest width Ds of a cross section of the wire 40, a specific ratio (= h1/Ds1) of the first cross section CS1 differs from a specific ratio (= h2/Ds2) of the second cross section CS2. For example, the specific ratio of the first cross section CS1 is larger than that of the second cross section CS2. In the present embodiment, the specific ratio of the first cross section CS1 differs from the specific ratio (= h3/Ds3) of the third cross section CS3. For example, the specific ratio of the first cross section CS1 is smaller than that of the third cross section CS3.

According to the guide wire 100H of the ninth embodiment, the wire 40 includes a portion in which the outer rim of a cross section consists of an arc line and a line segment connecting the two end portions of the arc line, and thus the area occupied by the wire 40 in the cross section of the guide wire 100H can be increased. This can increase the distal end load of the guide wire 100H, effectively improving the passability through the lesion 220.

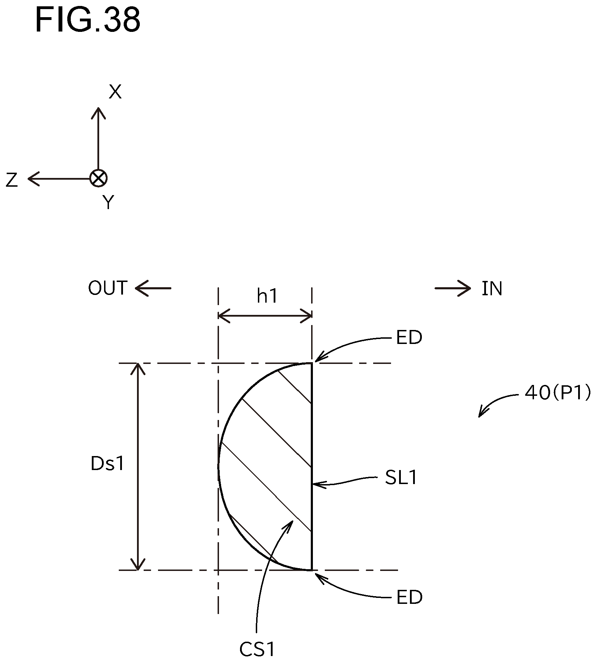

FIG. 38 shows a diagram illustrating a cross section of the wire 40 in a variation of the ninth embodiment. In a variation shown in FIG. 38, the first cross section CS1 of the first portion P1 in the wire 40 which forms the leading portion 20 is substantially semicircular in shape. The outer rim of the first cross section CS1 consists of an arch line and a line segment connecting the end portions of the arch line. The largest width Ds1 of the first cross section CS1 is substantially identical to the length of the first straight line portion SL1 which corresponds to the above line segment. In this variation, the first portion P1 is an example of the specific portion.

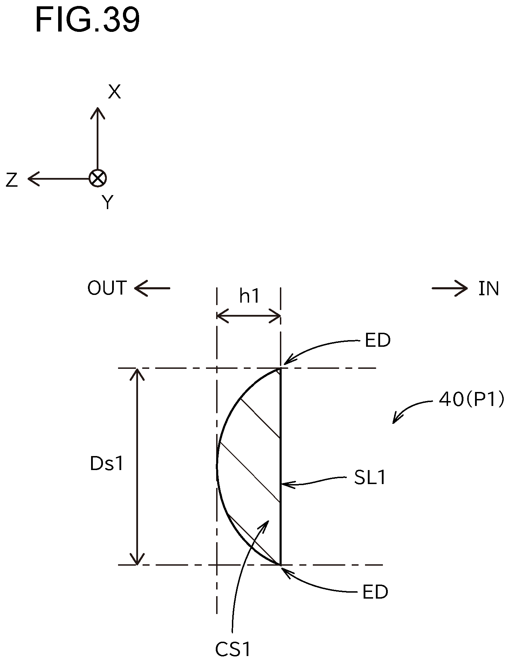

FIG. 39 shows a diagram illustrating a cross section of the wire 40 in another variation of the ninth embodiment. In a variation shown in FIG. 39, the first cross section CS1 of the first portion P1 in the wire 40 which forms the leading portion 20 has a substantially arch-like shape. The outer rim of the first cross section CS1 consists of an arch line and a line segment connecting the end portions of the arch line. The largest width Ds1 of the first cross section CS1 is substantially identical to the length of the first straight line portion SL1 which corresponds to the above line segment. In this variation, the first portion P1 is an example of the specific portion.

### (Tenth embodiment)

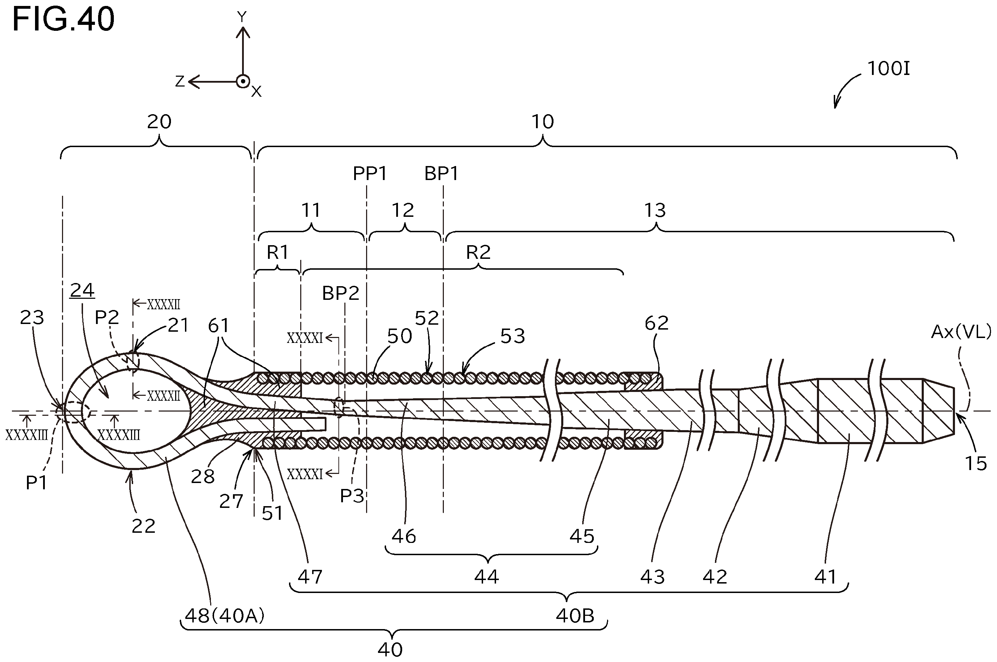

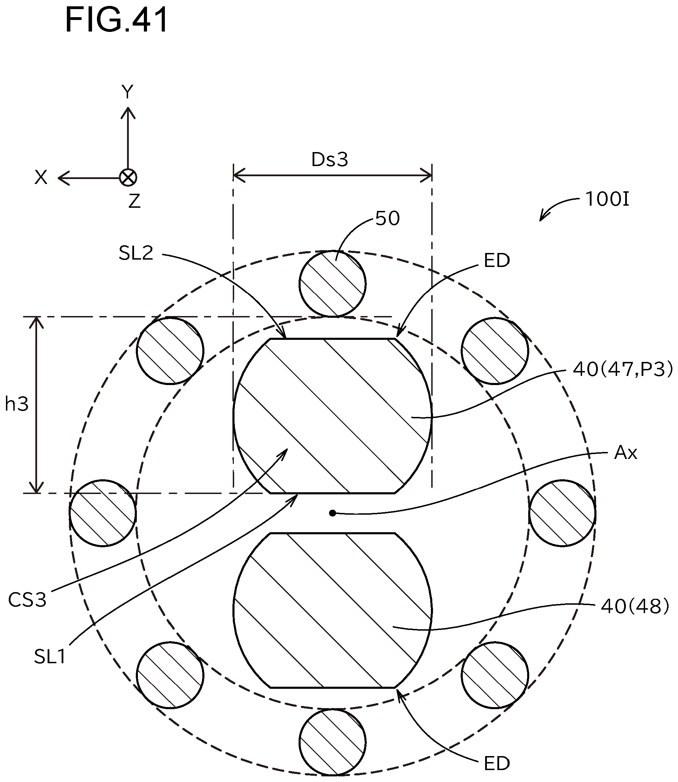

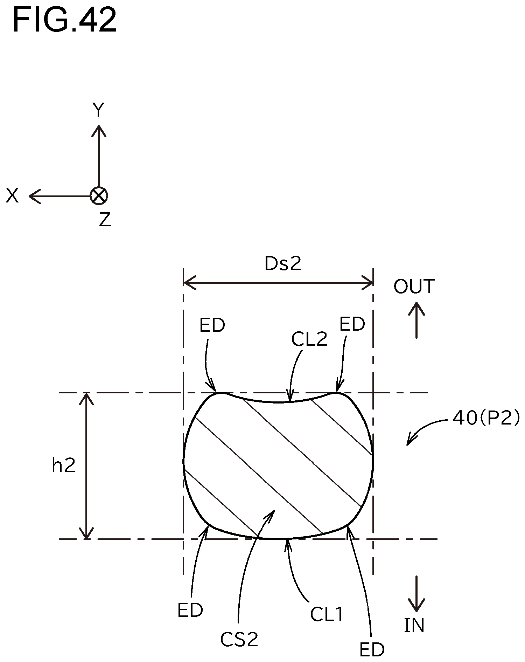

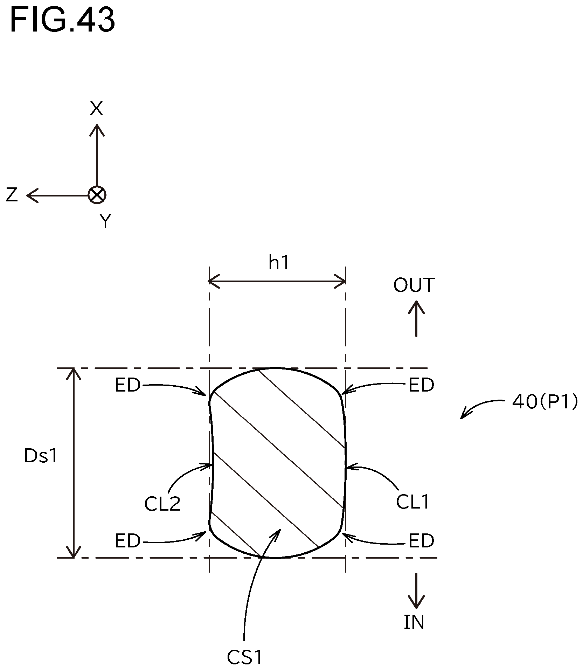

FIGs. 40 and 41 show diagrams schematically illustrating configurations of the guide wire 100I according to a tenth embodiment. FIG. 40 shows a Y-Z longitudinal section of the guide wire 100I. FIG. 41 shows an X-Y cross section of the guide wire 1001 at the position of XXXX1-XXXX1 cross section in FIG. 40. FIGs. 42 and 43 show diagrams illustrating cross sections of the wire 40 in the guide wire 1001 according to the tenth embodiment. FIG. 42 shows a cross section of the wire 40 at the position of XXXXII-XXXXII in FIG. 40. FIG. 43 shows a cross section of the wire 40 at the position of XXXXIII-XXXXIII in FIG. 40. Symbols indicating the outer peripheral side (OUT) and the central side (IN) in a loop of the leading portion 20 are shown in FIGs. 42 and 43. Hereinafter, among the configurations of the guide wire 100I according to the tenth embodiment, the descriptions of the same configurations as those of the guide wire 100 according to the first embodiment as described above will be approximately omitted, and the same symbols will be assigned.

In the guide wire 1001 according to the tenth embodiment, the configuration of the wire 40 differs from the configuration of the wire 40 of the guide wire 100 according to the first embodiment. As shown in FIG. 41, the third cross section CS3 of the third portion P3 which is a portion included in the main body part 10 among the first small-diameter portion 47 of the wire 40 is substantially rectangular in shape. More specifically, the third cross section CS3 is the shape of a circle from which the top and bottom are cut off from the circle by two opposing strings across the center of the circle.
In the present embodiment, a cross section of a portion included in the main body part 10 among the second small-diameter portions 48 of the wire 40 is also substantially rectangular in shape. The outline of the third cross section CS3 includes the first straight portion SL1 and the second straight portion SL2 which are opposite to each other across the center of gravity of that cross section. Edges ED are formed at the both end portions of the first straight portion SL1 and the both end portions of the second straight portion SL2 in the third portion P3 of the wire 40. The edges ED are shaped so as to scrape the lesion 220 when the leading portion 20 is rotated around the center axis Ax while pressed against the lesion 220. The leading portion 20 can enter into a space created by scraping the lesion 220 with the edges ED to form a through-hole in the lesion 220. In the present embodiment, a cross section of the proximal end side-second tapered portion 45 in the wire 40 is substantially circular in shape, and the shape of the cross section changes from a substantially circular form to a substantially rectangular form at the distal end side-second tapered portion 46.

As shown in FIG. 43, the first cross section CS1 of the first portion P1, which is a boundary between the first small-diameter portion 47 and the second small-diameter portion 48 in the wire 40 is substantially rectangular in shape. However, the outline of the first cross section CS1 does not includes a straight line portion. Among the outline of the first cross section CS1, a portion of an inner peripheral side which corresponds to the first straight portion SL1 in the third cross section CS3 is a convex curved portion CL1. Among the outline of the first cross section CS1, a portion of an outer peripheral side which corresponds to the second straight portion SL2 in the third cross section CS3 is a concave curved portion CL2. The edges ED are formed at the both end portions of the curved portion CL1 and the both end portions of the curved portion CL2 in the first portion P1 of the wire 40.

As shown in FIG. 42, the second cross section CS2 of the second portion P2 which is a portion included in the leading portion 20 among the first small-diameter portion 47 of the wire 40 is substantially rectangular in shape. However, the outline of the second cross section CS2 does not includes a straight line portion. A portion of an inner peripheral side which corresponds to the first straight portion SL1 in the third cross section CS3 among the outline of the second cross section CS2 serves as the convex curved portion CL1. A portion of an outer peripheral side which corresponds to the second straight portion SL2 in the third cross section CS3 among the outline of the second cross section CS2 serves as the concave curved portion CL2. The edges ED are formed at the both end portions of the curved part CL1 and the both end portions of the curved portion CL2 in the second portion P2 of the wire 40. The curvature of the convex curved portion CL1 in the outline of the second cross section CS2 may be larger than that of the convex curved portion CL1 in the outline of the first cross section CS1. The curvature of the convex curved portion CL2 in the outline of the second cross section CS2 may be larger than that of the concave curved portion CL2 in the outline of the first cross section CS1. In the present embodiment, an end portion of the leading portion 20 which is opposite to the second portion P2 across the virtual line VL also has a similar cross section as the second portion P2.

The shape of the first cross section CS1 of the first portion P1 included in the leading portion 20 among the wire 40 is different from that of the second cross section CS2 of the second portion P2 also included in the leading portion 20. The area of the first cross section CS1 is different from that of the second cross section CS2. For example, the area of the first cross section CS1 is larger than that of the second cross section CS2. The area of the second cross section CS2 may be smaller than that of the third cross section CS3.

In the present embodiment, with regard to the ratio (hereinafter referred to as a "specific ratio") of the height h in a direction orthogonal to the largest width Ds to the largest width Ds of a cross section of the wire 40, the specific ratio (= h1/Ds1) of the first cross section CS1 differs from the specific ratio (= h2/Ds2) of the second cross section CS2. For example, the specific ratio of the first cross section CS1 is larger than that of the second cross section CS2. In the present embodiment, the specific ratio of the second cross section CS2 differs from the specific ratio (= h3/Ds3) of the third cross section CS3. For example, the specific ratio of the second cross section CS2 is smaller than that of the third cross section CS3.

According to the guide wire 100I of the tenth embodiment, the wire 40 includes a portion having a rectangular cross section, and thus the drillability of the leading portion 20 can be improved, effectively increasing the passability through the lesion 220.

### (Eleventh embodiment)

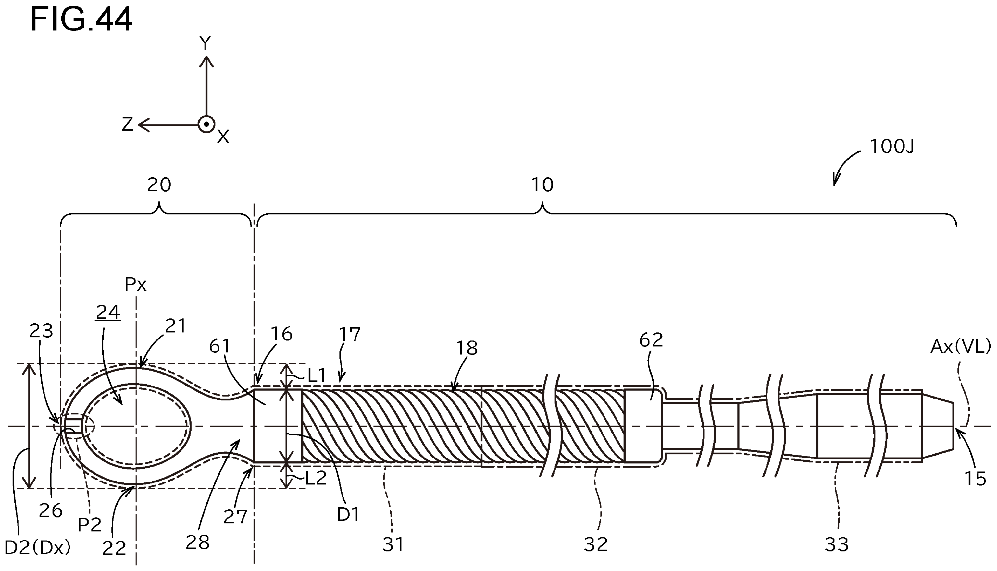

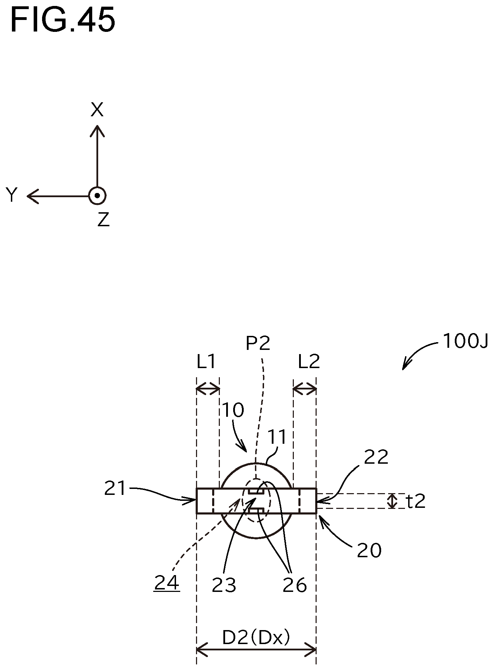

FIGs. 44 and 45 show diagrams schematically illustrating configurations of the guide wire 100J according to an eleventh embodiment. FIG. 44 shows an appearance of the guide wire 100J as viewed from a direction of the X-axis. FIG. 45 shows an appearance of the guide wire 100J as viewed from a direction of the Z-axis. Hereinafter, among the configurations of the guide wire 100J according to the eleventh embodiment, the descriptions of the same configurations as those of the guide wire 100 according to the first embodiment as described above will be approximately omitted, and the same symbols will be assigned.

In the guide wire 100J according to the eleventh embodiment, the configuration of the leading portion 20 differs from the configuration of the leading portion 20 of the guide wire 100 according to the first embodiment. More specifically, a pair of grooves 26 which are concave in a thickness direction (the X-axis direction) are formed in the second portion P2 which forms the distal end portion of the leading portion 20 among the wire 40. The grooves 26 are formed from the distal end of the second portion P2 to the proximal end along the center axis Ax. Due to the presence of the grooves 26, the second thickness t2 of the second portion P2 in the wire 40 is thinner than the thicknesses of other portions in the wire 40.

According to the guide wire 100J of the eleventh embodiment, the drillability of the leading portion 20 can be improved by arranging the second portion P2 having the second thickness t2 which is a relatively thin in a region forming the leading portion 20 among the wire 40. This can improve the passability through the lesion 220. Moreover, when a tensile force is applied to the leading portion 20, the second portion P2 having the second thickness t2 which is relatively thin may be preferentially subjected to fracturing, thereby more reliably preventing detachment of the leading portion 20 from the main body part 10.

In the guide wire 100J according to the eleventh embodiment, the second portion P2 having the second thickness t2 which is relatively thin is located at the distal end portion of the leading portion 20. According to this configuration, when a tensile force is applied to the leading portion 20, the distal end portion of the leading portion 20 may be preferentially subjected to fracturing, thereby more reliably preventing the leading portion 20 from detaching from the main body part 10.

### (Twelfth embodiment)

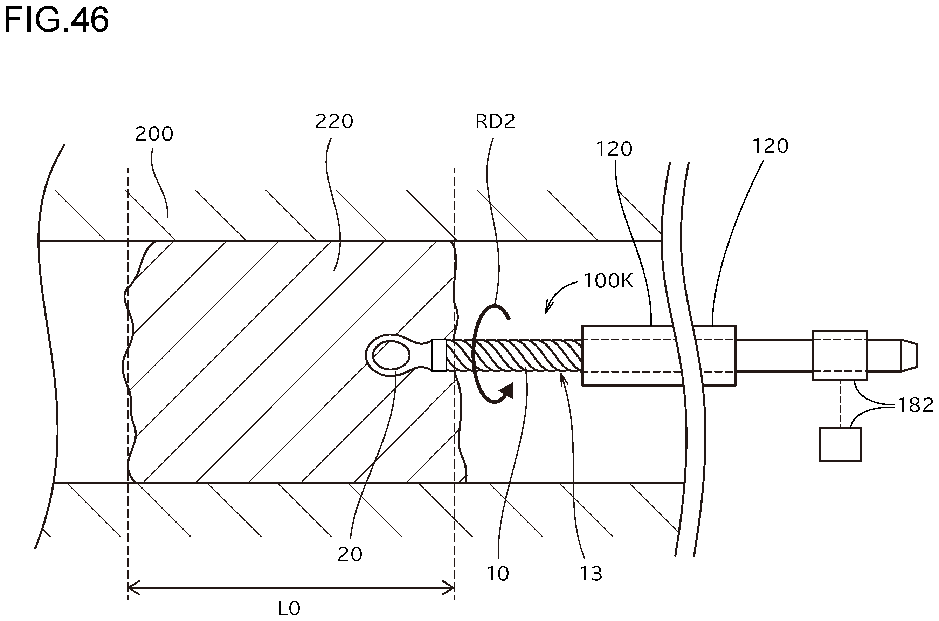

FIG. 46 shows a diagram illustrating a method of treatment using a guide wire 100K according a twelfth embodiment. Hereinafter, in the method of treatment according to the twelfth embodiment, the descriptions of the same method of treatment as that of the method of treatment according the first embodiment as described above will be appropriately omitted.

In the method of treatment according to the twelfth embodiment, the guide wire 100K is rotated using a driving force of an actuator 182 attached to the proximal end portion of the main body part 10 when the guide wire 100K is allowed to advance through the blood vessel 200 so that the leading portion 20 can enter into the lesion 220 (S150 in FIG. 7).

According to the method of treatment of the twelfth embodiment, the guide wire 100K can be stably rotated, improving the passability through the lesion 220. According to the method of treatment of the twelfth embodiment, a professional does not need to perform an action for rotating the guide wire 100K, allowing her or him to concentrate on other procedures. This can improve the ease of the procedures.

### (Thirteenth embodiment)

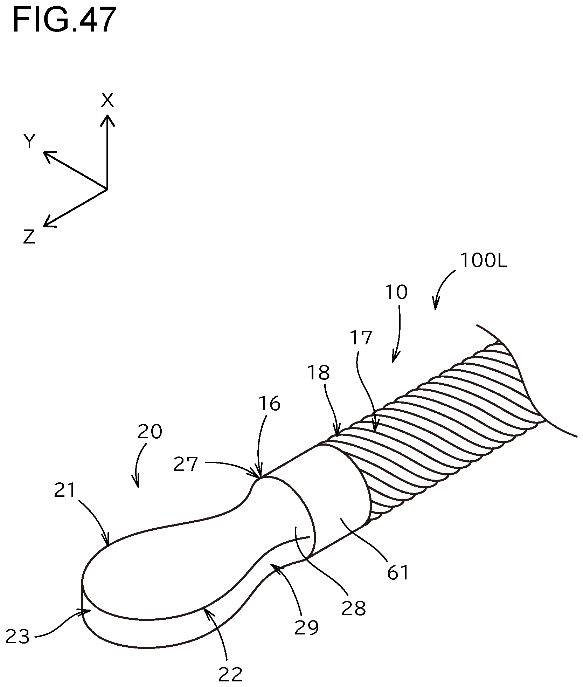

FIG. 47 shows a diagram schematically illustrating a configuration of a guide wire 100L according to a thirteenth embodiment. FIG. 47 shows a perspective view of an appearance of a distal end portion of the guide wire 100L. Hereinafter, among the configurations of the guide wire 100L according to the thirteenth embodiment, the descriptions of the same configurations as those of the guide wire 100 according to the first embodiment as described above will be approximately omitted, and the same symbols will be assigned.

In the guide wire 100L according to the thirteenth embodiment, the configuration of the leading portion 20 differs from the configuration of the leading portion 20 of the guide wire 100 according to the first embodiment. More specifically, the leading portion 20 has a paddle-shaped structure, instead of a loop-shaped structure. The paddle-shaped structure means a substantially flat plate-shaped structure in which a through-hole is not formed as in the loop-shaped structure. The leading portion 20 of such a shape can be made, for example, by pressing work or grinding work on the wire 40.

In the guide wire 100L according to the present embodiment, the paddle-shaped leading portion 20 can efficiently drill the lesion 220, improving the passability through the lesion 220.

### (Fourteenth embodiment)

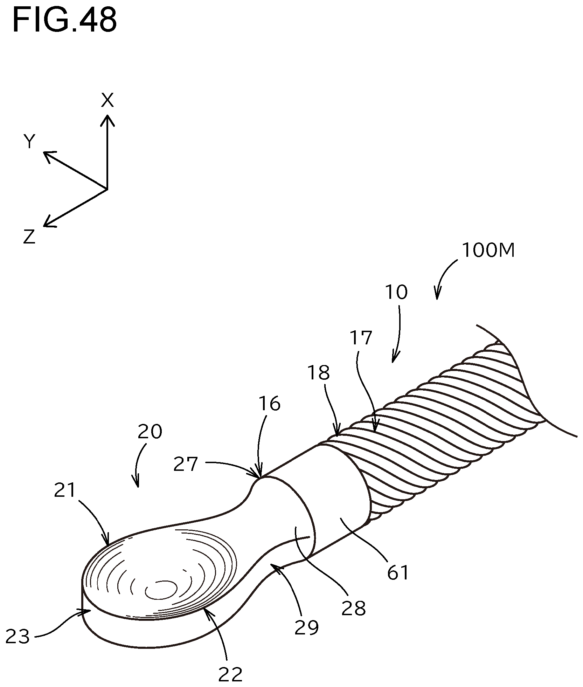

FIG. 48 shows a diagram schematically illustrating a configuration of a guide wire 100M according to a fourteenth embodiment. FIG. 48 shows a perspective view of an appearance of a distal end portion of the guide wire 100M. Hereinafter, among the configurations of the guide wire 100M according to the fourteenth embodiment, the descriptions of the same configurations as those of the guide wire 100 according to the first embodiment as described above will be approximately omitted, and the same symbols will be assigned.

In the guide wire 100M according to the fourteenth embodiment, the configuration of the leading portion 20 differs from the configuration of the leading portion 20 of the guide wire 100 according to the first embodiment. More specifically, the leading portion 20 has a spoon-shaped structure, instead of a loop-shaped structure. The spoon-shaped structure means a plate-shaped structure in which a through-hole is not formed as in the loop-shaped structure, but a concave portion is formed on a surface. The leading portion 20 of such a shape can be made, for example, by performing pressing work or grinding work on the wire 40.

In the guide wire 100M according to the present embodiment, the spoon-shaped leading portion 20 can efficiently drill the lesion 220, improving the passability through the lesion 220.

### (Examples)

A plurality of samples of the guide wire 100 were prepared, and subjected to performance evaluation using the plurality of samples. In the performance evaluation, 47 samples having mutually different combinations of the largest outer diameter Dx of the leading portion 20 and the largest outer diameter D1 of the distal end 16 of the main body part 10 were prepared to evaluate the passability through a lesion.

FIG. 49 shows a diagram illustrating a method of evaluation. An evaluator prepares a first simulated blood vessel 310 made of resin and having a path 311 which is curved with R65. The evaluator fixes a second simulated blood vessel 320 having a simulated lesion 322 arranged in a path 321 so as to be in communication with the first simulated blood vessel 310. The simulated lesion 322 is 4 mm-thick paraffin. The simulated lesion 322 is fixed via a gel 323 (hardness: 10,000 gf/cm² (980.665 kPa)) filled before and after the simulated lesion 322. The paraffin which forms the simulated lesion 322 contains 3 wt% of stearic acid.

The evaluator inserts a transparent tube (inner diameter: 6 mm) (not shown) into the path 311 of the first simulated blood vessel 310, and inserts a guiding sheath GS into the tube. The evaluator advances the guiding sheath GS until a distal end of the guiding sheath GS is located around the middle between an apex Vp of a curve and a straight portion SP of the path 311 of the first simulated blood vessel 310. The evaluator inserts a micro catheter MC into the guiding sheath GS, and advances the micro catheter MC until a distal end of the micro catheter MC is located before the simulated lesion 322.

The evaluator inserts a sample SA of the guide wire 100 into the micro catheter MC, and advances the sample SA until the distal end of the sample SA makes contact with the distal end of the simulated lesion 322. The evaluator advances the sample SA at a rate around which breakage is not caused while rotating the sample SA clockwise at a fixed rotating rate (120 rpm). The evaluator starts measurement of a passing time at the same time when the sample SA starts rotating, and a time for the distal end of the sample SA to pass through the simulated lesion 322 is recorded as the passing time.

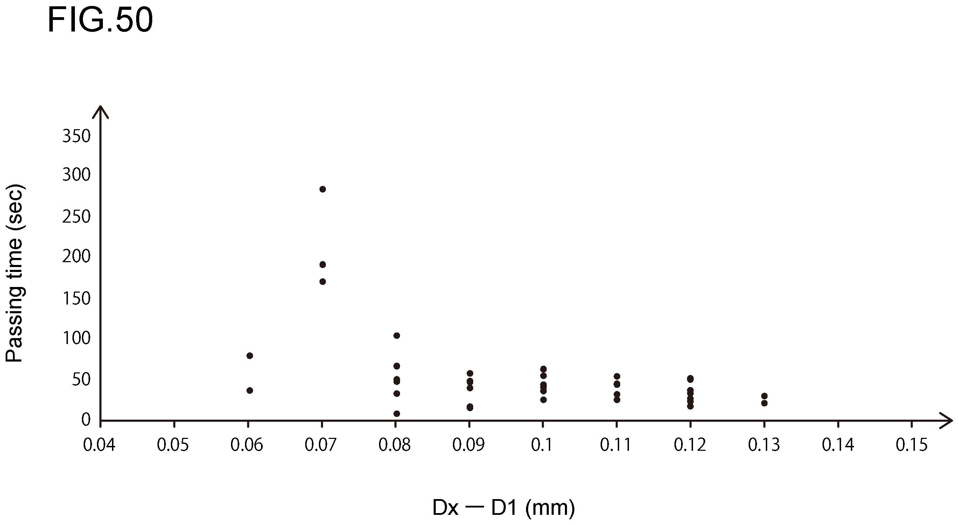

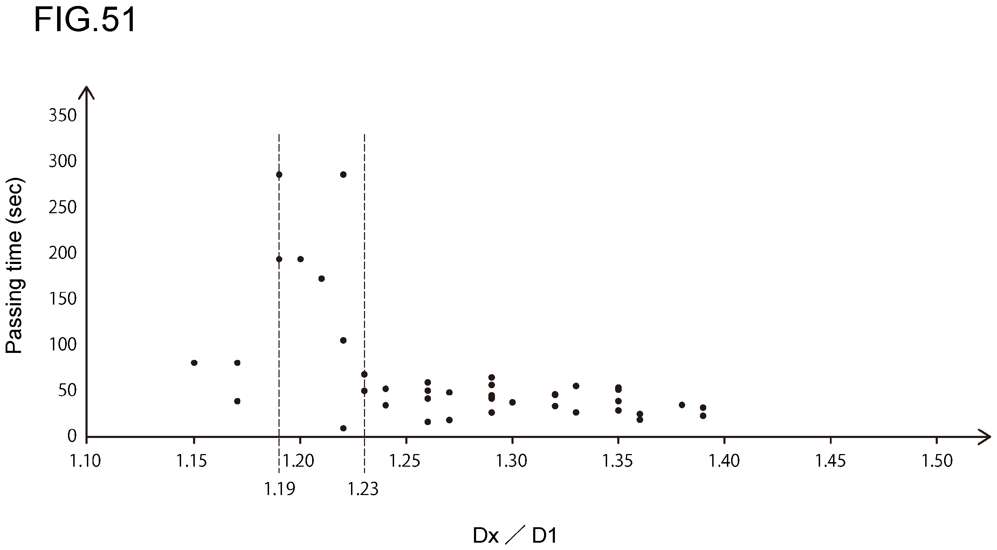

Table 1 shows evaluation results. FIGs. 50 and 51 show graphs representing evaluation results.

**[Table 1]**

| Sample No. | Largest outer diameter of leading portion Dx(mm) | Outer diameter of distal end of main body portion D1(mm) | Dx-D1 (mm) | Dx/D1 | Passing time (see) |
|---|---|---|---|---|---|
| 1 | 0.4 | 0.35 | 0.05 | 1.14 | Not passing through |
| 2 | 0.41 | 0.35 | 0.06 | 1.17 | 80 |
| 3 | 0.41 | 0.35 | 0.06 | 1.17 | 38 |
| 4 | 0.45 | 0.39 | 0.06 | 1.15 | 80 |
| 5 | 0.39 | 0.32 | 0.07 | 1.22 | 286 |
| 6 | 0.42 | 0.35 | 0.07 | 1.2 | 193 |
| 7 | 0.44 | 0.37 | 0.07 | 1.19 | 286 |
| 8 | 0.43 | 0.36 | 0.07 | 1.19 | 193 |
| 9 | 0.44 | 0.36 | 0.08 | 1.22 | 104 |
| 10 | 0.4 | 0.33 | 0.07 | 1.21 | 172 |
| 11 | 0.43 | 0.35 | 0.08 | 1.23 | 67 |
| 12 | 0.43 | 0.35 | 0.08 | 1.23 | 49 |
| 13 | 0.44 | 0.36 | 0.08 | 1.22 | 8 |
| 14 | 0.44 | 0.35 | 0.09 | 1.26 | 40 |
| 15 | 0.41 | 0.33 | 0.08 | 1.24 | 51 |
| 16 | 0.42 | 0.34 | 0.08 | 1.24 | 33 |
| 17 | 0.44 | 0.35 | 0.09 | 1.26 | 58 |
| 18 | 0.44 | 0.35 | 0.09 | 1.26 | 49 |
| 19 | 0.43 | 0.34 | 0.09 | 1.26 | 15 |
| 20 | 0.42 | 0.33 | 0.09 | 1.27 | 47 |
| 21 | 0.42 | 0.33 | 0.09 | 1.27 | 17 |
| 22 | 0.44 | 0.34 | 0.1 | 1.29 | 42 |
| 23 | 0.44 | 0.34 | 0.1 | 1.29 | 41 |
| 24 | 0.44 | 0.34 | 0.1 | 1.29 | 63 |
| 25 | 0.44 | 0.34 | 0.1 | 1.29 | 63 |
| 26 | 0.44 | 0.34 | 0.1 | 1.29 | 44 |
| 27 | 0.45 | 0.35 | 0.1 | 1.29 | 42 |
| 28 | 0.43 | 0.33 | 0.1 | 1.3 | 36 |
| 29 | 0.44 | 0.34 | 0.1 | 1.29 | 40 |
| 30 | 0.44 | 0.34 | 0.1 | 1.29 | 25 |
| 31 | 0.45 | 0.34 | 0.11 | 1.32 | 32 |
| 32 | 0.44 | 0.34 | 0.1 | 1.29 | 55 |
| 33 | 0.45 | 0.34 | 0.11 | 1.32 | 44 |
| 34 | 0.44 | 0.34 | 0.1 | 1.29 | 43 |
| 35 | 0.44 | 0.33 | 0.11 | 1.33 | 25 |
| 36 | 0.44 | 0.33 | 0.11 | 1.33 | 54 |
| 37 | 0.46 | 0.34 | 0.12 | 1.35 | 50 |
| 38 | 0.45 | 0.34 | 0.11 | 1.32 | 45 |
| 39 | 0.46 | 0.34 | 0.12 | 1.35 | 37 |
| 40 | 0.46 | 0.34 | 0.12 | 1.35 | 52 |
| 41 | 0.45 | 0.33 | 0.12 | 1.36 | 17 |
| 42 | 0.45 | 0.33 | 0.12 | 1.36 | 17 |
| 43 | 0.46 | 0.34 | 0.12 | 1.35 | 27 |
| 44 | 0.45 | 0.33 | 0.12 | 1.36 | 23 |
| 45 | 0.44 | 0.32 | 0.12 | 1.38 | 33 |
| 46 | 0.46 | 0.33 | 0.13 | 1.39 | 30 |
| 47 | 0.46 | 0.33 | 0.13 | 1.39 | 21 |

As shown in Table 1 and FIG. 50, a sample 1 in which a difference Δd (= Dx - D1) between the largest outer diameter Dx of the leading portion 20 and the largest outer diameter D1 of the distal end 16 of the main body part 10 was less than 0.06 mm was not able to pass through the simulated lesion 322. Samples 2 to 47 having the difference Δd of 0.06 mm or more and 0.15 mm or less were able to pass through the simulated lesion 322 within 300 seconds. Samples 5 to 47 having the difference Δd of 0.07 mm or more and 0.15 mm or less were able to pass through the simulated lesion 322 within 290 seconds. Samples 9 to 47 having the difference Δd of 0.08 mm or more and 0.15 mm or less were able to pass through the simulated lesion 322 within 110 seconds.

As shown in Table 1 and FIG. 51, the sample 1 in which a ratio Cd (= Dx/D1) of the largest outer diameter Dx of the leading portion 20 to the largest outer diameter D1 of the distal end 16 of the main body part 10 was less than 1.15 was not able to pass through the simulated lesion 322. Samples 2 to 47 having the ratio Cd of 1.15 or more and 1.50 or less were able to pass through the simulated lesion 322 within 300 seconds. Samples 5 to 47 having the ratio Cd of 1.19 or more and 1.50 or less were able to pass through the simulated lesion 322 within 290 seconds. Samples 11 to 47 having the ratio Cd of 1.23 or more and 1.50 or less were able to pass through the simulated lesion 322 within in 110 seconds.

### (Variations)

The technology disclosed herein is not limited to the embodiments described above, but can be modified into various aspects as long as they do not depart from the spirit of the present disclosure. For example, the following modifications can be made.

The configurations of the guide wire 100 in the above embodiments are merely examples, and can be modified in various ways. For example, the leading portion 20 may not need to have an end portion located outside the outer peripheral surface 17 of the main body part 10. The largest outer diameter Dx of the leading portion 20 may be equal to or less than the largest diameter D1 of the distal end 16 of the main body part 10.

The guide wire 100 may not need to include at least one of the coil 50, the distal end side-jointing material 61, the proximal end side-jointing material 62, the first coating 31, the second coating 32, and the third coating 33.

A tubular material may be used to cover both the leading portion 20 and the main body part 10, and may be squeezed to fix the leading portion 20 and the main body part 10.

The materials of the members in the above embodiments are merely examples, and can be modified in various ways. The method of manufacturing the guide wire 100 according to the above embodiments and the method of treatment using the guide wire 100 are merely examples, and can be modified in various ways.

In the above embodiments, the present disclosure was described with reference to the guide wire 100 for treating a lesion in a blood vessel as an example. The technology disclosed herein can be applied to general medical devices for treating a lesion in a living body lumen as well.

The present disclosure includes the following items:
1) A medical device (100) comprising:
   an elongated main body part (10); and
   a leading portion (20) which enters into a lesion (220), the leading portion (20) being connected to a distal end (16) of the main body part (10) and having a first end portion (21) located outside an outer peripheral surface (17) of the main body part (10).
2) The medical device (100) according to 1), wherein
   the main body part (10) extends along an axis (Ax), and
   the first end portion (21) is located outside the outer peripheral surface (17) of the main body part (10) in a first direction orthogonal to the axis (Ax) of the main body part (10).
3) The medical device (100) according to 2), wherein
   the leading portion (20) has a second end portion (22) located outside the outer peripheral surface (17) of the main body part (10) in a second direction orthogonal to the axis (Ax).
4) The medical device (100) according to 3), wherein
   a protrusion amount of the leading portion (20) from the outer peripheral surface (17) of the main body part (10) at the first end portion (21) is different from a protrusion amount of the leading portion (20) from the outer peripheral surface (17) of the main body part (10) at the second end portion (22).
5) The medical device (100) according to 3) or 4), wherein
   the first end portion (21) and the second end portion (22) of the leading portion (20) are opposite to each other across a virtual line (VL) extended from the axis Ax of the main body part (10).
6) The medical device (100) according to any one of 2) to 5), wherein
   in the leading portion (20), a third end portion (25) opposite to the first end portion (21) across a virtual line (VL) extended from the axis Ax of the main body part (10) is located inside the outer peripheral surface (17) of the main body part (10) in the first direction.
7) The medical device (100) according to any one of 2) to 6), wherein
   an outer diameter of the leading portion (20) in a direction orthogonal to the axis (Ax) of the main body part (10) is largest at a position of the first end portion (21) in a direction where the axis (Ax) extends.
8) The medical device (100) according to any one of 1) to 7), wherein
   a largest outer diameter (Dx) of the leading position (20) is larger than a largest outer diameter (D1) of the distal end (16) of the main body part (10), and a thickness (T2) of the leading position (20) as an outer diameter in a direction orthogonal to the largest outer diameter (Dx) of the leading position (20) is smaller than the largest outer diameter (D1) of the distal end (16) of the main body part (10).
9) The medical device (100) according to any one of 1) to 8), wherein
   a difference between a largest outer diameter (Dx) of the leading portion (20) and a largest outer diameter (D1) of the distal end (16) of the main body part (10) is 0.06 mm or more and 0.15 mm or less.
10) The medical device (100) according to 9), wherein
   the difference is 0.07 mm or more and 0.15 mm or less.
11) The medical device (100) according to 9), wherein
   the difference is 0.08 mm or more and 0.15 mm or less.
12) The medical device (100) according to any one of 1) to 11), wherein
   a ratio of a largest outer diameter (Dx) of the leading portion (20) to a largest outer diameter (D1) of the distal end (16) of the main body part (10) is 1.15 or more and 1.50 or less.
13) The medical device (100) according to 12), wherein
   the ratio is 1.19 or more and 1.50 or less.
14) The medical device (100) according to 12), wherein
   the ratio is 1.23 or more and 1.50 or less.
15) A medical device (100) comprising:
   an elongated main body part (10) having a lesion discharge function; and
   a leading portion (20) which enters into a lesion (220), the leading portion (20) being connected to a distal end (16) of the main body part (10).
16) The medical device (100) according to 15), wherein
   the lesion discharge function discharges a small piece of the lesion (220) generated by contact between the leading portion (20) and the lesion (220) from a distal end side toward a proximal end side of the main body part (10).
17) The medical device (100) according to 15) or 16), wherein
   the lesion discharge function is achieved by virtue of a spiral groove (18) formed on an outer peripheral surface (17) of the main body part (10).
18) The medical device (100) according to 17), wherein
   the main body part (10) includes a coil (50), and
   the spiral groove (18) is formed on an outer peripheral surface (52) of the coil (50).
19) The medical device (100) according to 17) or 18), wherein
   a small piece of the lesion (220) moves from the distal end side toward the proximal end side of the main body part (10) along the groove (18).
20) The medical device (100) according to any one of 15) to 19), wherein
   the lesion discharge function is achieved by a structure in which a largest outer diameter (D1) of the distal end (16) of the main body part (10) is smaller than a largest outer diameter (Dx) of the leading portion (20).
21) The medical device (100) according to any one of 15) to 20), wherein
   the lesion discharge function is achieved by a structure in which the main body part (10) has a tapered portion (14) that reduces in diameter from the proximal end side toward the distal end side.
22) The medical device (100) according to any one of 15) to 21), wherein
   the leading portion (20) includes an edge shaped so as to scrape the lesion (220) when rotated around its axis while pressed against the lesion (220).
23) The medical device (100) according to 22), wherein
   the leading portion (20) enters into a space created by scraping the lesion (220) with the edge of the leading portion (20) to form a through-hole in the lesion (220).
24) A medical device (100) comprising:
   an elongated main body part (10) extending along an axis (Ax); and
   a leading portion (20) which enters into a lesion (220), the leading portion (20) being connected to a distal end (16) of the main body part (10),
   wherein
   a lesion passability when the medical device (100) is rotated in a first rotational direction around the axis (Ax) differs from a lesion passability when the medical device (100) is rotated in a second rotational direction opposite to the first rotational direction.
25) The medical device (100) according to 24), wherein
   the lesion passability when the medical device (100) is rotated in the first rotational direction is higher than the lesion passability when the medical device (100) is rotated in the second rotational direction, and
   a torque generated when the medical device (100) is rotated in the first rotational direction is lower than a torque generated when the medical device 100 is rotated in the second rotational direction.
26) The medical device (100) according to 24) or 25), wherein
   a spiral groove (18) is formed on an outer peripheral surface (17) of the main body part (10).
27) The medical device (100) according to 26), wherein
   the main body part (10) includes a coil (50), and
   the spiral groove (18) is formed on an outer peripheral surface (52) of the coil (50).
28) The medical device (100) according to 24), wherein
   the first rotational direction is a clockwise direction as centered around its rotation axis extending from a proximal end to a distal end of the main body part (10), and
   a spiral groove (18) is formed on an outer peripheral surface (17) of the main body part (10), and
   the spiral groove (18) is Z-wound, and the lesion passability when the medical device (100) is rotated in the first rotational direction is higher than the lesion passability when the medical device (100) is rotated in the second rotational direction.
29) The medical device (100) according to 28), wherein
   a torque generated when the medical device (100) is rotated in the first rotational direction is lower than a torque generated when the medical device 100 is rotated in the second rotational direction.
30) The medical device (100) according to 24), wherein
   the first rotational direction is a clockwise direction as centered around its rotation axis extending from a proximal end to a distal end of the main body part (10), and
   a spiral groove (18) is formed on an outer peripheral surface (17) of the main body part (10), and
   the spiral groove (18) is S-wound, and the lesion passability when the medical device (100) is rotated in the first rotational direction is lower than the lesion passability when the medical device (100) is rotated in the second rotational direction.
31) The medical device (100) according to 30), wherein
   a torque generated when the medical device (100) is rotated in the first rotational direction is higher than a torque generated when the medical device (100) is rotated in the second rotational direction.
32) The medical device (100) according to 24), wherein
   the first rotational direction is a clockwise direction as centered around its rotation axis extending from a proximal end to a distal end of the main body part (10), and
   the main body part (10) includes a coil (50), and
   the coil (50) is Z-wound, and when the coil (50) is rotated in the first rotational direction, a pitch of the coil (50) is widened, and the lesion passability when the medical device (100) is rotated in the first rotational direction is higher than the lesion passability when the medical device (100) is rotated in the second rotational direction.
33) The medical device (100) according to 32), wherein
   a torque generated when the medical device (100) is rotated in the first rotational direction is lower than a torque generated when the medical device (100) is rotated in the second rotational direction.
34) The medical device (100) according to 24), wherein
   the first rotational direction is a clockwise direction as centered around its rotation axis extending from a proximal end to a distal end of the main body part (10), and
   the main body part (10) includes a coil (50), and
   the coil (50) is S-wound, and when the coil (50) is rotated in the first rotational direction, a pitch of the coil (50) is narrowed, and the lesion passability when the medical device (100) is rotated in the first rotational direction is lower than the lesion passability when the medical device (100) is rotated in the second rotational direction.
35) The medical device (100) according to 34), wherein
   a torque generated when the medical device (100) is rotated in the first rotational direction is higher than a torque generated when the medical device (100) is rotated in the second rotational direction.
36) The medical device (100) according to any one of 24) to 35), wherein
   the leading portion (20) includes an edge shaped so as to scrape the lesion (220) when rotated around its axis while pressed against the lesion (220).
37) The medical device (100) according to 36), wherein
   the leading portion (20) enters into a space created by scraping the lesion (220) with the edge of the leading portion (20) to form a through-hole in the lesion (220).
38) A method of treatment comprising:
   inserting a medical device (100) including an elongated main body part (10) extending along an axis (Ax) and a loop-shaped leading portion (20) connected to a distal end (16) of the main body part (10) into a living body lumen (200) from a side of the leading portion (20); and
   advancing the leading portion (20) through a lesion (220) while rotating the medical device (100) around the axis (Ax) within a living body lumen (200).
39) The method of treatment according to 38), wherein
   the living body lumen (200) is a blood vessel of a human subject, and
   the lesion (220) is located in the blood vessel at one of the legs of the human subject, and
   the insertion of the medical device (100) includes:
      inserting the medical device (100) into the blood vessel from the leg in which the lesion (220) is located; and
      advancing the medical device (100) in a direction of a flow of blood in the blood vessel.
40) The method of treatment according to 38), wherein
   the living body lumen (200) is a blood vessel of a human subject, and
   the lesion (220) is located in the blood vessel at one of the legs of the human subject, and
   the insertion of the medical device (100) includes:
      inserting the medical device (100) into the blood vessel from either of the ankle or the instep of the leg in which the lesion 220 is located; and
      advancing the medical device (100) in a direction opposite to the direction of a flow of blood in the blood vessel.
41) The method of treatment according to 38), wherein
   the living body lumen (200) is a blood vessel of a human subject, and
   the lesion (220) is located in the blood vessel at one of the legs of the human subject, and
   the insertion of the medical device (100) includes inserting the medical device (100) into the blood vessel from the other leg in which the lesion (220) is not located.
42) The method of treatment according to 38), wherein
   the living body lumen (200) is a blood vessel of a human subject, and
   the lesion (220) is located in the blood vessel at one of the legs of the human subject, and
   the insertion of the medical device (100) includes inserting the medical device (100) into the blood vessel from an arm of the human subject.
43) The method of treatment according to 38), wherein
   the advancement of the medical device (100) includes:
   advancing the medical device (100) while rotating the medical device (100) in a first rotational direction; and
   advancing the medical device (100) while rotating the medical device (100) in a second rotational direction opposite to the first rotational direction.
44) The method of treatment according to 38), wherein
   the advancement of the medical device (100) includes advancing the medical device (100) while rotating the medical device (100) with the rotational direction of the medical device 100 fixed to a first rotational direction.
45) The method of treatment according to 38), wherein
   a length of the lesion (220) along an extension direction of the living body lumen (200) is 100 mm or more.
46) The method of treatment according to 38), wherein
   the advancement of the medical device (100) includes enabling a professional to grip the medical device (100) and rotate the medical device (100).
47) The method of treatment according to 38), wherein
   the advancement of the medical device (100) includes rotating the medical device (100) using a driving force from an actuator (182).
48) The method of treatment according to 38), wherein
   the lesion (220) is a highly calcified lesion.
49) The method of treatment according to 38), wherein
   prior to the insertion of the medical device (100), a sheath is inserted into the living body lumen (200), and
   the medical device (100) is then passed through inside the sheath to allow the medical device (100) to be inserted into the living body lumen (200).
50) The method of treatment according to 38), wherein
   prior to the insertion of the medical device (100), a guide wire (110) is inserted into the living body lumen (200), and a catheter (120) is inserted into the living body lumen (200) along the guidewire (110), and the guidewire (110) is then removed from the living body lumen (200), and then the medical device (100) is inserted into the catheter (120) which remains inserted into the living body lumen (200).
51) The method of treatment according to 38), wherein
   the advancement of the medical device (100) is performed in a situation where none of other medical devices are passed through the lesion (220).
52) The method of treatment according to 38), wherein
   after the medical device (100) is passed through the lesion (220),
   a guide wire is passed through the lesion (220) and,
   an atherectomy device is then inserted along the guide wire.
53) The method of treatment according to 38), wherein
   after the medical device (100) is passed through the lesion (220),
   a guide wire is passed through the lesion (220) and,
   a balloon catheter is then inserted along the guide wire.
54) The method of treatment according to 38), wherein
   after the medical device (100) is passed through the lesion (220),
   a guide wire is passed through the lesion (220) and,
   a stent is then inserted along the guide wire.
55) The method of treatment according to 38), wherein
   the leading portion (20) includes an edge shaped so as to scrape the lesion (220) when rotated around its axis while pressed against the lesion (220).
56) The method of treatment according to 55), wherein
   the medical device (100) has the leading portion (20) for forming a through-hole in the lesion (220) by the leading portion (20) entering a space created by scraping the lesion (220) with the edge of the leading portion (20).
57) A medical device (100) comprising:
   an elongated main body part (10); and
   a leading portion (20) which enters into a lesion (220), the leading portion (20) being connected to a distal end (16) of the main body part (10),
   wherein
   the leading portion (20) has a loop-shaped structure formed with a portion of a wire (40), and
   the main body part (10) includes another portion of the wire (40), and
   the wire (40) has a first portion (P1) having a first thickness and a second portion (P2) having a second thickness thinner than the first thickness, a thickness direction of the wire (40) being orthogonal to a direction of a largest outer diameter (Dx) of the leading portion (20).
58) The medical device (100) according to 57), wherein
   the first portion (P1) and the second portion (P2) are present in the leading portion (20).
59) The medical device (100) according to 58), wherein
   the second portion (P2) is a portion in which a groove (26) concave in the thickness direction is formed.
60) The medical device (100) according to 58) or 59), wherein
   the second portion (P2) corresponds to at least one end portion of the leading portion (20) in the direction of the largest outer diameter (Dx) of the leading portion (20).
61) The medical device (100) according to 58) or 59), wherein
   the second portion (P2) corresponds to a distal end portion of the leading portion (20).
62) A medical device (100) comprising:
   an elongated main body part (10); and
   a leading portion (20) which enters into a lesion (220), the leading portion (20) being connected to a distal end (16) of the main body part (10),
      wherein
   the leading portion (20) has a loop-shaped structure formed with a portion of a wire (40), and
   the main body part (10) includes another portion of the wire (40), and
   the wire (40) has a first portion (P1) having a first cross section (CS1) and a second portion (P2) having a second cross section (CS2) different in shape from the first cross section (CS1).
63) The medical device (100) according to 62), wherein
   the area of the first cross section (CS1) is different from that of the second cross section (CS2).
64) The medical device (100) according to 62) or 63), wherein
   a cross section of the wire (40) included in the main body part (10) is circular in shape, and
   a circularity of the first cross section (CS1) is different from that of the second cross section (CS2).
65) The medical device (100) according to any one of 62) to 64), wherein
   the first portion (P1) is provided in the main body part (10), and the second portion (P2) is provided in the leading portion (20).
66) The medical device (100) according to any one of 62) to 64), wherein
   a cross section of the wire (40) included in the main body part (10) is circular in shape, and
   the first portion (P1) is present in the leading portion (20), and
   a curvature of an outer peripheral side and a curvature of an inner peripheral side are different from each other along an outline of the first cross section (CS1).
67) The medical device (100) according to 66), wherein
   the curvature of the outer peripheral side along the outline of the first cross section (CS1) is smaller than that of the inner peripheral side.
68) The medical device (100) according to 62) or 63), wherein
   an outline of a cross section of the wire included in the main body part (10) consists of an arc line and a line segment connecting end portions of the arc line, and
   with respect to a ratio of a height in a direction orthogonal to a direction of a largest width of a cross section to the largest width, the ratio in the first cross section (CS1) is different from that in the second cross section (CS2).
69) The medical device (100) according to 62) or 63), wherein
   an outline of a cross section of the wire (40) included in the main body part (10) includes a first straight line portion, and,
   a portion corresponding to the first straight line portion is a curved line in an outline of a cross section of the wire (40) included in the leading portion (20).
70) The medical device (100) according to 69), wherein
   the outline of the cross section of the wire (40) included in the main body part (10) includes a second straight line portion.
71) The medical device (100) according to 70), wherein
   the first straight line portion and the second straight line portion are opposite to each other across the center of gravity of the cross section of the wire (40).
72) The medical device (100) according to any one of 69) to 71), wherein
   a portion of an outer peripheral side is concave, and a portion of an inner peripheral side is convex in the outline of the cross section of the wire (40) included in the leading portion (20).
73) A medical device (100) comprising:
   an elongated main body part (10); and
   a leading portion (20) which enters into a lesion (220), the leading portion (20) being connected to a distal end (16) of the main body part (10),
      wherein
   the leading portion (20) has a loop-shaped structure formed with a portion of a wire (40), and
   the main body part (10) includes another portion of the wire (40), and
   the wire (40) has a specific portion (P3, P1), and an outline of a cross section of the specific portion (P3, P1) consists of an arc line and a line segment connecting end portions of the arc line.
74) The medical device (100) according to 73), wherein
   the specific portion (P3) is present in the main body part (10).
75) The medical device (100) according to 73) or 74), wherein
   the specific portion (P1) is present in the leading portion (20).
76) The medical device (100) according to any one of 73) to 75), wherein
   a largest width of a cross section of the specific portion (P1) is larger than a length of the line segment.
77) The medical device (100) according to any one of 73) to 75), wherein
   the largest width of the cross section of the specific portion (P1) is substantially identical to the length of the line segment.
78) A medical device (100) comprising:
   an elongated main body part (10) having a first rigid portion (11) having a first rigidity and a second rigid portion (12) located at a proximal end side of the first rigid portion (11) and having a second rigidity lower than the first rigidity; and
   a leading portion (20) which enters into a lesion (220), the leading portion (20) being connected to a distal end (16) of the main body part (10).
79) The medical device (100) according to 78), wherein
   the main body part (10) has a third rigid portion (13) located at a proximal end side of the second rigid portion (12) and having a third rigidity higher than the second rigidity.
80) The medical device (100) according to 79), wherein
   the first rigidity is equal to or lower than the third rigidity.
81) The medical device (100) according to 79), wherein
   the first rigidity is higher than the third rigidity.
82) The medical device (100) according to any one of 78) to 81), wherein
   the leading portion (20) has a loop-shaped structure formed with a part of a base portion (40B) and a part of a folded-back portion (40A) of a wire (40) including the base portion (40B) and the folded-back portion (40A) folded back from the base portion (40B), and
   the main body part (10) includes a part of the base portion (40B) and a part of the folded-back portion (40A) of the wire (40), and
   the first rigid portion (11) is a portion of the main body part (10) which includes the folded-back portion (40A) of the wire (40).
83) The medical device (100) according to 82), wherein
   the base portion (40B) has a tapered portion (42, 44) having a largest width decreased toward a distal end side of the main body part (10).
84) The medical device (100) according to 83), wherein
   the tapered portion is a first tapered portion (42), and,
   the base portion (40B) has a second tapered portion (44) provided at a distal end side of the first tapered portion (42) and having a largest width decreased toward the distal end side of the main body part (10) and having a slope different from that of the first tapered portion (42).
85) The medical device (100) according to any one of 82) to 84), wherein
   the main body part (10) has a parallel portion (11) in which the base portion (40B) and the folded-back portion (40A) extend along an axis (Ax) of the main body part (10), and
   the base portion (40B) and the folded-back portion (40A) are spaced in the parallel section (11).
86) A medical device (100) comprising:
   an elongated main body part (10); and
   a leading portion (20) which enters into a lesion (220), the leading portion (20) being connected to a distal end (16) of the main body part (10) and having a reinforcing portion (28) located at a connection site with the main body part (10).
87) The medical device (100) according to 86), wherein
   the leading portion (20) includes a wire (40), and
   the main body part (10) includes a coil (50) joined to the wire (40) via a joining material (61), and
   the reinforcing portion (28) is formed with a portion of the joining material (61).
88) The medical device (100) according to 87), wherein
   the joining material (61) is provided inside the coil (50).
89) The medical device (100) according to any one of 86) to 88), wherein
   the leading portion (20) has a loop-shaped structure formed with a part of a base portion (40B) and a part of a folded-back portion (40A) of a wire (40) including the base portion (40B) and the folded-back portion (40A) folded back from the base portion (40B), and
   the main body part (10) includes a part of the base portion (40B) and a part of the folded-back portion (40A) of the wire (40), and
   the joining portion (61) is provided between the base portion (40B) and the folded-back portion (40A).
90) The medical device (100) according to 89), wherein
   the main body part (10) includes:
   a joining region (R1) in which a part of the base portion (40B), a part of the folded-back portion (40A), and the main body part (10) are joined; and
   a non-joining region (R2) in which a part of the base portion (40B), a part of the folded-back portion (40A), and the main body part (10) are not joined.
91) The medical device (100) according to any one of 88) to 90), wherein
   a width of the reinforcing portion (28) in a direction orthogonal to an axis (Ax) of the main body part (10) is decreased toward a distal end of the reinforcing portion (28).
92) The medical device (100) according to any one of 88) to 90), wherein
   a width of the reinforcing portion (28) in a direction orthogonal to the axis (Ax) of the main body part (10) is increased toward the distal end of the reinforcing portion (28).
93) The medical device (100) according to any one of 88) to 92), wherein
   an outer peripheral surface of the reinforcing portion (28) has a curved surface recessed toward a radially inward side of the reinforcing portion (28).
94) The medical device (100) according to any one of 88) to 93), wherein
   the reinforcing portion (28) is formed with a welded portion of the leading portion (20) and the main body part (10).
95) A medical device (100) comprising:
   a main body part (10) which is elongated along an axis (Ax), and has a curved portion (19); and
   a leading portion (20) which enters into a lesion (220), the leading portion (20) being connected to a distal end (16) of the main body part (10).
96) The medical device (100) according to 95), wherein
   the leading portion (20) has a largest outer diameter (Dx) in a first direction orthogonal to the axis (Ax), and
   an angle between a first virtual line (VL1) parallel to the first direction and a first virtual plane (VP) encompassing the axis (Ax) in the curved portion (19) of the main body part (10) is 80 degrees or less.
97) The medical device (100) according to 96), wherein
   the angle is 10 degrees or less.
98) The medical device (100) according to 96), wherein
   the first virtual line (VL1) and the first virtual plane (VP) are substantially parallel.
99) The medical device (100) according to 96), wherein
   the first virtual line (VL1) and the first virtual plane (VP) are orthogonal.
100) A medical device (100) comprising:
   an elongated main body part (10);
   a leading portion (20) which enters into a lesion (220), the leading portion (20) being connected to a distal end (16) of the main body part (10);
   a first coating (31) having a first slidability, and covering at least a part of the leading portion (20); and
   a second coating (32) having a second slidability different from the first slidability, and covering at least a part of the main body part (10).
101) The medical device (100) according to 100), wherein
   the first slidability is lower than the second slidability.
102) The medical device (100) according to 101), wherein
   the first coating (31) is hydrophobic, and
   the second coating (32) is hydrophilic.
103) The medical device (100) according to any one of 100) to 102), wherein
   a distal end portion of the main body part (10) is covered with the first coating (31).
104) A method of manufacturing a medical device (100) including an elongated main body part (10) having a tubular body (50) and a leading portion (20) which enters into a lesion (220), the leading portion (20) being connected to a distal end (16) of the main body part (10) and having a loop-shaped wire (40),
   the method comprising:
   performing a bending work of pressing the wire (40) against a pin to bend the wire (40) into a loop-shaped structure; and
   joining the wire (40) to the tubular body (50).
105) The method of manufacture according to 104), comprising
   inserting the wire (40) into a hollow space of the tubular body (50) prior to the bending work.
106) The method of manufacture according to 104) or 105), wherein
   the joining includes using a soldering material.
107) The method of manufacture according to 106), wherein
   the tubular body (50) is a coil (50), and
   the joining includes feeding the soldering material into a hollow space of the coil (50) through a pitch portion of the coil (50).
108) The method of manufacture according to 106) or 107), wherein
   the leading portion (20) has a reinforcing portion (28) located at a connection site with the main body part (10), and
   the joining includes forming the reinforcing portion (28) with the soldering material.
109) The method of manufacture according to any one of 104) to 108), wherein
   the bending work includes deforming a cross section of the wire (40).
110) The method of manufacture according to any one of 104) to 109), wherein
   a narrowed portion (29) is formed at a proximal end portion of the leading portion (20), and
   the bending work includes forming the narrowed portion (29).
111) The method of manufacture according to any one of 104) to 110), comprising performing grinding work of the wire (40) prior to the bending work.
112) The method of manufacture according to any one of 104) to 111), comprising bending a distal end portion of the main body part (10) after the joining.
113) A medical device (100) comprising:
   an elongated main body part (10); and
   a leading portion (20) which enters into a lesion (220), the leading portion (20) being connected to a distal end (16) of the main body part (10), having a loop-shaped structure formed with a portion of a wire (40), and having an intersecting portion (49) in a proximal end portion in which the wire (40) intersects.
114) The medical device (100) according to 113), wherein
   the leading portion (20) has a reinforcing portion (28) located at a connection site with the main body part (10), and
   a part of the reinforcing portion (28) is a joining material covering an outer periphery of the intersecting portion (49).

## Claims

1. A medical device (100) comprising:
an elongated main body part (10) having a lesion discharge function; and
a leading portion (20) which enters into a lesion (220), the leading portion (20) being connected to a distal end (16) of the main body part (10).

2. The medical device (100) according to claim 1, wherein
the lesion discharge function discharges a small piece of the lesion (220) generated by contact between the leading portion (20) and the lesion (220) from a distal end side toward a proximal end side of the main body part (10).

3. The medical device (100) according to claim 1 or 2, wherein
the lesion discharge function is achieved by virtue of a spiral groove (18) formed on an outer peripheral surface (17) of the main body part (10).

4. The medical device (100) according to claim 3, wherein
the main body part (10) includes a coil (50), and
the spiral groove (18) is formed on an outer peripheral surface (52) of the coil (50).

5. The medical device (100) according to claim 3 or 4, wherein
a small piece of the lesion (220) moves from the distal end side toward the proximal end side of the main body part (10) along the groove (18).

6. The medical device (100) according to any one of claims 1 to 5, wherein
the lesion discharge function is achieved by a structure in which a largest outer diameter (D1) of the distal end (16) of the main body part (10) is smaller than a largest outer diameter (Dx) of the leading portion (20).

7. The medical device (100) according to any one of claims 1 to 6, wherein
the lesion discharge function is achieved by a structure in which the main body part (10) has a tapered portion (14) that reduces in diameter from the proximal end side toward the distal end side.

8. The medical device (100) according to any one of claims 1 to 7, wherein
the leading portion (20) includes an edge shaped so as to scrape the lesion (220) when rotated around its axis while pressed against the lesion (220).

9. The medical device (100) according to claim 8, wherein
the leading portion (20) enters into a space created by scraping the lesion (220) with the edge of the leading portion (20) to form a through-hole in the lesion (220).
